(19) Europäisches Patentamt
European Patent Office
Office européen des brevets

(11) EP 2 011 870 A1

(12) EUROPEAN PATENT APPLICATION
published in accordance with Art. 153(4) EPC

(43) Date of publication:
**07.01.2009 Bulletin 2009/02**

(21) Application number: **07741539.6**

(22) Date of filing: **12.04.2007**

(51) Int Cl.:
***C12N 15/09*** *(2006.01)* ***A01K 67/027*** *(2006.01)*
***A61K 39/395*** *(2006.01)* ***A61P 1/00*** *(2006.01)*
***A61P 19/02*** *(2006.01)* ***A61P 29/00*** *(2006.01)*
***A61P 35/00*** *(2006.01)* ***A61P 35/02*** *(2006.01)*
***A61P 35/04*** *(2006.01)* ***A61P 37/04*** *(2006.01)*
***A61P 37/06*** *(2006.01)* ***C07K 16/28*** *(2006.01)*
***C07K 16/46*** *(2006.01)* ***C12N 1/15*** *(2006.01)*
***C12N 1/19*** *(2006.01)* ***C12N 1/21*** *(2006.01)*
***C12N 5/10*** *(2006.01)* ***C12P 21/02*** *(2006.01)*
***C12P 21/08*** *(2006.01)*

(86) International application number:
**PCT/JP2007/058103**

(87) International publication number:
**WO 2007/119796 (25.10.2007 Gazette 2007/43)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **14.04.2006 JP 2006111957**
**24.04.2006 JP 2006119607**

(71) Applicants:
- **Medical and Biological Laboratories Co., Ltd.**
**Nagoya-shi,**
**Aichi 460-0002 (JP)**
- **Otsuka Chemical Co., Ltd.**
**Osaka-shi,**
**Osaka 540-0021 (JP)**

(72) Inventors:
- **TSUJI, Takashi**
**Tokyo 162-8601 (JP)**
- **KAJIHARA, Yasuhiro**
**Yokohama-shi**
**Kanagawa 224-0014 (JP)**
- **NAMBU, Yuri**
**Tokushima-shi**
**Tokushima 771-0193 (JP)**
- **FUKAE, Kazuhiro**
**Tokushima-shi**
**Tokushima 771-0193 (JP)**
- **ASAI, Hiroaki**
**Tokushima-shi**
**Tokushima 771-0193 (JP)**
- **MURAKAMI, Akihiro**
**Ina-shi**
**Nagano 396-0002 (JP)**

(74) Representative: **Denison, Christopher Marcus et al**
**Mewburn Ellis LLP**
**33 Gutter Lane**
**London**
**EC2V 8AS (GB)**

# (54) MUTANT POLYPEPTIDE HAVING EFFECTOR FUNCTION

(57) Mutant polypeptides of the present invention contain an Fc region in which a cysteine residue is substituted for the second amino acid from the glycosylation site to the N terminal side in the Fc region. The Fc region is preferably a human IgG Fc region. The mutant polypeptides of the present invention may also contain an N-linked sugar chain at the glycosylation site in Fc region. Furthermore, a polypeptide domain other than the Fc region of the mutant polypeptides of the present invention may be a polypeptide molecule that recognizes a human cell surface molecule.

EP 2 011 870 A1

## Description

Technical Field

The present invention relates to mutant polypeptides containing an Fc region. More particularly, the present invention relates to polypeptides containing an Fc region that has been altered by amino acid substitution yet retains effector function.

Background Art

Due to their high specificity of antigen recognition and strong interaction with antigens, antibodies have wide applicability as pharmaceutical and diagnostic agents, test reagents, and so on. The applicability of antibodies is expected to be broader in the future.

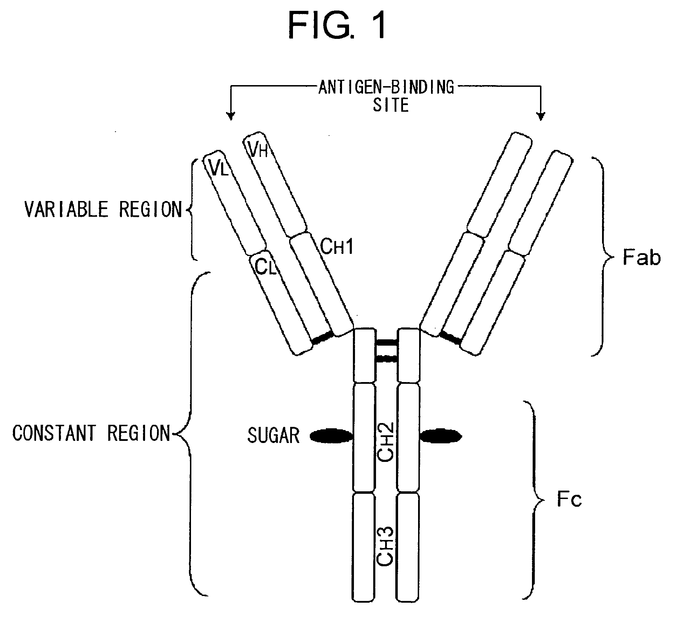

As shown in Fig. 1, an antibody is a polypeptide tetramer composed of two heavy chains and two light chains. The heavy chains and light chains are linked together via disulfide bonds. An antibody has variable regions that serve as the antigen-binding site, and constant/regions. The C terminal domain of the heavy chain constant region is composed of the Fc region that is composed of CH2 and CH3. The Fc region does not directly participate in antigen binding but has various effector functions with cells having surface Fc receptor (FcR) via binding to FcR. Specifically, the binding of the Fc receptor-binding site of Fc region to the FcR on the surface of effector cells can trigger a number of divergent important biological responses, including phagocytotic uptake and disruption of antibody-coated particles, clearance of immune complex, cytolysis of antibody-coated target cells by activated effector cells (referred to as antibody dependent cellular cytotoxicity (ADCC)), release of inflammatory mediators, placental transfer, and control of immunoglobulin production.

The binding of the Fc region to FcR on the surface of effector cells is essential for the ADCC of IgG. It has been suggested that the hinge region and second domain of the constant region (hereinafter abbreviated as the "CH2 domain") of the antibody contain amino acid residues that play important roles in binding (Eur. J. Immunol., 23, 1098 (1993); Immunology, 86, 319 (1995); Chemical Immunology, 65, 88 (1997)). Furthermore, a sugar chain linked to a CH2 domain has been demonstrated to play an important role in the binding between the Fc region and the FcR (Chemical Immunology, 65, 88 (1997); The Third Symposium of Japan Consortium for Glycobiology and Glycotechnology, Abstract p. 30 (2005)). Previous reports suggest that the production of antibodies having greater effector function can be achieved through optimization of the sugar chain structure (Japanese Patent Application Kokai Publication No. (JP-A) 2005-224240 (unexamined, published Japanese patent application)). These reports suggest that the glycosylated CH2 domain of Fc region plays a very important role in the effector functions of human IgG and antibodies having greater effector functions can be produced by optimizing amino acids or sugar chain structure in this region.

Amino acid substitutions in the Fc have been actively investigated. Based on extensive alanine substitution for Fc amino acids, Presta *et al.* found that some amino acid substitutions (S298A, E333A, and K334A) improved the binding affinity for FcγRIIIa and increased the ADCC (International Patent Application Publication WO00/42072 pamphlet). Furthermore, data reported by Lazar *et al.* suggests that simultaneous substitution of S239D/A330L/I332E increases the ADCC of various antibodies (US2005/0054832A1).

Much time and effort has been spent in producing such antibodies, in particular, in creating cells producing antibodies that recognize antigens of interest. In addition, animal cell-based production methods are required to secure sufficient antibody activity. This boosts the cost for antibody production, a major hurdle in the future of antibody medicine. Efficient antibody development requires inexpensive antibody production methods to reduce cost and methods for producing antibodies with strong activity.

Patent Document 1: JP-A (Kokai) 2005-224240
Patent Document 2: International Patent Application Publication WO00/42072 pamphlet
Patent Document 3: U.S. Patent Application No. 2005/0054832 specification
Non-Patent Document 1: Eur. J. Immunol., 23, 1098 (1993)
Non-Patent Document 2: Immunology, 86, 319 (1995)
Non-Patent Document 3: Chemical Immunology, 65, 88 (1997)
Non-Patent Document 4: The Third Symposium of Japan Consortium for Glycobiology and Glycotechnology, Abstract p. 30 (2005)

Disclosure of the Invention

[Problems to be Solved by the Invention]

An objective of the present invention is to provide mutant polypeptides having enhanced effector functions; simple methods for producing such mutant polypeptides; and widely applicable therapeutic compositions containing such mutant polypeptides, which have few side effects and strong pharmacological activity that enable reduction of dose.

[Means for Solving the Problems]

Dedicated studies were performed to achieve the objective described above, and as a result, it was discovered that the effector functions could be significantly improved by substituting a particular amino acid residue for another amino acid residue at a specific site in the Fc region of an antibody. Moreover, it was discovered that when used as therapeutic antibody compositions, polypeptides having such a mutation could produce greater therapeutic effect even at low doses. Accordingly, the present invention was completed.

Specifically, the present invention provides mutant polypeptides that include an Fc region having a substitution of a cysteine residue for the second amino acid from the glycosylation site to the N terminal side in IgG Fc region. The Fc region may be a human IgG Fc region. The mutant polypeptides of the present invention may contain an N-linked sugar chain at the glycosylation site in the Fc region. The mutant polypeptides of the present invention have improved effector functions as compared to their unmodified counterparts. The mutant polypeptides of the present invention may serve as polypeptide molecules, in which a polypeptide domain other than the Fc region recognizes a human cell surface molecule.

The mutant polypeptides of the present invention may constitute antibodies. Specifically, the present invention provides mutant antibodies having a substitution of a cysteine residue for an amino acid at EU index 295 according to the Kabat numbering system. Antibodies constituted by the mutant polypeptides may recognize at least any one selected from the group consisting of cytokine receptors, cell adhesion molecules, cancer cell surface molecules, cancer stem cell surface molecules, blood cell surface molecules, and surface molecules of virus-infected cells. Antibodies constituted by the mutant polypeptides may be antibodies that recognize at least any one selected from the group consisting of antigen CD3, CD11a, CD20, CD22, CD25, CD28, CD33, and CD52, Her2/neu, EGF receptor, EpCAM, MUC1, GD3, CEA, CA125, HLA-DR, TNFalpha receptor, VEGF receptor, CTLA-4, AILIM/ICOS, and integrin molecules.

The mutant polypeptides of the present invention may be polypeptide molecules not derived from any antibody, in which a polypeptide domain other than the Fc region binds to at least one human cell surface molecule selected from the group consisting of cytokine receptors, cell adhesion molecules, cancer cell surface molecules, cancer stem cell surface molecules, blood cell surface molecules, and surface molecules of virus-infected cells. The polypeptide domain other than the Fc region may be a polypeptide molecule that binds to at least one human cell surface molecule selected from the group consisting of CD3, CD11a, CD20, CD22, CD25, CD28, CD33, CD52, Her2/neu, EGF receptor, EpCAM, MUC1, GD3, CEA, CA125, HLA-DR, TNFalpha receptor, VEGF receptor, AILIM/ICOS, CTLA-4, B7h, CD80, CD86, and integrin molecules.

The present invention also provides isolated nucleic acids encoding such mutant polypeptides of the present invention, vectors carrying such nucleic acids, and host cells or host organisms harboring such vectors.

Furthermore, the present invention also provides methods for producing such mutant polypeptides, which include the step of culturing the host cells or host organisms of the present invention to express the mutant polypeptides encoded by the nucleic acids.

Moreover, the present invention provides therapeutic compositions containing the mutant polypeptides of the present invention.

Herein, the term "Fc region" refers to the C terminal region of an antibody heavy chain. The Fc region consists of CH2 and CH3, which are situated on the C terminal side in the heavy chain constant region. The phrase "the second amino acid from the glycosylation site to the N terminal side in the Fc region" means an amino acid indicated by an EU index according to the Kabat numbering system (Sequence of Proteins of Immunological Interest, 5th Ed. Public Health Service, National Institutes of Health, Bethesda, MD, 1991). For example, the amino acid at the glycosylation site in the IgG1 Fc region is N297 in the EU index of the Kabat numbering system, while the second amino acid from the site to the N terminal side is Q295. Accordingly, when a mutant polypeptide of the present invention contains the Fc region of IgG1, it includes a substitution of C295 for the amino acid residue Q295.

Herein, the phrase "mutant polypeptide" refers to a mutant polypeptide containing an Fc region in which a cysteine residue is substituted for the amino acid at EU index 295 according to the Kabat numbering system.

Furthermore, the mutant polypeptides of the present invention may partially contain other peptides or polypeptides. Examples of such peptides or polypeptides include known peptides such as FLAG (Hopp, T. P. et al., BioTechnology 6, 1204-1210 (1988)), 6x His consisting of six histidine (His) residues, 10x His, influenza hemagglutinin (HA), human c-myc fragment, VSV-GP fragment, p18HIV fragment, T7-tag, HSV-tag, E-tag, SV40 T antigen fragment, lck tag, α-tubulin fragment, B-tag, and protein C fragment. Furthermore, other polypeptides such as glutathione-S-transferase (GST), Influenza hemagglutinin (HA), β-galactosidase, and maltose-binding protein (MBP) may be fused with the mutant polypeptides of the present invention.

The present invention provides:

[1] a mutant polypeptide that comprises an Fc region in which a cysteine residue is substituted for an amino acid that is second from the glycosylation site on the N terminal side in the Fc region of an IgG;
[2] a mutant polypeptide that comprises an Fc region in which a cysteine residue is substituted for an amino acid at EU index 295 according to the Kabat numbering system;
[3] the mutant polypeptide of [1] or [2], whose effector function is improved as compared to that before substitution;
[4] the mutant polypeptide of any one of [1] to [3], wherein the Fc region is a human IgG Fc region;
[5] the mutant polypeptide of any one of [1] to [4], which has an N-linked sugar chain at the glycosylation site of the Fc region;
[6] the mutant polypeptide of any one of [1] to [5], which comprises a site that recognizes a human cell surface molecule;
[7] the mutant polypeptide of any one of [1] to [6], which is a mutant antibody comprising a substitution of a cysteine residue for an amino acid at EU index 295 according to the Kabat numbering system;
[8] the mutant polypeptide of [7], which recognizes at least any one selected from the group consisting of cytokine receptors, cell adhesion molecules, cancer cell surface molecules, cancer stem cell surface molecules, blood cell surface molecules, and surface molecules of virus-infected cells;
[9] the mutant polypeptide of [8], which recognizes at least any one selected from the group consisting of antigens CD3, CD11a, CD20, CD22, CD25, CD28, CD33, and CD52, Her2/neu, EGF receptor, EpCAM, MUC1, GD3, CEA, CA125, HLA-DR, TNFalpha receptor, VEGF receptor, CTLA-4, AILIM/ICOS, and integrin molecules;
[10] the mutant polypeptide of any one of [1] to [6], which is a mutant chimeric molecule comprising a cell surface molecule recognition site and an Fc region that comprises a substitution of a cysteine residue for an amino acid at EU index 295 according to the Kabat numbering system;
[1] the mutant polypeptide of [10], which binds to at least one human cell surface molecule selected from the group consisting of cytokine receptors, cell adhesion molecules, cancer cell surface molecules, cancer stem cell surface molecules, blood cell surface molecules, and surface molecules of virus-infected cells;
[12] the mutant polypeptide of [11], which binds to at least one human cell surface molecule selected from the group consisting of CD3, CD11a, CD20, CD22, CD25, CD28, CD33, CD52, Her2/neu, EGF receptor, EpCAM, MUC1, GD3, CEA, CA125, HLA-DR, TNFalpha receptor, VEGF receptor, AILIM/ICOS, CTLA-4, B7h, CD80, CD86, and integrin molecules;
[13] an isolated nucleic acid encoding the mutant polypeptide of any one of [1] to [12];
[14] a vector carrying the nucleic acid of [13];
[15] a host cell or host organism harboring the vector of [14];
[16] a method for producing a mutant polypeptide, which comprises the step of culturing the host cell or host organism of [15] so that the host expresses the mutant polypeptide encoded by a nucleic acid;
[17] a therapeutic composition comprising the mutant polypeptide of any one of [1] to [12];
[18] a method for producing a mutant antibody having improved effector function, which comprises the steps of:

(1) substituting a cysteine residue for an amino acid residue at EU index 295 according to the Kabat numbering system in an antibody having effector function;
(2) introducing a host cell or host organism with a DNA encoding an L chain and a DNA encoding an H chain having an Fc region comprising the substitution of a cysteine residue for an amino acid residue at EU index 295 according to the Kabat numbering system, and expressing the DNA; and
(3) collecting the expression product;

[19] a method for producing a mutant chimeric molecule having improved effector function, which comprises the steps of:

(1) substituting a cysteine residue for an amino acid residue at EU index 295 according to the Kabat numbering system in the Fc region of a chimeric molecule with the effector function, which comprises a cell surface molecule recognition site in addition to the Fc region;
(2) introducing a host cell or host organism with a DNA encoding the mutant chimeric molecule comprising a cell surface molecule recognition site and the Fc region comprising a substitution of a cysteine residue for an amino acid residue at EU index 295 according to the Kabat numbering system, and expressing the DNA; and

(3) collecting the expression product;

[20] a method for producing a mutant chimeric molecule having improved effector function, which comprises the steps of:

(1) producing a mutant chimeric molecule comprising a cell surface molecule recognition site and an Fc region;
(2) judging whether the produced chimeric molecule has the effector function;
(3) substituting a cysteine residue for an amino acid residue at EU index 295 according to the Kabat numbering system in the Fc region of the chimeric molecule that has been judged to have the effector function;
(4) introducing and expressing in a host cell or host organism a DNA encoding the mutant chimeric molecule that comprises the cell surface molecule recognition site and the Fc region comprising a substitution of a cysteine residue for an amino acid residue at EU index 295 according to the Kabat numbering system; and
(5) collecting the expression product;

[21] a method for improving antibody effector function, which comprises the step of substituting a cysteine residue for an amino acid residue at EU index 295 according to the Kabat numbering system in an antibody having effector function;
[22] a method for improving the effector function of a chimeric molecule, which comprises the step of substituting a cysteine residue for an amino acid residue at EU index 295 according to the Kabat numbering system in the Fc region of the chimeric molecule with the effector function, which comprises a cell surface molecule recognition site in addition to the Fc region; and
[23] a method for improving the effector function of a chimeric molecule, which comprises the steps of:

(1) producing a chimeric molecule that comprises a cell surface molecule recognition site and an Fc region;
(2) judging whether the produced chimeric molecule has the effector function; and
(3) substituting a cysteine residue for an amino acid residue at EU index 295 according to the Kabat numbering system in the Fc region of the chimeric molecule that has been judged to have the effector function.

Brief Description of the Drawings

Fig. 1 is a schematic view of an IgG 1 antibody.
Fig. 2 is a photograph depicting the results of electrophoresis of purified, wild-type and 295Cys-type chimeric antibodies.
Fig. 3 is composed of a pair of graphs depicting the results of flow cytometric analysis of purified, wild-type and 295Cys-type chimeric antibodies.
Fig. 4 is a photograph depicting the results of electrophoresis of purified, wild-type and 295Cys-type chimeric antibodies.
Fig. 5 is composed of a pair of graphs depicting the results of flow cytometric analysis of human anti-EGFR antibodies.

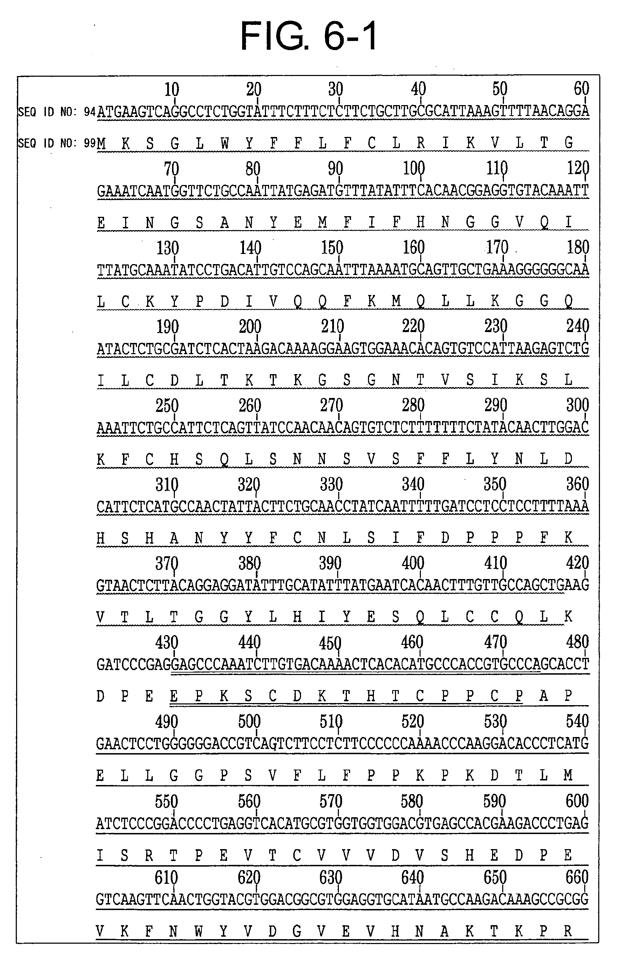

Fig. 6-1 depicts the nucleotide sequence and amino acid sequence of AILIM-IgFc Cys mutant. In the figure, the sequence of the extracellular region of AILIM/ICOS is underlined with a wavy line, the sequence of the hinge region is double underlined, and that of the Fc region of human IgG is underlined. There is a linker sequence between the sequence of the extracellular domain of AILIM/ICOS and that of the hinge region. The cysteine of the substituted position and the codon that encodes this cysteine are indicated with boxes. Here, the cysteine and "TGC" encoding the cysteine, each indicated with a box, are glutamine and "CAG" encoding glutamine, respectively, if no substitution is made.
Fig. 6-2 is a continuation of Fig. 6-1.
Fig. 7 is a graph depicting the evaluation results of the measurement of the ADCC activities for the anti-CD20 antibody and its Cys295 mutant, obtained in Example 1.
Fig. 8 is a graph depicting the results of the measurement of the ADCC activities for the wild-type and 295Cys-type human anti-CD20 antibodies, obtained in Example 2.
Fig. 9 is a graph depicting the results of the measurement of the ADCC activities for the wild-type and 295Cys-type human anti-EGFR antibodies, obtained in Example 3.
Fig. 10 is a graph depicting the evaluation results of the measurement of the ADCC activities for the AILIM/ICOS-IgFc chimeric molecule and its Cys 295 mutant, obtained in Example 4.
Fig. 11 is a graph depicting the results of analysis on reactivity of the Cys 295 mutant of anti-CD20 antibody with CD16.
Fig. 12 is a graph depicting the results of analysis on binding reactivity of AILIM-IgFc chimeric molecule Gln223Cys

with CD16.

Best Mode for Carrying Out the Invention

A specific feature of the mutant polypeptides of the present invention is the substitution of a cysteine residue for the second amino acid from the glycosylation site to the N terminal side in the IgG Fc region. This feature significantly enhances the effector function of the mutant polypeptides of the present invention. Illustrative examples of such effector function include phagocytotic uptake and disruption of antibody-coated particles, clearance of immune complex, cytolysis of antibody-coated target cells (referred to as antibody dependent cellular cytotoxicity (ADCC)) by activated effector cells, disruption of cell membrane by complement proteins (complement dependent cytotoxicity (CDC)), release of inflammatory mediators, placental transfer, and control of immunoglobulin production. Particularly, the mutant polypeptides of the present invention are suited to enhancing ADCC and CDC, and particularly suited to enhancing ADCC.
The mutant polypeptides of the present invention can mediate ADCC more efficiently in the presence of human effector cells. ADCC is a cell-mediated response wherein effector cells recognize and lyse target cells. Illustrative examples of effector cells include killer T cells, natural killer (NK) cells, neutrophiles, and macrophages. These effector cells normally express Fc receptor (FcR) on the surface. Cells expressing structurally bindable FcR are recognized to exert the cytotoxic activity.
The mutant polypeptides can induce and enhance ADCC by recognizing effector cells via the Fc region. The Fc region containing the mutation readily binds to the molecules on the surface of effector cells (FcR or such) and thus can efficiently activate the cells. Perhaps because of this, even a small amount of the mutant polypeptide of the present invention can rapidly induce strong ADCC.
CDC is a complement protein-mediated response wherein the complement protein recognizes target cells and triggers the disruption of target cell membrane. The complement protein is activated upon binding to the Fc region of the mutant polypeptide. In the present invention, the Fc region containing the mutation readily binds to the complement proteins (C1q or such) and thus can efficiently activate them. Perhaps because of this, even a small amount of the mutant polypeptide of the present invention can rapidly induce strong CDC.
The Fc region of the present invention preferably has a structure that enables effective mediation of the effector function (in particular, ADCC, CDC, and such). From this viewpoint, IgG Fc regions are commonly used as the Fc region for cysteine substitution (parental Fc region). Among them, human IgG Fc regions are preferred. More preferably, Fc regions of human IgGI, IgG2, IgG3, and IgG4, and the like are used. Since Fc regions of human IgG1 and IgG3 originally have ADCC, they are preferably used in the present invention. Meanwhile, the killing effect by CDC varies depending on the subclass, and Fc regions of human IgG1, human IgG4, and such can be used in the present invention. The nucleotide sequences of human Cγ1, Cγ2, Cγ3, and Cγ4 before cysteine substitution are shown in SEQ ID NOs: 103, 105, 107, and 109, respectively. The amino acid sequences of human Cγ1, Cγ2, Cγ3, and Cγ4 before cysteine substitution are also shown in SEQ ID NOs: 104, 106, 108, and 110, respectively.
All of the constant region sequences shown herein were designed according to Sequence of Proteins of Immunological Interest, 5th Ed. Public Health Service, National Institutes of Health, Bethesda, MD, 1991.

[Table 1]

| | IgG1(Cγ1) | | IgG2(Cγ2) | | IgG3(Cγ3) | | IgG4(Cγ4) | | |
|---|---|---|---|---|---|---|---|---|---|
| EU INDEX of Kabat | DNA sequence | Amino acid sequence | DNA sequence | Amino acid sequence | DNA sequence | Amino acid sequence | DNA sequence | Amino acid sequence | |
| 118 | gcc | Ala | gcc | Ala | gct | Ala | gct | Ala | Human IgG1 constant region(CH1) |
| 119 | tcc | Ser | tcc | Ser | tcc | Ser | tcc | Ser | |
| 120 | acc | Thr | acc | Thr | acc | Thr | acc | Thr | |
| 121 | aag | Lys | aag | Lys | aag | Lys | aag | Lys | |
| 122 | ggc | Gly | ggc | Gly | ggc | Gly | ggc | Gly | |
| 123 | cca | Pro | cca | Pro | cca | Pro | cca | Pro | |
| 124 | tcg | Ser | tcg | Ser | tcg | Ser | tcc | Ser | |
| 125 | gtc | Val | gtc | Val | gtc | Val | gtc | Val | |
| 126 | ttc | Phe | ttc | Phe | ttc | Phe | ttc | Phe | |
| 127 | ccc | Pro | ccc | Pro | ccc | Pro | ccc | Pro | |
| 128 | ctg | Leu | ctg | Leu | ctg | Leu | ctg | Leu | |
| 129 | gca | Ala | gcg | Ala | gcg | Ala | gcg | Ala | |
| 130 | ccc | Pro | ccc | Pro | ccc | Pro | ccc | Pro | |
| 131 | tcc | Ser | tgc | Cys | tgc | Cys | tgc | Cys | |
| 132 | tcc | Ser | tcc | Ser | tcc | Ser | tcc | Ser | |
| 133 | aag | Lys | agg | Arg | agg | Arg | agg | Arg | |
| 134 | agc | Ser | agc | Ser | agc | Ser | agc | Ser | |
| 135 | acc | Thr | acc | Thr | acc | Thr | acc | Thr | |
| 136 | tct | Ser | tcc | Ser | tct | Ser | tcc | Ser | |
| 137 | ggg | Gly | gag | Glu | ggg | Gly | gag | Glu | |
| 138 | ggc | Gly | agc | Ser | ggc | Gly | agc | Ser | |
| 139 | aca | Thr | aca | Thr | aca | Thr | aca | Thr | |
| 140 | gcg | Ala | gcc | Ala | gcg | Ala | gcc | Ala | |
| 141 | gcc | Ala | gcc | Ala | gcc | Ala | gcc | Ala | |
| 142 | ctg | Leu | ctg | Leu | ctg | Leu | ctg | Leu | |
| 143 | ggc | Gly | ggc | Gly | ggc | Gly | ggc | Gly | |
| 144 | tgc | Cys | tgc | Cys | tgc | Cys | tgc | Cys | |
| 145 | ctg | Leu | ctg | Leu | ctg | Leu | ctg | Leu | |
| 146 | gtc | Val | gtc | Val | gtc | Val | gtc | Val | |
| 147 | aag | Lys | aag | Lys | aag | Lys | aag | Lys | |
| 148 | gac | Asp | gac | Asp | gac | Asp | gac | Asp | |
| 149 | tac | Tyr | tac | Tyr | tac | Tyr | tac | Tyr | |
| 150 | ttc | Phe | ttc | Phe | ttc | Phe | ttc | Phe | |
| 151 | ccc | Pro | ccc | Pro | ccc | Pro | ccc | Pro | |
| 152 | gaa | Glu | gaa | Glu | gaa | Glu | gaa | Glu | |
| 153 | ccg | Pro | ccg | Pro | ccg | Pro | ccg | Pro | |
| 154 | gtg | Val | gtg | Val | gtg | Val | gtg | Val | |
| 155 | acg | Thr | acg | Thr | acg | Thr | acg | Thr | |
| 156 | gtg | Val | gtg | Val | gtg | Val | gtg | Val | |
| 157 | tcg | Ser | tcg | Ser | tcg | Ser | tcg | Ser | |
| 158 | tgg | Trp | tgg | Trp | tgg | Trp | tgg | Trp | |
| 159 | aac | Asn | aac | Asn | aac | Asn | aac | Asn | |
| 160 | tca | Ser | tca | Ser | tca | Ser | tca | Ser | |
| 161 | ggc | Gly | ggc | Gly | ggc | Gly | ggc | Gly | |
| 162 | gcc | Ala | gct | Ala | gcc | Ala | gcc | Ala | |
| 163 | ctg | Leu | ctg | Leu | ctg | Leu | ctg | Leu | |
| 164 | acc | Thr | acc | Thr | acc | Thr | acc | Thr | |
| 165 | agc | Ser | agc | Ser | agc | Ser | agc | Ser | |
| 166 | ggc | Gly | ggc | Gly | ggc | Gly | ggc | Gly | |
| 167 | gtg | Val | gtg | Val | gtg | Val | gtg | Val | |
| 168 | cac | His | cac | His | cac | His | cac | His | |

[Table 2]

| 169 | acc | Thr | acc | Thr | acc | Thr | acc | Thr |
|---|---|---|---|---|---|---|---|---|
| 170 | ttc | Phe | ttc | Phe | ttc | Phe | ttc | Phe |
| 171 | ccg | Pro | cca | Pro | ccg | Pro | ccg | Pro |
| 172 | gct | Ala | gct | Ala | gct | Ala | gct | Ala |
| 173 | gtc | Val | gtc | Val | gtc | Val | gtc | Val |
| 174 | cta | Leu | cta | Leu | cta | Leu | cta | Leu |
| 175 | cag | Gln | cag | Gln | cag | Gln | cag | Gln |
| 176 | tcc | Ser | tcc | Ser | tcc | Ser | tcc | Ser |
| 177 | tca | Ser | tca | Ser | tca | Ser | tca | Ser |
| 178 | gga | Gly | gga | Gly | gga | Gly | gga | Gly |
| 179 | ctc | Leu | ctc | Leu | ctc | Leu | ctc | Leu |
| 180 | tac | Tyr | tac | Tyr | tac | Tyr | tac | Tyr |
| 181 | tcc | Ser | tcc | Ser | tcc | Ser | tcc | Ser |
| 182 | ctc | Leu | ctc | Leu | ctc | Leu | ctc | Leu |
| 183 | agc | Ser | agc | Ser | agc | Ser | agc | Ser |
| 184 | agc | Ser | agc | Ser | agc | Ser | agc | Ser |
| 185 | gtg | Val | gtg | Val | gtg | Val | gtg | Val |
| 186 | gtg | Val | gtg | Val | gtg | Val | gtg | Val |
| 187 | acc | Thr | acc | Thr | acc | Thr | acc | Thr |
| 188 | gtg | Val | gtg | Val | gtg | Val | gtg | Val |
| 189 | ccc | Pro | ccc | Pro | ccc | Pro | ccc | Pro |
| 190 | tcc | Ser | tcc | Ser | tcc | Ser | tcc | Ser |
| 191 | agc | Ser | agc | Ser | agc | Ser | agc | Ser |
| 192 | agc | Ser | aac | Asn | agc | Ser | agc | Ser |
| 193 | ttg | Leu | ttc | Phe | ttg | Leu | ttg | Leu |
| 194 | ggc | Gly | ggc | Gly | ggc | Gly | ggc | Gly |
| 195 | acc | Thr | acc | Thr | acc | Thr | acg | Thr |
| 196 | cag | Gln | cag | Gln | cag | Gln | aag | Lys |
| 197 | acc | Thr | acc | Thr | acc | Thr | acc | Thr |
| 198 | tac | Tyr | tac | Tyr | tac | Tyr | tac | Tyr |
| 199 | atc | Ile | acc | Thr | acc | Thr | acc | Thr |
| 200 | tgc | Cys | tgc | Cys | tgc | Cys | tgc | Cys |
| 201 | aac | Asn | aac | Asn | aac | Asn | aac | Asn |
| 202 | gtg | Val | gta | Val | gtg | Val | gta | Val |
| 203 | aat | Asn | gat | Asp | aat | Asn | gat | Asp |
| 204 | cac | His | cac | His | cac | His | cac | His |
| 205 | aag | Lys | aag | Lys | aag | Lys | aag | Lys |
| 206 | ccc | Pro | ccc | Pro | ccc | Pro | ccc | Pro |
| 207 | agc | Ser | agc | Ser | agc | Ser | agc | Ser |
| 208 | aac | Asn | aac | Asn | aac | Asn | aac | Asn |
| 209 | acc | Thr | acc | Thr | acc | Thr | acc | Thr |
| 210 | aag | Lys | aag | Lys | aag | Lys | aag | Lys |
| 211 | gtg | Val | gtg | Val | gtg | Val | gtg | Val |
| 212 | gac | Asp | gac | Asp | gac | Asp | gac | Asp |
| 213 | aag | Lys | aag | Lys | aag | Lys | aag | Lys |
| 214 | aaa | Lys | aca | Thr | aga | Arg | aga | Arg |
| | | | gtt | Val | gtt | Val | gtt | Val |
| 215 | gca | Ala | | | | | | |
| 216 | gag | Glu | gag | Glu | gag | Glu | gag | Glu |
| 217 | ccc | Pro | cgc | Arg | ctc | Leu | tcc | Ser |
| 218 | aaa | Lys | aaa | Lys | aaa | Lys | aaa | Lys |
| 219 | tct | Ser | | | acc | Thr | tat | Tyr |
| 220 | tgt | Cys | | | cca | Pro | ggt | Gly |
| 221 | gac | Asp | tgt | Cys | ctt | Leu | | |
| | | | tgt | Cys | ggt | Gly | | |

constant region(Hing)

[Table 3]

| | | | | | gac | Asp | | | Human IgG1 |
|---|---|---|---|---|---|---|---|---|---|
| 222 | aaa | Lys | gtc | Val | aca | Thr | | | |
| 223 | act | Thr | | | act | Thr | | | |
| 224 | cac | His | gag | Glu | cac | His | ccc | Pro | |
| 225 | aca | Thr | | | aca | Thr | cca | Pro | |
| 226 | tgc | Cys | tgc | Cys | tgc | Cys | tgc | Cys | |
| 227 | cca | Pro | cca | Pro | cca | Pro | cca | Pro | |
| 228 | ccg | Pro | ccg | Pro | cgg | Arg | tca | Ser | |
| | | | | | tgc | Cys | | | |
| | | | | | cca | Pro | | | |
| | | | | | gag | Glu | | | |
| | | | | | ccc | Pro | | | |
| | | | | | aaa | Lys | | | |
| | | | | | tct | Ser | | | |
| | | | | | tgt | Cys | | | |
| | | | | | gac | Asp | | | |
| | | | | | aca | Thr | | | |
| | | | | | cct | Pro | | | |
| | | | | | ccc | Pro | | | |
| | | | | | ccg | Pro | | | |
| | | | | | tgc | Cys | | | |
| | | | | | cca | Pro | | | |
| | | | | | cgg | Arg | | | |
| | | | | | tgc | Cys | | | |
| | | | | | cca | Pro | | | |
| | | | | | gag | Glu | | | |
| | | | | | ccc | Pro | | | |
| | | | | | aaa | Lys | | | |
| | | | | | tct | Ser | | | |
| | | | | | tgt | Cys | | | |
| | | | | | gac | Asp | | | |
| | | | | | aca | Thr | | | |
| | | | | | cct | Pro | | | |
| | | | | | ccc | Pro | | | |
| | | | | | cca | Pro | | | |
| | | | | | tgc | Cys | | | |
| | | | | | cca | Pro | | | |
| | | | | | cgg | Arg | | | |
| | | | | | tgc | Cys | | | |
| | | | | | cca | Pro | | | |
| | | | | | gag | Glu | | | |
| | | | | | ccc | Pro | | | |
| | | | | | aaa | Lys | | | |
| | | | | | tct | Ser | | | |
| | | | | | tgt | Cys | | | |
| | | | | | gac | Asp | | | |
| | | | | | aca | Thr | | | |
| | | | | | cct | Pro | | | |
| | | | | | ccc | Pro | | | |
| | | | | | ccg | Pro | | | |
| | | | | | tgc | Cys | | | |
| | | | | | cca | Pro | | | |
| | | | | | agg | Arg | | | |
| 229 | tgc | Cys | tgc | Cys | tgc | Cys | tgc | Cys | |
| 230 | cca | Pro | cca | Pro | cca | Pro | cca | Pro | |

[Table 4]

| | | | | | | | | | |
|---|---|---|---|---|---|---|---|---|---|
| 231 | gca | Ala | gca | Ala | gca | Ala | gca | Ala | Human IgG1 constant region(CH2) |
| 232 | cct | Pro | cca | Pro | cct | Pro | cct | Pro | |
| 233 | gaa | Glu | cct | Pro | gaa | Glu | gag | Glu | |
| 234 | ctc | Leu | gtg | Val | ctc | Leu | ttc | Phe | |
| 235 | ctg | Leu | gca | Ala | ctg | Leu | ctg | Leu | |
| 236 | ggg | Gly | | | gga | Gly | ggg | Gly | |
| 237 | gga | Gly | gga | Gly | gga | Gly | gga | Gly | |
| 238 | ccg | Pro | ccg | Pro | ccg | Pro | cca | Pro | |
| 239 | tca | Ser | tca | Ser | tca | Ser | tca | Ser | |
| 240 | gtc | Val | gtc | Val | gtc | Val | gtc | Val | |
| 241 | ttc | Phe | ttc | Phe | ttc | Phe | ttc | Phe | |
| 242 | ctc | Leu | ctc | Leu | ctc | Leu | ctg | Leu | |
| 243 | ttc | Phe | ttc | Phe | ttc | Phe | ttc | Phe | |
| 244 | ccc | Pro | ccc | Pro | ccc | Pro | ccc | Pro | |
| 245 | cca | Pro | cca | Pro | cca | Pro | cca | Pro | |
| 246 | aaa | Lys | aaa | Lys | aaa | Lys | aaa | Lys | |
| 247 | ccc | Pro | ccc | Pro | ccc | Pro | ccc | Pro | |
| 248 | aag | Lys | aag | Lys | aag | Lys | aag | Lys | |
| 249 | gac | Asp | gac | Asp | gat | Asp | gac | Asp | |
| 250 | acc | Thr | acc | Thr | acc | Thr | act | Thr | |
| 251 | ctc | Leu | ctc | Leu | ctt | Leu | ctc | Leu | |
| 252 | atg | Met | atg | Met | atg | Met | atg | Met | |
| 253 | atc | Ile | atc | Ile | att | Ile | atc | Ile | |
| 254 | tcc | Ser | tcc | Ser | tcc | Ser | tcc | Ser | |
| 255 | cgg | Arg | cgg | Arg | cgg | Arg | cgg | Arg | |
| 256 | acc | Thr | acc | Thr | acc | Thr | acc | Thr | |
| 257 | cct | Pro | cct | Pro | cct | Pro | cct | Pro | |
| 258 | gag | Glu | gag | Glu | gag | Glu | gag | Glu | |
| 259 | gtc | Val | gtc | Val | gtc | Val | gtc | Val | |
| 260 | aca | Thr | acg | Thr | acg | Thr | acg | Thr | |
| 261 | tgc | Cys | tgc | Cys | tgc | Cys | tgc | Cys | |
| 262 | gtg | Val | gtg | Val | gtg | Val | gtg | Val | |
| 263 | gtg | Val | gtg | Val | gtg | Val | gtg | Val | |
| 264 | gtg | Val | gtg | Val | gtg | Val | gtg | Val | |
| 265 | gac | Asp | gac | Asp | gac | Asp | gac | Asp | |
| 266 | gtg | Val | gtg | Val | gtg | Val | gtg | Val | |
| 267 | agc | Ser | agc | Ser | agc | Ser | agc | Ser | |
| 268 | cac | His | cac | His | cac | His | cag | Gln | |
| 269 | gaa | Glu | gaa | Glu | gaa | Glu | gaa | Glu | |
| 270 | gac | Asp | gac | Asp | gac | Asp | gac | Asp | |
| 271 | cct | Pro | ccc | Pro | ccc | Pro | ccc | Pro | |
| 272 | gag | Glu | gag | Glu | gag | Glu | gag | Glu | |
| 273 | gtc | Val | gtc | Val | gtc | Val | gtc | Val | |
| 274 | aag | Lys | cag | Gln | cag | Gln | cag | Gln | |
| 275 | ttc | Phe | ttc | Phe | ttc | Phe | ttc | Phe | |
| 276 | aac | Asn | aac | Asn | aag | Lys | aac | Asn | |
| 277 | tgg | Trp | tgg | Trp | tgg | Trp | tgg | Trp | |
| 278 | tac | Tyr | tac | Tyr | tac | Tyr | tac | Tyr | |
| 279 | gtg | Val | gtg | Val | gtg | Val | gtg | Val | |
| 280 | gac | Asp | gac | Asp | gac | Asp | gat | Asp | |
| 281 | ggc | Gly | ggc | Gly | ggc | Gly | ggc | Gly | |
| 282 | gtg | Val | gtg | Val | gtg | Val | gtg | Val | |
| 283 | gag | Glu | gag | Glu | gag | Glu | gag | Glu | |
| 284 | gtg | Val | gtg | Val | gtg | Val | gtg | Val | |
| 285 | cat | His | cat | His | cat | His | cat | His | |

[Table 5]

| 286 | aat | Asn | aat | Asn | aat | Asn | aat | Asn | |
|---|---|---|---|---|---|---|---|---|---|
| 287 | gcc | Ala | gcc | Ala | gcc | Ala | gcc | Ala | |
| 288 | aag | Lys | aag | Lys | aag | Lys | aag | Lys | |
| 289 | aca | Thr | aca | Thr | aca | Thr | aca | Thr | |
| 290 | aag | Lys | aag | Lys | aag | Lys | aag | Lys | |
| 291 | ccg | Pro | cca | Pro | ccg | Pro | ccg | Pro | |
| 292 | cgt | Arg | cgg | Arg | cgg | Arg | cgg | Arg | |
| 293 | gag | Glu | gag | Glu | gag | Glu | gag | Glu | |
| 294 | gag | Glu | gag | Glu | gag | Glu | gag | Glu | |
| 295 | cag | Gln | cag | Gln | cag | Gln | cag | Gln | |
| 296 | tac | Tyr | ttc | Phe | tac | Tyr | ttc | Phe | |
| 297 | aac | Asn | aac | Asn | aac | Asn | aac | Asn | |
| 298 | agc | Ser | agc | Ser | agc | Ser | agc | Ser | |
| 299 | acg | Thr | acg | Thr | acg | Thr | acg | Thr | |
| 300 | tac | Tyr | ttc | Phe | ttc | Phe | tac | Tyr | |
| 301 | cgt | Arg | cgt | Arg | cgt | Arg | cgt | Arg | |
| 302 | gtg | Val | gtg | Val | gtg | Val | gtg | Val | |
| 303 | gtc | Val | gtc | Val | gtc | Val | gtc | Val | |
| 304 | agc | Ser | agc | Ser | agc | Ser | agc | Ser | |
| 305 | gtc | Val | gtc | Val | gtc | Val | gtc | Val | |
| 306 | ctc | Leu | ctc | Leu | ctc | Leu | ctc | Leu | |
| 307 | acc | Thr | acc | Thr | acc | Thr | acc | Thr | |
| 308 | gtc | Val | gtt | Val | gtc | Val | gtc | Val | |
| 309 | ctg | Leu | gtg | Val | ctg | Leu | ctg | Leu | |
| 310 | cac | His | cac | His | cac | His | cac | His | |
| 311 | cag | Gln | cag | Gln | cag | Gln | cag | Gln | |
| 312 | gac | Asp | gac | Asp | gac | Asp | gac | Asp | |
| 313 | tgg | Trp | tgg | Trp | tgg | Trp | tgg | Trp | |
| 314 | ctg | Leu | ctg | Leu | ctg | Leu | ctg | Leu | |
| 315 | aat | Asn | aac | Asn | aac | Asn | aac | Asn | |
| 316 | ggc | Gly | ggc | Gly | ggc | Gly | ggc | Gly | |
| 317 | aag | Lys | aag | Lys | aag | Lys | aag | Lys | |
| 318 | gag | Glu | gag | Glu | gag | Glu | gag | Glu | |
| 319 | tac | Tyr | tac | Tyr | tac | Tyr | tac | Tyr | |
| 320 | aag | Lys | aag | Lys | aag | Lys | aag | Lys | |
| 321 | tgc | Cys | tgc | Cys | tgc | Cys | tgc | Cys | |
| 322 | aag | Lys | aag | Lys | aag | Lys | aag | Lys | |
| 323 | gtc | Val | gtc | Val | gtc | Val | gtc | Val | |

(continued)

| | | | | | | | | | |
|---|---|---|---|---|---|---|---|---|---|
| 324 | tcc | Ser | tcc | Ser | tcc | Ser | tcc | Ser | |
| 325 | aac | Asn | aac | Asn | aac | Asn | aac | Asn | |
| 326 | aaa | Lys | aaa | Lys | aaa | Lys | aaa | Lys | |
| 327 | gcc | Ala | ggc | Gly | gcc | Ala | ggc | Gly | |
| 328 | ctc | Leu | ctc | Leu | ctc | Leu | ctc | Leu | |
| 329 | cca | Pro | cca | Pro | cca | Pro | ccg | Pro | |
| 330 | gcc | Ala | gcc | Ala | gcc | Ala | tcc | Ser | |
| 331 | ccc | Pro | ccc | Pro | ccc | Pro | tcc | Ser | |
| 332 | atc | Ile | atc | Ile | atc | Ile | atc | Ile | |
| 333 | gag | Glu | gag | Glu | gag | Glu | gag | Glu | |
| 334 | aaa | Lys | aaa | Lys | aaa | Lys | aaa | Lys | |
| 335 | acc | Thr | acc | Thr | acc | Thr | acc | Thr | |
| 336 | atc | Ile | atc | Ile | atc | Ile | atc | Ile | |
| 337 | tcc | Ser | tcc | Ser | tcc | Ser | tcc | Ser | |
| 338 | aaa | Lys | aaa | Lys | aaa | Lys | aaa | Lys | |
| 339 | gcc | Ala | acc | Thr | gcc | Thr | gcc | Ala | |
| 340 | aaa | Lys | aaa | Lys | aaa | Lys | aaa | Lys | |

[Table 6]

| 341 | ggg | Gly | ggg | Gly | gga | Gly | ggg | Gly | Human IgG1 constant region(CH3) |
|---|---|---|---|---|---|---|---|---|---|
| 342 | cag | Gln | cag | Gln | cag | Gln | cag | Gln | |
| 343 | ccc | Pro | ccc | Pro | ccc | Pro | ccc | Pro | |
| 344 | cga | Arg | cga | Arg | cga | Arg | cga | Arg | |
| 345 | gaa | Glu | gaa | Glu | gaa | Glu | gag | Glu | |
| 346 | cca | Pro | cca | Pro | cca | Pro | cca | Pro | |
| 347 | cag | Gln | cag | Gln | cag | Gln | cag | Gln | |
| 348 | gtg | Val | gtg | Val | gtg | Val | gtg | Val | |
| 349 | tac | Tyr | tac | Tyr | tac | Tyr | tac | Tyr | |
| 350 | acc | Thr | acc | Thr | acc | Thr | acc | Thr | |
| 351 | ctg | Leu | ctg | Leu | ctg | Leu | ctg | Leu | |
| 352 | ccc | Pro | ccc | Pro | ccc | Pro | ccc | Pro | |
| 353 | cca | Pro | cca | Pro | cca | Pro | cca | Pro | |
| 354 | tcc | Ser | tcc | Ser | tcc | Ser | tcc | Ser | |
| 355 | cgg | Arg | cgg | Arg | cgg | Arg | cag | Gln | |
| 356 | gat | Asp | gag | Glu | gag | Glu | gag | Glu | |
| 357 | gag | Glu | gag | Glu | gag | Glu | gag | Glu | |
| 358 | ctg | Leu | atg | Met | atg | Met | atg | Met | |
| 359 | acc | Thr | acc | Thr | acc | Thr | acc | Thr | |
| 360 | aag | Lys | aag | Lys | aag | Lys | aag | Lys | |
| 361 | aac | Asn | aac | Asn | aac | Asn | aac | Asn | |
| 362 | cag | Gln | cag | Gln | cag | Gln | cag | Gln | |
| 363 | gtc | Val | gtc | Val | gtc | Val | gtc | Val | |
| 364 | agc | Ser | agc | Ser | agc | Ser | agc | Ser | |
| 365 | ctg | Leu | ctg | Leu | ctg | Leu | ctg | Leu | |
| 366 | acc | Thr | acc | Thr | acc | Thr | acc | Thr | |
| 367 | tgc | Cys | tgc | Cys | tgc | Cys | tgc | Cys | |
| 368 | ctg | Leu | ctg | Leu | ctg | Leu | ctg | Leu | |
| 369 | gtc | Val | gtc | Val | gtc | Val | gtc | Val | |
| 370 | aaa | Lys | aaa | Lys | aaa | Lys | aaa | Lys | |
| 371 | ggc | Gly | ggc | Gly | ggc | Gly | ggc | Gly | |
| 372 | ttc | Phe | ttc | Phe | ttc | Phe | ttc | Phe | |
| 373 | tat | Tyr | tac | Tyr | tac | Tyr | tac | Tyr | |
| 374 | ccc | Pro | ccc | Pro | ccc | Pro | ccc | Pro | |
| 375 | agc | Ser | agc | Ser | agc | Ser | agc | Ser | |
| 376 | gac | Asp | gac | Asp | gac | Asp | gac | Asp | |
| 377 | atc | Ile | atc | Ile | atc | Ile | atc | Ile | |
| 378 | gcc | Ala | gcc | Ala | gcc | Ala | gcc | Ala | |
| 379 | gtg | Val | gtg | Val | gtg | Val | gtg | Val | |
| 380 | gag | Glu | gag | Glu | gag | Glu | gag | Glu | |
| 381 | tgg | Trp | tgg | Trp | tgg | Trp | tgg | Trp | |
| 382 | gag | Glu | gag | Glu | gag | Glu | gag | Glu | |
| 383 | agc | Ser | agc | Ser | agc | Ser | agc | Ser | |
| 384 | aat | Asn | aat | Asn | agc | Ser | aat | Asn | |
| 385 | ggg | Gly | ggg | Gly | ggg | Gly | ggg | Gly | |
| 386 | cag | Gln | cag | Gln | cag | Gln | cag | Gln | |
| 387 | ccg | Pro | ccg | Pro | ccg | Pro | ccg | Pro | |
| 388 | gag | Glu | gag | Glu | gag | Glu | gag | Glu | |
| 389 | aac | Asn | aac | Asn | aac | Asn | aac | Asn | |
| 390 | aac | Asn | aac | Asn | aac | Asn | aac | Asn | |
| 391 | tac | Tyr | tac | Tyr | tac | Tyr | tac | Tyr | |
| 392 | aag | Lys | aag | Lys | aag | Lys | aag | Lys | |
| 393 | acc | Thr | acc | Thr | acc | Thr | acc | Thr | |
| 394 | acg | Thr | aca | Thr | acg | Thr | acg | Thr | |
| 395 | cct | Pro | cct | Pro | cct | Pro | cct | Pro | |

[Table 7]

| | | | | | | | | | |
|---|---|---|---|---|---|---|---|---|---|
| 396 | ccc | Pro | ccc | Pro | ccc | Pro | ccc | Pro | |
| 397 | gtg | Val | atg | Met | atg | Met | gtg | Val | |
| 398 | ctg | Leu | ctg | Leu | ctg | Leu | ctg | Leu | |
| 399 | gac | Asp | gac | Asp | gac | Asp | gac | Asp | |
| 400 | tcc | Ser | tcc | Ser | tcc | Ser | tcc | Ser | |
| 401 | gac | Asp | gac | Asp | gac | Asp | gac | Asp | |
| 402 | ggc | Gly | ggc | Gly | ggc | Gly | ggc | Gly | |
| 403 | tcc | Ser | tcc | Ser | tcc | Ser | tcc | Ser | |
| 404 | ttc | Phe | ttc | Phe | ttc | Phe | ttc | Phe | |
| 405 | ttc | Phe | ttc | Phe | ttc | Phe | ttc | Phe | |
| 406 | ctc | Leu | ctc | Leu | ctc | Leu | ctc | Leu | |
| 407 | tac | Tyr | tac | Tyr | tac | Tyr | tac | Tyr | |
| 408 | agc | Ser | agc | Ser | agc | Ser | agc | Ser | |
| 409 | aag | Lys | aag | Lys | aag | Lys | agg | Arg | |
| 410 | ctc | Leu | ctc | Leu | ctc | Leu | cta | Leu | |
| 411 | acc | Thr | acc | Thr | acc | Thr | acc | Thr | |
| 412 | gtg | Val | gtg | Val | gtg | Val | gtg | Val | |
| 413 | gac | Asp | gac | Asp | gac | Asp | gac | Asp | |
| 414 | aag | Lys | aag | Lys | aag | Lys | aag | Lys | |
| 415 | agc | Ser | agc | Ser | agc | Ser | agc | Ser | |
| 416 | agg | Arg | agg | Arg | agg | Arg | agg | Arg | |
| 417 | tgg | Trp | tgg | Trp | tgg | Trp | tgg | Trp | |
| 418 | cag | Gln | cag | Gln | cag | Gln | cag | Gln | |
| 419 | cag | Gln | cag | Gln | cag | Gln | gag | Glu | |
| 420 | ggg | Gly | ggg | Gly | ggg | Gly | ggg | Gly | |
| 421 | aac | Asn | aac | Asn | aac | Asn | aat | Asn | |
| 422 | gtc | Val | gtc | Val | atc | Ile | gtc | Val | |
| 423 | ttc | Phe | ttc | Phe | ttc | Phe | ttc | Phe | |
| 424 | tca | Ser | tca | Ser | tca | Ser | tca | Ser | |
| 425 | tgc | Cys | tgc | Cys | tgc | Cys | tgc | Cys | |
| 426 | tcc | Ser | tcc | Ser | tcc | Ser | tcc | Ser | |
| 427 | gtg | Val | gtg | Val | gtg | Val | gtg | Val | |
| 428 | atg | Met | atg | Met | atg | Met | atg | Met | |
| 429 | cat | His | cat | His | cat | His | cat | His | |
| 430 | gag | Glu | gag | Glu | gag | Glu | gag | Glu | |
| 431 | gct | Ala | gct | Ala | gct | Ala | gct | Ala | |
| 432 | ctg | Leu | ctg | Leu | ctg | Leu | ctg | Leu | |
| 433 | cac | His | cac | His | cac | His | cac | His | |

(continued)

| 434 | aac | Asn | aac | Asn | aac | Asn | aac | Asn | |
|---|---|---|---|---|---|---|---|---|---|
| 435 | cac | His | cac | His | cgc | Arg | cac | His | |
| 436 | tac | Tyr | tac | Tyr | ttc | Phe | tac | Tyr | |
| 437 | acg | Thr | acg | Thr | acg | Thr | aca | Thr | |
| 438 | cag | Gln | cag | Gln | cag | Gln | cag | Gln | |
| 439 | aag | Lys | aag | Lys | aag | Lys | aag | Lys | |
| 440 | agc | Ser | agc | Ser | agc | Ser | agc | Ser | |
| 441 | ctc | Leu | ctc | Leu | ctc | Leu | ctc | Leu | |
| 442 | tcc | Ser | tcc | Ser | tcc | Ser | tcc | Ser | |
| 443 | ctg | Leu | ctg | Leu | ctg | Leu | ctg | Leu | |
| 444 | tct | Ser | tct | Ser | tct | Ser | tct | Ser | |
| 445 | ccg | Pro | ccg | Pro | ccg | Pro | ctg | Leu | |
| 446 | ggt | Gly | ggt | Gly | ggt | Gly | ggt | Gly | |
| 447 | aaa | Lys | aaa | Lys | aaa | Lys | aaa | Lys | |

However, the parental Fc region is not limited to the Fc regions of naturally occurring antibodies, and may be an Fc region that includes modifications such as deletion, addition, and substitution in an amino acid sequence, excluding the second amino acid from the glycosylation site to the N terminal side of a naturally occurring Fc region, or may be a synthetic polypeptide composed of the corresponding amino acid sequence. The substitution of a cysteine residue in the parental Fc region can be achieved by known methods using genetic recombination techniques.

The Fc region of the present invention includes a glycosylation site. However, a sugar chain is not necessarily linked to this glycosylation site. Nevertheless, at least one sugar chain is preferably linked at this site to enhance the ADCC. The sugar chain is not particularly limited, so long as it contains one or more monosaccharides. The sugar chain may be, for example, a sugar chain linked to the Fc region of an antibody heavy chain, its glycoform, or a synthetic sugar chain. The sugar chain represented by the following formula is an example of sugar chain linked to the glycosylation site in the Fc region of an IgG 1 antibody heavy chain:

GlcNAc(β 1,2) → Man α 1,6 ↘ (to Man)

Fuc(α 1,6) ↓ (to GlcNAc)

Man(β 1,4) → GlcNAc(β 1,4) → GlcNAc

GlcNAc(β 1,2) → Man α 1,3 ↗ (to Man)

In general, the structure of sugar chain linked to the Fc region is known to be closely involved in the effector function. Sugar chains obtained by appropriately modifying natural sugar chains are preferably used to improve the effector function. Examples of such sugar chains include sugar chains in which the fucose linked to the N-acetylglucosamine in the above formula includes modifications such as addition, deletion, or substitution. A sugar chain linked at the glycosylation site may be of an O- or N-linked type; however, N-linked sugar chains are preferably used.

In general, the sugar chain of human IgG antibody Fc region is very important for the ADCC of IgG antibody. The presence and structure of the sugar chain are known to be closely involved in ADCC. Furthermore, the amino acid sequence Asn-X-Ser/Thr (X represents an arbitrary amino acid residue) is necessary for the sugar chain linkage to the glycosylation site (in particular, for the expression of N-linked sugar chains). The mutant polypeptides of the present invention include an "Fc region having a substitution of a cysteine residue for the second amino acid from the glycosylation site to the N terminal side in the Fc region of IgG". Therefore, the amino acid sequence at the C terminal end which contains the glycosylation site of IgG Fc region (for example, the amino acid sequence Asn-Ser-Thr at positions 297 to

299) is conserved, and thus a sugar chain such as an N-linked sugar chain can be expressed at the glycosylation site. This may result in much improved ADCC. For example, when a mutation is introduced into the amino acid sequence containing the glycosylation site on the C terminal side in an IgG Fc region, the ADCC may be eliminated, or ADCC-improving effect may be decreased. Alternatively, when a cysteine residue is substituted for the first amino acid position from the glycosylation site to the N terminal side in the IgG Fc region, sufficient ADCC-improving effect cannot be obtained. This may be because a sugar chain is hardly linked to the glycosylation site, or even when linked to the site, the sugar chain conformation is not retained.

The mutant polypeptides of the present invention preferably contain, in addition to the IgG Fc region, a polypeptide domain that recognizes a cell surface molecule. The cell surface molecule may be a molecule that is present on the surface of human cells or nonhuman cells (mouse cells or such); however, human cell surface molecules are suitable.

Illustrative examples of human cell surface molecules include cytokine receptors, cell adhesion molecules, cancer cell surface molecules, cancer stem cell surface molecules, blood cell surface molecules, and surface molecules of virus-infected cells. Specific examples of human cell surface molecules include CD3, CD11a, CD20, CD22, CD25, CD28, CD33, CD52, Her2/neu, EGF receptor, EpCAM, MUC1, GD3, CEA, CA125, HLA-DR, TNFalpha receptor, VEGF receptor, CTLA-4, AILIM/ICOS, and integrin molecules.

The mutant polypeptides of the present invention can be constituted, for example, by a mutant antibody having an IgG Fc region, or a mutant polypeptide (antibody-like molecule) such as a chimeric molecule composed of an IgG Fc region and a polypeptide molecule that recognizes a cell surface molecule (in particular, human cell surface molecule). Such mutant antibody and antibody-like molecule may be used alone or in combination.

More specifically, the mutant polypeptides of the present invention include mutant antibodies containing a substitution of a cysteine residue for an amino acid at EU index 295 according to the Kabat numbering system, and mutant chimeric molecules composed of a cell surface molecule recognition site and an Fc region having a substitution of a cysteine residue for an amino acid at EU index 295 according to the Kabat numbering system. The mutant polypeptides of the present invention have improved effector functions as compared to those before the substitution. The Fc region in a mutant polypeptide of the present invention includes human IgG Fc regions, preferably Fc regions of human IgGI, IgG2, IgG3, and IgG4. As described above, the Fc region of the present invention preferably has an N-linked sugar chain at the glycosylation site.

The antibodies of the present invention are not particularly limited, so long as they are molecules having an antigen-binding site and capable of inducing the effector functions (in particular, ADCC). Examples of such antibodies include natural antibodies such as human antibodies and nonhuman antibodies, such as mouse antibodies; derivatives thereof; and recombinant antibodies, such as chimeric antibodies, humanized antibodies, and single-chain antibodies, capable of undergoing an antigen-antibody reaction. These antibodies may be monoclonal or polyclonal antibodies, and may be used alone or in combination. The derivatives of natural antibodies refer to antibodies having mutations such as deletion, addition, and substitution in a portion of the amino acid sequence of a natural antibody. Such mutations may be spontaneous or artificial. The antigen-binding site includes variable region VH and VL (in particular, hypervariable region CDR1 to 3) in the Fab region of a natural antibody and domains homologous thereto.

Of them, recombinant antibodies, chimeric antibodies, humanized antibodies, and the like in particular are preferably used, because they exert preferred ADCC and thus are suitable as antibody pharmaceuticals. The chimeric antibody refers to an antibody composed of a homologous or heterologous fusion protein and includes, for example, antibodies constituted by a human IgG Fc region and an Fab region of a nonhuman IgG (mouse IgG or such). The humanized antibody includes, for example, antibodies resulting from fusing the hypervariable region of a nonhuman IgG (mouse IgG or such) into the remaining portion containing the Fc region derived from human IgG.

It is possible to use antibodies against various antigens. Antigens recognized by such antibodies include, for example, polypeptides such as receptors including transmembrane molecules and ligands including growth factors; and non-polypeptide antigens described in U.S. Patent No. 5091178 such as tumor-related glycolipid antigens. Specific examples of such antigens include rennin; growth hormones including human and bovine growth hormones; growth hormone-releasing factor; parathyroid hormone; thyroid hormones; lipoproteins; α-1-antitrypsin; insulin A chain; insulin B chain; proinsulin; follicle-stimulating hormone; calcitonin; luteinizing hormone; glucagon; coagulation factors such as factor VIIIC, factor IX, tissue factor, and von Willebrand's factor; anticoagulation factors such as protein C; atrial natriuretic factor; pulmonary surfactants; plasminogen activators such as urokinase, human urinary plasminogen activator, and tissue plasminogen activator (t-PA); bombesin; thrombin; hematopoietic growth factor; tumor necrosis factor-α and -β; enkephalinase; regulated on activation normally T-cell expressed and secreted (RANTES); human macrophage inflammatory protein (MIP-1-α); serum albumins such as human serum albumin; Müllerian inhibitory substance; relaxin A chain; relaxin B chain; prorelaxin; mouse gonadotropin-related peptides; proteins of microorganisms, such as beta-lactamase; DNase; IgE; cytotoxic T lymphocyte associated antigens (CTLA) such as CTLA-4; inhibin; activin; vascular endothelial growth factor (VEGF); hormone receptors and growth factor receptors; protein A and protein D; rheumatoid factor; nerve growth factors such as brain-derived neurotrophic factor (BDNF), neurotrophin-3, -4, -5, and -6 (N-3, NT-4, NT-5, and NT-6), and NGF-β; platelet-derived growth factor (PDGF); fibroblast growth factors such as aFGF and

bFGF; epidermal growth factor (EGF); transforming growth factors (TGFs) such as TGF-α and EGF-β (TGF-β1, EGF-β2, EGF-β3, TGF-β4, EGF-β5, and so on); insulin-like growth factor-I and -II (IGF-1 and IGF-II); des(1-3)-IGF-I (brain IGF-I); insulin-like growth factor-binding proteins; CD proteins such as CD3, CD4, CD8, CD19, and CD20; erythropoietin; osteoinductive factor; immunotoxins; bone morphogenetic proteins (BMPs); interferons such as interferon-α, -β, and -γ; colony stimulating factors (CSFs) such as M-CSF, GM-CSF, and G-CSF; interleukins (ILs) such as IL-1 to IL-10; superoxide dismutase; T cell receptor; cell surface membrane proteins; decay accelerating factor; viral antigens, for example, components of AIDS envelope; transporter proteins; homing receptors; addressin; regulatory proteins; integrins such as CD11a, CD11b, CD11c, CD 18, ICAM, VLA-4, and VCAM; tumor-associated antigens such as HER2, HER3, and HER4 receptors; and fragments of the polypeptides listed above.

Preferred molecular targets of antibodies include, for example, CD proteins such as CD3, CD4, CD8, CD 19, CD20, and CD34; ErbB receptor family such as HER2, HER3, and HER4 receptors; cell adhesion molecules such as LFA-1, Mac1, p150.95, VLA-4, ICAM-1, VCAM, and αv/β3 integrins that contain any of the α and β subunits thereof (for example, anti-CD 11a, anti-CD 18, and anti-CD 11b antibodies); growth factors such as VEGF; IgE; blood group antigens; flk2/flt3 receptors; obesity (OB) receptor; mpl receptor; CTLA-4; and protein C.

Of the illustrative antigens listed above, soluble antigens and fragments thereof can be used as immunogens to produce antibodies. For example, when transmembrane molecules such as receptors are used, for example, fragments of extracellular domains can serve as immunogens. Alternatively, it is possible to use cells expressing transmembrane molecules as an immunogen. Such cells can be isolated from natural sources (for example, cancer cell lines), or may be cells which have been transformed with recombinant vectors so that they express antigen region to be recognized of a transmembrane molecule or the like.

The antibodies may contain a domain capable of binding with cell surface molecules, in addition to the antigen-binding site. Because of improved efficiency of target cell recognition, antibodies having such a structural feature can exert the effector function with high efficiency even at a small dose.

The present invention provides, for example, the following mutant anti-CD20 antibodies:

(1) mutant antibodies containing an H chain that contains a CDR1 having the amino acid sequence of SEQ ID NO: 54, a CDR2 having the amino acid sequence of SEQ ID NO: 56, a CDR3 having the amino acid sequence of SEQ ID NO: 58, and a CH having the amino acid sequence of SEQ ID NO: 60;
(2) mutant antibodies containing an H chain having the amino acid sequence of SEQ ID NO: 52 (the full-length H chain amino acid sequence);
(3) mutant antibodies containing an H chain that contains a CDR1 having the amino acid sequence of SEQ ID NO: 54, a CDR2 having the amino acid sequence of SEQ ID NO: 56, a CDR3 having the amino acid sequence of SEQ ID NO: 58, and an Fc region having the amino acid sequence of SEQ ID NO: 72; and
(4) mutant antibodies containing both an H chain of any one of (1), (2), and (3) described above and an L chain of (i) or (ii) described below;

(i) an L chain that contains a CDR1 having the amino acid sequence of SEQ ID NO: 64, a CDR2 having the amino acid sequence of SEQ ID NO: 66, a CDR3 having the amino acid sequence of SEQ ID NO: 68, and a CL having the amino acid sequence of SEQ ID NO: 70;
(ii) an L chain having the amino acid sequence of SEQ ID NO: 62 (the full-length L chain amino acid sequence).

Furthermore, the present invention also provides the following mutant anti-EGFR antibodies:

(1) mutant antibodies containing an H chain that contains a CDR1 having the amino acid sequence of SEQ ID NO: 73, a CDR2 having the amino acid sequence of SEQ ID NO: 75, a CDR3 having the amino acid sequence of SEQ ID NO: 77, and a CH having the amino acid sequence of SEQ ID NO: 60;
(2) mutant antibodies containing an H chain having the amino acid sequence of SEQ ID NO: 87 (the full-length H chain amino acid sequence);
(3) mutant antibodies containing an H chain that contains a CDR1 having the amino acid sequence of SEQ ID NO: 73, a CDR2 having the amino acid sequence of SEQ ID NO: 75, a CDR3 having the amino acid sequence of SEQ ID NO: 77, and an Fc region having the amino acid sequence of SEQ ID NO: 72; and
(4) mutant antibodies containing both an H chain of any one of (1), (2), and (3) described above and an L chain of (i) or (ii) described below;

(i) an L chain containing a CDR having the amino acid sequence of SEQ ID NO: 79, a CDR2 having the amino acid sequence of SEQ ID NO: 81, a CDR3 having the amino acid sequence of SEQ ID NO: 83, and a CL having the amino acid sequence of SEQ ID NO: 85;
(ii) an L chain having the amino acid sequence of SEQ ID NO: 89 (the full-length L chain amino acid sequence).

The present invention provides, for example, the following mutant anti-CD20 antibodies:

(1) mutant antibodies containing an H chain that contains a CDR1 encoded by the nucleotide sequence of SEQ ID NO: 53, a CDR2 encoded by the nucleotide sequence of SEQ ID NO: 55, a CDR3 encoded by the nucleotide sequence of SEQ ID NO: 57, and a CH encoded by the nucleotide sequence of SEQ ID NO: 59;
(2) mutant antibodies containing an H chain encoded by the nucleotide sequence of SEQ ID NO: 51;
(3) mutant antibodies containing an H chain that contains a CDR1 encoded by the nucleotide sequence of SEQ ID NO: 53, a CDR2 encoded by the nucleotide sequence of SEQ ID NO: 55, a CDR3 encoded by the nucleotide sequence of SEQ ID NO: 57, and an Fc region encoded by the nucleotide sequence of SEQ ID NO: 71; and
(4) mutant antibodies containing both an H chain of any one of (1), (2), and (3) described above and an L chain of (i) or (ii) described below;

(i) an L chain containing a CDR1 encoded by the nucleotide sequence of SEQ ID NO: 63, a CDR2 encoded by the nucleotide sequence of SEQ ID NO: 65, a CDR3 encoded by the nucleotide sequence of SEQ ID NO: 67, and a CL encoded by the nucleotide sequence of SEQ ID NO: 69;
(ii) an L chain encoded by the nucleotide sequence of SEQ ID NO: 61.

Furthermore, the present invention provides the following mutant anti-EGFR antibodies:

(1) mutant antibodies containing an H chain that contains a CDR1 encoded by the nucleotide sequence of SEQ ID NO: 74, a CDR2 encoded by the nucleotide sequence of SEQ ID NO: 76, a CDR3 encoded by the nucleotide sequence of SEQ ID NO: 78, and a CH encoded by the nucleotide sequence of SEQ ID NO: 59;
(2) mutant antibodies containing an H chain encoded by the nucleotide sequence of SEQ ID NO: 88;
(3) mutant antibodies containing an H chain that contains a CDR1 encoded by the nucleotide sequence of SEQ ID NO: 74, a CDR2 encoded by the nucleotide sequence of SEQ ID NO: 76, a CDR3 encoded by the nucleotide sequence of SEQ ID NO: 78, and an Fc region encoded by the nucleotide sequence of SEQ ID NO: 71; and
(4) mutant antibodies containing both an H chain of any one of (1), (2), and (3) described above and an L chain of (i) or (ii) described below;

(i) an L chain containing a CDR1 encoded by the nucleotide sequence of SEQ ID NO: 80, a CDR2 encoded by the nucleotide sequence of SEQ ID NO: 82, a CDR3 encoded by the nucleotide sequence of SEQ ID NO: 84, and a CL encoded by the nucleotide sequence of SEQ ID NO: 86;
(ii) an L chain encoded by the nucleotide sequence of SEQ ID NO: 90.

The antibody-like molecules of the present invention differ from antibodies in that they lack the antigen-binding site. However, the antibody-like molecules of the present invention are functionally similar to antibodies in that they have an intramolecular domain that recognizes a cell surface molecule and can contribute to the achievement of effector functions via binding of the domain to the cell surface molecule.

In the context of the present invention, examples of antibody-like molecules include recombinant molecules such as chimeric molecules containing an Fc region and a domain that recognizes a cell surface molecule. In particular, the present invention provides mutant chimeric molecules that include a cell surface molecule recognition site and an Fc region having a substitution of a cysteine residue for an amino acid at EU index 295 according to the Kabat numbering system. The mutant chimeric molecules of the present invention may include a peptide region between the cell surface molecule recognition site and the Fc region having a substitution of a cysteine residue for an amino acid at EU index 295 according to the Kabat numbering system, as long as they have the effector function. Illustrative examples of such peptide regions include, but are not limited to, (1) linkers, (2) antibody hinge regions, and (3) linkers and antibody hinge regions. The "antibody hinge region" includes not only full-length antibody hinge regions but also portions of the hinge regions as long as they contribute to (or do not inhibit) the effector function.

The cell surface molecules are as exemplified above. In particular, the present invention provides mutant chimeric molecules that bind to at least one human cell surface molecule selected from the group consisting of cytokine receptors, cell adhesion molecules, cancer cell surface molecules, cancer stem cell surface molecules, blood cell surface molecules, and surface molecules of virus-infected cells. The domain that recognizes a cell surface molecule may be constituted, for example, by a binding domain of an adhesion protein. Alternatively, the domain that recognizes a cell surface molecule may be constituted, for example, by an antibody H chain and/or L chain variable regions.

The technique of the present invention can be applied to molecules composed of an antibody Fc region and a domain that binds to an antigen, like an antigen recognition site of an antibody, and having the effector function. Accordingly, the applicable target of the technique of the present invention is not limited to antibody molecules. The technique of the present invention is applicable to general chimeric molecules that contain an antibody Fc region and a cell surface

molecule recognition site (for example, a binding domain such as an adhesion protein, adhesion molecule, and signaling molecule). Herein, the adhesion protein refers, for example, to a molecule that mediates intercellular interaction and structurally contains an extracellular domain that serves as a cell recognition site. An antibody-like molecule of the present invention uses the extracellular domain structure as the binding domain, and thus the molecule can recognize the cell surface molecule to which the molecule originally binds.

Illustrative examples of adhesion protein include extracellular domains of cell surface molecules such as immunoglobulin superfamily (IgSF), cytokine receptors such as receptors having tyrosin kinase activity (receptor tyrosin kinases), hemopoietic and nerve growth factor receptor superfamily, integrins, cadherins, and (E-, L-, and P-) selectins; and polypeptide molecules that have affinity for (can bind to) these domains. The term "IgSF" encompasses not only transmembrane cell adhesion molecules such as NCAM and L1 but also GPI anchor cell adhesion molecules without transmembrane domain.

More specifically, the present invention provides mutant chimeric molecules that bind to at least one human cell surface molecule selected from the group consisting of CD3, CD 11 a, CD20, CD22, CD25, CD28, CD33, CD52, Her2/neu, EGF receptor, EpCAM, MUC1, GD3, CEA, CA125, HLA-DR, TNFalpha receptor, VEGF receptor, AILIM/ICOS, CTLA-4, B7h, CD80, CD86, and integrin molecules.

Antibody-like molecules of the present invention and mutants thereof preferably have a structure wherein the binding domain (herein also referred to as a "cell surface molecule recognition site") is situated on the N terminal side and the Fc region is placed on the C terminal side. However, the structural arrangement is not so limited, and includes a structure wherein the Fc region is situated on the N terminal side and the binding domain is placed on the C terminal side. The Fc region constituting the antibody-like molecules and mutants thereof preferably contains CH2 and CH3 domains. The binding domain constituting the antibody-like molecules and mutants thereof is preferably arranged at a position corresponding to the position of CH1 of antibody heavy chain and/or antibody light chain.

The present invention provides the following mutant chimeric molecules:

(1) mutant chimeric molecules having the amino acid sequence of SEQ ID NO: 96 as a cell surface molecule recognition site, and the amino acid sequence of SEQ ID NO: 97 as an Fc region containing a substitution of a cysteine residue for an amino acid at EU index 295 according to the Kabat numbering system;
(2) mutant chimeric molecules having the amino acid sequence of SEQ ID NO: 96 as a cell surface molecule recognition site, the amino acid sequence of SEQ ID NO: 98 as an antibody hinge region, and the amino acid sequence of SEQ ID NO: 97 as an Fc region containing a substitution of a cysteine residue for an amino acid at EU index 295 according to the Kabat numbering system; and
(3) the mutant chimeric molecule having the amino acid sequence of SEQ ID NO: 99.

Furthermore, the present invention provides the following mutant chimeric molecules:

(1) mutant chimeric molecules containing a cell surface molecule recognition site encoded by the nucleotide sequence of SEQ ID NO: 91, and an Fc region encoded by the nucleotide sequence of SEQ ID NO: 92, which contains a substitution of a cysteine residue for an amino acid at EU index 295 according to the Kabat numbering system;
(2) mutant chimeric molecules containing a cell surface molecule recognition site encoded by the nucleotide sequence of SEQ ID NO: 91, an antibody hinge region encoded by the nucleotide sequence of SEQ ID NO: 93, and an Fc region encoded by the nucleotide sequence of SEQ ID NO: 92, which contains a substitution of a cysteine residue for an amino acid at EU index 295 according to the Kabat numbering system; and
(3) the mutant chimeric molecule encoded by the nucleotide sequence of SEQ ID NO: 94.

The chimeric molecules of the present invention may further include a linker sequence between the antibody hinge region and the cell surface molecule recognition site. Such linker sequences are not particularly limited, so long as they contribute to (or do not inhibit) the desired effector function. Illustrative examples of suitable linker sequences include linker sequences comprising the amino acid sequence of SEQ ID NO: 100. DNAs encoding the amino acid sequence of SEQ ID NO: 100 include, for example, DNAs having the nucleotide sequence of SEQ ID NO: 95, but are not limited thereto. The "antibody hinge region" of the present invention includes not only full-length antibody hinge regions but also partial antibody hinge regions, so long as they contribute to (or do not inhibit) the effector function.

The mutant polypeptides of the present invention can be produced by known methods using genetic recombination techniques. The mutant polypeptides of the present invention can be produced using the following procedure. A vector is constructed so as to contain an isolated nucleic acid encoding the mutant polypeptide of the present invention. Host cells or host organisms are transformed with this vector, and the host cells or host organisms are cultured to express the polypeptide encoded by the nucleic acid.

Conventional methods for producing antibodies can be used when the mutant polypeptides are constituted by antibodies. The mutant antibodies can be produced by methods described in Molecular Cloning 2nd Ed.; Current Protocols

in Molecular Biology; Antibodies, A Laboratory manual, Cold Spring Harbor Laboratory, 1988; Monoclonal Antibodies: principles and practice, 3rd Ed., Acad. Press, 1993; and Antibody Engineering, A Practical Approach, IRL Press at Oxford University Press, 1996. The mutant antibodies can be obtained, for example, by expressing them in host cells as described below. The same methods can also be used to produce the antibody-like molecules.

The production of the mutant polypeptides includes, for example, the steps of isolating a nucleic acid encoding a polypeptide containing the parental Fc region, whose sequence includes a substitution whereby it encodes a cysteine residue instead of the second amino acid from the glycosylation site to the N terminal side in the IgG Fc region; producing a replicative recombinant vector by inserting the isolated nucleic acid encoding the mutant polypeptide into an expression vector having an appropriate promoter; transforming host cells or host organisms with the resulting recombinant vector; and producing the mutant polypeptide by culturing the transformed host cells or host organisms.

Such polypeptides containing the parental Fc region may contain modifications such as deletion, addition, and substitution in amino acids other than the second amino acid from the glycosylation site to the N terminal side in the IgG Fc region or sugar chain linked to the glycosylation site.

Nucleic acids encoding the mutant polypeptides of the present invention can be obtained by conventional methods for introducing mutations into nucleotide sequences. Such nucleic acids can be prepared, for example, by PCR using as a template a cDNA containing the parental Fc region and a synthetic oligonucleotide having a nucleotide sequence that includes a substitution of "TGC" or "TCT" for a nucleotide sequence encoding the original amino acid residue to be replaced in the parental Fc region. The mutant polypeptides of the present invention may be further modified in the step of or after mutagenesis for cysteine substitution, so long as such modifications do not inhibit the effector function. For example, some portions of amino acid sequences or sugar chains linked to the glycosylation site may contain modifications such as deletion, addition, and substitution. In particular, the present invention is advantageous in that the effector function can be improved by altering the sugar chain linked to the glycosylation site of the Fc region through known modifications.

The replicative recombinant vector can be constructed by inserting a nucleic acid (DNA fragment or full-length cDNA) encoding the mutant polypeptide of the present invention downstream of a promoter in an appropriate expression vector. Such vectors can be appropriately selected depending on the type of host cells or host organisms to be introduced. The vectors used are self-replicative ones or those allow integration into the chromosome, and have a promoter capable of transcribing a nucleic acid encoding a mutant polypeptide of interest. Such recombinant vectors may be used alone or in combination.

In the present invention, two or more recombinant vectors may be used in combination. Such recombinant vectors used in combination include, for example, expression vectors having an appropriate promoter, which carry a complementary sequence of a nucleic acid encoding an antibody light chain or heavy chain, desired receptor or ligand-binding domain, desired sugar chain modification enzyme, or such. The combined use of these recombinant vectors allows for the production of mutant polypeptides that enable achievement of the effector function with higher efficiency. When two or more recombinant vectors are used, the host cells or host organisms may be different or the same.

The host cells include yeast, animal cells, insect cells, and plant cells, so long as they can express genes of interest. Furthermore, in the context of the present invention, it is possible to select host cells in which the activity of modification enzymes that act on the sugar chain linked to the glycosylation site, which affect the effector function, has been eliminated or reduced, or it is possible to use host cells in which such activity has been eliminated or reduced by artificial methods. Such enzymes include those involved in modification of N-glycoside linked sugar chains and the like. Specifically, the enzymes include those involved in synthesis of the intracellular nucleotide sugar GDP-fucose, those involved in sugar chain modification of $\alpha$ linkage formed between position 1 of fucose and position 6 of N-acetylglucosamine at the reducing end of an N-glycoside-linked complex-type sugar chain, and such. The host organisms include, for example, animals and plants.

When yeast is used as the host cells, YEP13 (ATCC37115), YEp24 (ATCC37051), YCp50 (ATCC37419), and the like can be used as the expression vector. Any promoters may be used so long as they allow for the expression in cells of yeast strains. Illustrative examples of suitable promoters include promoters of glycolytic genes such as hexokinase, PHO5 promoter, PGK promoter, GAP promoter, ADH promoter, gal1 promoter, gal10 promoter, heat shock protein promoter, MF$\alpha$1 promoter, and CUP 1 promoter. The host cells include microorganisms belonging to the genera *Saccharomyces, Schizosaccharomyces, Kluyveromyces, Trichosporon,* and *Schwanniomyces;* for example, *Saccharomyces cerevisiae, Schizosaccharomyces pombe, Kluyveromyces lactis, Trichosporon pullulans,* and *Schwanniomyces alluvius.*

Any method for introducing DNA into yeast can be used to introduce the recombinant vectors into yeast. Such methods include, for example, electroporation (Methods. Enzymol., 194,182 (1990)), spheroplast method (Proc. Natl. Acad. Sci. U.S.A), 84, 1929 (1978)), lithium acetate method (J. Bacteriology, 153, 163 (1983); Proc. Natl. Acad. Sci. U.S.A, 75, 1929 (1978)).

When animal cells are used as the host, the expression vectors used may be, for example, pcDNAI, pcDM8 (available from Funakoshi), pAGE107 (JP-A (Kokai) H03-22979; Cytotechnology, 3, 133 (1990)), pAS3-3 (JP-A (Kokai)

H02-227075), pCDM8 (Nature, 329, 840 (1987)), pcDNAI/Amp (Invitrogen), pREP4 (Invitrogen), pAGE103 (J. Biochemistry, 101, 1307 (1987)), and pAGE210. Any prompter can be used so long as it enables the expression in animal cells. Illustrative examples of suitable promoter include cytomegalovirus (CMV) immediate early (IE) gene promoter, SV40 early promoter, retroviral promoter, metallothionein promoter, heat shock promoter, and SR$\alpha$ promoter. Furthermore, human CMV IE gene enhancer may be used in combination with such promoters. The host cells include, for example, Namalwa cells (human cell line), COS cells (monkey cell line), CHO cells (Chinese hamster cell line), HBT5637 (JP-A (Kokai) S63-299), rat myeloma cells, mouse myeloma cells, Syrian hamster kidney cells, embryonic stem cells, and cells of fertilized eggs.

Any method for introducing DNA into animal cells can be used to introduce the recombinant vectors into animal cells. Such methods include, for example, electroporation (Cytotechnology, 3, 133 (1990)), calcium phosphate method (JP-A (Kokai) H02-227075), lipofection method (Proc. Natl. Acad. Sci. U.S.A., 84, 7413 (1987)), injection method (Manipulating the Mouse Embryo, A Laboratory Manual), methods using particle gun (gene gun) ((Granted/ Registered) Japanese Patent No. 2606856; (Granted/Registered) Japanese Patent No. 2517813), DEAE-Dextran method (Bio Manual Series Vol. 4, Idenshidohnyu to Hatsugen/Kaiseki Hoh (Gene transfer and Method of Expression Analysis), Yodosha, Japan, Eds., Takashi Yokota and Kenichi Arai, 1994), and viral vector method (Manipulating the Mouse Embryo, 2nd Ed.).

When insect cells are used as the host, proteins can be expressed, for example, by the methods described in Current Protocols in Molecular Biology; Baculovirus Expression Vectors, A Laboratory Manual, W. H. Freeman and Company, New York, 1992 and Bio/Technology, 6, 47 (1988). Specifically, proteins can be expressed by co-introducing recombinant gene transfer vectors and baculovirus into insect cells, and then infecting insect cells with the obtained recombinant virus in the culture supernatant of the insect cells. Gene transfer vectors used in this method include, for example, pVL1392, pVL1393, and pBlueBacIII (all from Invitrogen).

Such baculovirus includes, for example, *Autographa californica* nuclear polyhedrosis virus, which is a virus that infect insects of Mamestra. The insect cells include Sf9 and Sf21, which are ovary cells of *Spodopterafrugiperda* (Current Protocols in Molecular Biology; Baculovirus Expression Vectors, A Laboratory Manual, W. H. Freeman and Company, New York, 1992), and High5, an ovary cell of *Trichoplusiani* (Invitrogen).

Methods for co-introducing the gene transfer vectors and baculovirus into insect cells to prepare recombinant virus include, for example, the calcium phosphate method (JP-A (Kokai) H02-227075) and lipofection method (Proc. Natl. Acad. Sci. U.S.A., 84, 7413 (1987)).

When plant cells are used as the host cells, expression vectors such as Ti plasmid and tobacco mosaic virus can be used. Any promoter can be used as long as it enables the expression in plant cells. Such examples include the 35S promoter of cauliflower mosaic virus (CaMV) and rice actin 1 promoter. The host cells include plant cells such as tobacco, potato, tomato, carrot, soybean, oilseed rape, alfalfa, rice plant, wheat, and barley.

Any method for introducing DNA into plant cells can be used to introduce recombinant vectors into plant cells. Such methods include, for example, methods using Agrobacterium (JP-A (Kokai) S59-140885; JP-A (Kokai) S60-70080; WO94/00977), electroporation (JP-A (Kokai) S60-251887), and particle gun (gene gun) ((Granted/Registered) Japanese Patent No. 2606856; (Granted/Registered) Japanese Patent No. 2517813). Genes may be directly expressed, or can be secreted or expressed as fusion proteins between Fc region and other proteins according to methods described in Molecular Cloning 2nd Ed., and so on.

As described above, mutant antibodies and mutant antibody-like molecules (mutant polypeptides) can be produced and accumulated in cell culture by culturing the transformants introduced with one or more recombinant vectors in media suitable for each host by known methods.

Media that are used to culture transformants obtained by using as hosts prokaryotes such as *E. coli* or eukaryotes such as yeast include both natural media and synthetic media, so long as they contain a carbon source that can be assimilated by the organisms, a nitrogen source, inorganic salts, and the like and allow for the efficient culture of the transformants. Any carbon sources may be used, so long as they can be assimilated by the microorganisms. Illustrative examples of suitable carbon sources include glucose, fructose, sucrose, molasses containing them, carbohydrates such as starch and starch hydrolysates, organic acids such as acetic acid and propionic acid, and alcohols such as ethanol and propanol. Examples of suitable nitrogen sources include ammonia, ammonium chloride, ammonium salts of inorganic and organic acids such as ammonium sulfate, ammonium acetate, and ammonium phosphate, other nitrogen-containing compounds, peptone, meat extract, yeast extract, corn steep liquor, casein hydrolysate, soybean cake and its hydrolysate, and various fermentative bacterial cells and digests thereof. Examples of suitable inorganic salts include monopotassium phosphate, dipotassium phosphate, magnesium phosphate, magnesium sulfate, sodium chloride, ferrous sulfate, manganese sulfate, copper sulfate, and calcium carbonate.

In general, transformants obtained using as hosts prokaryotes such as E. *coli* and eukaryotes such as yeast are cultured under aerobic conditions, such as shaking culture or stirring culture with deep aeration. The preferred culture temperature ranges from 15°C to 40°C. In general, the culture period is 16 hours to 7 days. The pH is maintained at 3.0 to 9.0 during culture. The pH may be adjusted using an inorganic or organic acid, alkaline solution, urea, calcium carbonate, ammonia, and the like. If necessary, antibiotics such as ampicillin and tetracycline may be added to the

medium during culture. When microorganisms transformed with a recombinant vector having an inducible promoter are cultured, an inducer may be added to the medium if needed. For example, when microorganisms transformed with a recombinant vector having the lac promoter are cultured, isopropyl-β-D-thiogalactopyranoside or the like may be added to the medium. When microorganisms transformed with a recombinant vector having the trp promoter are cultured, indoleacrylic acid or the like may be added to the medium.

Media that are used to culture transformants obtained by using animal cell hosts include conventional media such as RPMI1640 (The Journal of the American Medical Association, 199, 519 (1967)), Eagle's MEM (Science, 122, 501 (1952)), Dulbecco's modified MEM (Virology, 8, 396 (1959)), and 199 medium (Proceeding of the Society for the Biological Medicine, 73, 1 (1950)), Whitten medium (Hassei Kohgaku Jikken Manual, Transgenic Mouse no Tsukurikata (Experimental Manual for Developmental Engineering, Method for Creating transgenic Mice), Ed. Motoya Katsuki, Kodansha, 1987) with or without fetal calf serum or such. In general, the transformants are cultured at pH 6 to 8 and 30°C to 40°C under 5% $CO_2$ for one to seven days. If necessary, antibiotics such as kanamycin and penicillin may be added to the medium during culture.

Media that are used to culture transformants obtained by using insect cell hosts include conventional media such as TNM-FH medium (Pharmingen), Sf-900 II SFM medium (Life Technologies), ExCell400 and ExCell405 (both from JRH Biosciences), and Grace's Insect Medium (Nature, 195, 788 (1962)). In general, the transformants are cultured at pH 6 to 7 and 25°C to 30°C for one to five days. If necessary, antibiotics such as gentamicin may be added to the medium during culture.

Transformants obtained by using plant cell hosts can be cultured as cells or cultivated after differentiation into plant cells or organs. Media that are used to culture such transformants include conventional Murashige and Skoog (MS) medium, White medium, and these media supplemented with plant hormones such as auxin and cytokinin. In general, the transformants are cultured at pH 5 to 9 and 20°C to 40°C for 3 to 60 days. If necessary, antibiotics such as kanamycin and hygromycin may be added to the medium during culture.

The transformants can express and produce the mutant polypeptides spontaneously or upon induction during culture, and the polypeptides are accumulated in the culture. Such mutant polypeptides can be expressed directly. In addition, production and secretion of the mutant polypeptides, expression of fusion proteins, and the like can be achieved according to methods described in Molecular Cloning 2nd Ed., or such. Methods for producing the mutant polypeptides include methods for producing them in host cells, methods for secreting them to the outside of host cells, and methods for producing them on the host cell membrane. Any of the methods can be selected depending on the type of host cell and the structure of a mutant polypeptide to be produced.

When produced in host cells or on host cell surface membrane, the mutant polypeptides can be actively secreted to the outside of host cells by using the method of Paulson et al. (J. Biol. Chem., 264, 17619 (1989)), the method of Lowe et al. (Proc. Natl. Acad. Sci. U.S.A., 86, 8227 (1989); Genes Develop., 4, 1288 (1990)), or the methods described in JP-A (Kokai) H05-336963 and JP-A (Kokai) H06-823021.

Specifically, the mutant polypeptide of interest can be actively secreted to the outside of host cells by inserting a DNA encoding a mutant polypeptide and a DNA encoding a signal peptide suitable for expression of the mutant polypeptide into an expression vector using genetic recombination techniques, introducing the expression vector to host cells, and expressing the mutant polypeptide. Furthermore, the productivity can be increased by the method described in JP-A (Kokai) H02-227075 based on the gene amplification system using the dihydrofolate reductase gene or such. Alternatively, the polypeptide can be produced using animals or plants by redifferentiating animal or plant cells introduced with the gene to create gene-introduced animals (transgenic nonhuman animals) or plants (transgenic plants).

When the transformant is an animal or plant, the mutant polypeptide can be produced by breeding or cultivating the animal or plant according to conventional methods, and collecting the polypeptide produced and accumulated from the animal or plant. Methods for producing the mutant polypeptides using animals include, for example, methods for producing the mutant polypeptides of interest in animals created by introducing the genes into them according to known methods (American Journal of Clinical Nutrition, 63, 639S (1996); American Journal of Clinical Nutrition, 63, 627S (1996); Bio/Technology, 9, 830 (1991)).

When the transformant is an animal, the mutant polypeptide can be produced, for example, by creating and breeding a transgenic nonhuman animal introduced with DNA encoding the mutant polypeptide, and collecting the mutant polypeptide produced and accumulated from the animal. The polypeptides may be produced/accumulated in, for example, milk (JP-A (Kokai) S63-309192) and eggs of the animal. Any promoter can be used for this purpose, as long as it enables the expression in animals. For example, mammary gland cell-specific promoters such as α-casein promoter, β-casein promoter, β-lactoglobulin promoter, and whey acidic protein promoter are preferably used.

Methods for producing the mutant polypeptides using plants include, for example, those in which a transgenic plant introduced with a DNA encoding an antibody molecule by known methods (Soshiki Baiyo (Tissue Culture), 20 (1994); Soshiki Baiyo (Tissue Culture), 21 (1995); Trends in Biotechnology, 15, 45 (1997)) is cultivated so as to produce and accumulate the mutant polypeptide in the plant, at which point the polypeptide can be collected from the plant. When a mutant polypeptide produced by transformants introduced with a gene encoding the polypeptide is expressed as a

soluble form in cells, the cells may be collected by centrifugation after culture, suspended in aqueous buffer, and then crushed using a sonicator, French press, manton gaulin homogenizer, Dyno-mill to prepare cell-free extract. A supernatant is obtained by centrifuging the cell-free extract. A purified sample of the mutant polypeptide can be obtained from the supernatant using one or a combination of conventional methods for isolating and purifying enzymes, specifically, solvent extraction, salting out such as using ammonium sulfate, desalting, precipitation using organic solvents, anion-exchange chromatography using resins such as diethylaminoethyl (DEAE) Sepharose or DIAION HPA-75 (Mitsubishi Chemical Co. Ltd.), cation-exchange chromatography using resins such as S-Sepharose FF (Pharmacia), hydrophobic chromatography using resins such as butyl-Sepharose and phenyl-Sepharose, gel filtration using molecular sieve, affinity chromatography, chromatofocusing, and electrophoretic methods such as isoelectrofocusing.

Alternatively, when the mutant polypeptide is expressed as an insoluble form in cells, the collected cells can be crushed in the same way, and then the insoluble mutant polypeptide can be collected as the precipitation fraction by centrifugation. The collected insoluble mutant polypeptide may be solubilized using a protein denaturant. The resulting solution may be diluted or dialyzed to refold the mutant polypeptide into the normal conformation. Then, a purified sample of the mutant polypeptide can be obtained using the same isolation/purification methods as described above.

When the mutant polypeptide is secreted to the outside of cells, the mutant polypeptide or a derivative thereof can be collected from the culture supernatant. Specifically, a soluble fraction may be obtained by treating the culture using the same methods as described above such as centrifugation. A purified sample of the mutant polypeptide can be obtained from the soluble fraction using the same isolation/purification methods as described above.

Illustrative examples of such mutant polypeptides obtained by the methods described above include mutant antibodies, fragments of mutant antibodies, and fusion proteins containing the Fc region of a mutant antibody (recombinant antibodies capable of undergoing an antigen-antibody reaction, such as chimeric antibodies, humanized antibodies, and single-chain antibodies; and antibody-like molecules such as chimeric molecules).

In particular, the present invention provides methods for producing mutant antibodies with improved effector function, which include the step of substituting a cysteine residue for an amino acid residue at EU index 295 according to the Kabat numbering system in an antibody having the effector function. More specifically, the present invention provides methods for producing mutant antibodies with improved effector function, which comprise the steps of:

(1) substituting a cysteine residue for an amino acid residue at EU index 295 according to the Kabat numbering system in an antibody having the effector function;
(2) introducing host cells or host organisms with a DNA encoding L chain and a DNA encoding H chain having an Fc region including a substitution of a cysteine residue for an amino acid residue at EU index 295 according to the Kabat numbering system, and expressing the DNAs; and
(3) collecting the expression product.

In one embodiment of the present invention, first, a cysteine residue is substituted for an amino acid residue at EU index 295 according to the Kabat numbering system in an antibody which is known in the art to have the effector function. In another embodiment of the present invention, first, an antibody having the effector function is produced, and then a cysteine residue is substituted for an amino acid residue at EU index 295 according to the Kabat numbering system in the produced antibody. For example, antibodies having the effector function can be produced by obtaining antibodies that bind to a desired antigen by the method described below, and then judging whether the obtained antibodies have the effector function by methods known to those skilled in the art. Methods for assessing the effector function include, for example, the methods described in Examples.

In the context of the present invention, methods for substituting a cysteine residue for an amino acid residue are not particularly limited. The substitution can be achieved, for example, by site-directed mutagenesis (Oligonucleotide-directed mutagenesis using M13-derived vector: an efficient and general procedure for the production of point mutations in any fragment of DNA, Mark J. Zoller and Michael Smith, Nucleic Acids Research, Volume 10, Number 20, 6487-6500 (1982); Directed evolution of green fluorescent protein by a new versatile PCR strategy for site-directed and semi-random mutagenesis, Asako Sawano and Atsushi Miyawaki, Nucleic Acids Research, Volume 28, Number 16, e78 (2000)).

In the context of the present invention, as a next step, host cells or host organisms may be introduced with a DNA encoding L chain and a DNA encoding H chain having an Fc region comprising a substitution of a cysteine residue for an amino acid residue at EU index 295 according to the Kabat numbering system to express the DNAs using a method known to those skilled in the art. Then, the expression product (mutant antibody) can be collected using a method known to those skilled in the art.

The DNA encoding H chain having an Fc region containing a substitution of a cysteine residue for an amino acid residue at EU index 295 according to the Kabat numbering system can be prepared as a set of partial DNAs. The set of such partial DNAs include, for example, the combination of a DNA encoding variable region and a DNA encoding constant region, and the combination of a DNA encoding Fab region and a DNA encoding Fc region, but is not limited thereto. Likewise, the DNA encoding L chain can also be prepared as a set of partial DNAs.

A method for preparing antibodies that bind to a desired antigen is described below.

A known sequence can be used for the gene encoding an antibody H chain or L chain. Alternatively, the gene can also be obtained by methods known to those skilled in the art. The gene can be prepared, for example, from an antibody library. Alternatively, the antibody-encoding gene can be cloned from hybridomas producing the monoclonal antibody.

There are many known antibody libraries. Methods for constructing antibody libraries are also well known in the art. Thus, those skilled in the art can readily obtain appropriate antibody libraries. For example, for antibody phage libraries, one can refer to the documents such as Clackson et al., Nature 352, 624-8 (1991); Marks et al., J. Mol. Biol. 222, 581-97 (1991); Waterhouses et al., Nucleic Acids Res. 21, 2265-6 (1993); Griffiths et al., EMBO J. 13, 3245-60 (1994); Vaughan et al., Nature Biotechnology 14, 309-14 (1996); Japanese Patent Kohyo Publication No. (JP-A) H20-504970 (unexamined Japanese national phase publication corresponding to a non-Japanese international publication). Furthermore, it is possible to use other known methods, such as methods using eukaryotic cells as libraries (WO95/15393 pamphlet) and ribosome display methods. Furthermore, panning techniques for obtaining human antibodies from human antibody libraries are also known. For example, variable regions of human antibodies can be expressed on the surface of phages as single chain antibodies (scFvs) using phage display methods, and phages that bind to antigens can be selected. Genetic analysis of the selected phages can determine the DNA sequences encoding the variable regions of human antibodies that bind to the antigen. Once the DNA sequences ofscFvs that bind to the antigens is revealed, suitable expression vectors can be produced based on these sequences to obtain human antibodies. These methods are already known, and one can refer to WO92/01047, WO92/20791, WO93/06213, WO93/11236, WO93/19172, WO95/01438, and WO95/15388.

Basically, genes encoding antibodies can be obtained from hybridomas using known techniques. Immunization is carried out using desired antigens or cells expressing desired antigens as sensitizing antigens according to conventional immunization methods. The resulting immune cells are fused with known parent cells by conventional cell fusion methods. Monoclonal antibody-producing cells (hybridomas) are selected by conventional screening methods. mRNAs are prepared from the obtained hybridomas. cDNAs of antibody variable regions (V regions) are synthesized from the mRNAs using reverse transcriptase, and then ligated to DNAs encoding the constant regions (C regions) of the desired antibody.

More specifically, such sensitizing antigens used to obtain the antibody genes encoding the H chains and L chains of the present invention include, but not limited to, both complete antigens with immunogenicity and incomplete antigens including haptens or such without immunogenicity. For example, it is possible to use full-length proteins of interest or partial peptides. Such antigens can be prepared by methods known to those skilled in the art, for example, methods using baculoviruses (for example, WO98/46777). Hybridomas can be produced, for example, according to the method of Milstein *et al.* (G. Kohler and C. Milstein, Methods Enzymol. 73, 3-46 (1981)) or such. When the immunogenicity of an antigen is low, it can be linked to a macromolecule that has immunogenicity, such as albumin, and then used for immunization. Furthermore, they can be converted into soluble antigens by linking antigens with other molecules if necessary. When transmembrane molecules such as receptors are used as antigens, portions of the extracellular regions of the receptors can be used as a fragment, or cells expressing transmembrane molecules on their cell surface may be used as immunogens.

Antibody-producing cells can be obtained by immunizing animals using suitable sensitizing antigens described above. Alternatively, antibody-producing cells can be prepared by *in vitro* immunization of lymphocytes that can produce antibodies. Various mammals can be used for immunization, but rodents, lagomorphas, and primates are generally preferred. Illustrative examples of such animals include rodents such as mice, rats, and hamsters; lagomorphas such as rabbits; and primates such as monkeys including cynomolgus monkey, rhesus monkey, hamadryas, and chimpanzees. In addition, transgenic animals carrying human antibody gene repertoires are also known. Human antibodies can also be obtained by using these animals (see WO96/34096; Mendez et al., Nat. Genet. 15, 146-56 (1997)). Instead of using such transgenic animals, for example, desired human antibodies having binding activity to antigens can be obtained by *in vitro* sensitization of human lymphocytes with desired antigens or cells expressing desired antigens, and then fusing the sensitized lymphocytes with human myeloma cells such as U266 (see Japanese Patent Application Kokoku Publication No. (JP-B) H01-59878 (examined, approved Japanese patent application published for opposition)). Furthermore, desired human antibodies can be obtained by immunizing transgenic animals carrying a complete repertoire of human antibody genes with desired antigens (see WO93/12227, WO92/03918, WO94/02602, WO96/34096, and WO96/33735).

Animals can be immunized, for example, according to the following procedure. First, a sensitizing antigen is appropriately diluted or suspended in Phosphate-Buffered Saline (PBS), physiological saline, or such. If necessary, the antigen is mixed with an adjuvant to obtain emulsion. The antigen is injected into animals intraperitoneally or subcutaneously. Then, the sensitizing antigen mixed with Freund's incomplete adjuvant is preferably administered several times every 4 to 21 days. Antibody production can be confirmed by measuring the target antibody titer in animal sera using conventional methods.

Antibody-producing cells obtained from lymphocytes or animals immunized with a desired antigen can be fused with myeloma cells to generate hybridomas using conventional fusing agents (for example, polyethylene glycol) (Goding,

Monoclonal Antibodies: Principles and Practice, Academic Press, 1986, 59-103). If necessary, hybridoma cells are cultured and grown, and the binding specificity of the antibody produced from these hybridomas is determined using known assay methods such as immunoprecipitation, radioimmunoassay (RIA), and enzyme-linked immunosorbent assay (ELISA). Thereafter, hybridomas that produce antibodies with desired specificity, affinity, or activity can be subcloned by methods such as limiting dilution if necessary.

Next, genes encoding the selected antibodies can be cloned from hybridomas or antibody-producing cells (sensitized lymphocytes or such) using probes capable of specifically binding to the antibodies (for example, oligonucleotides complementary to sequences encoding the antibody constant regions). It is also possible to clone such genes from mRNA using RT-PCR.

The antibodies can be prepared by expressing the constructed antibody genes by known methods. When mammalian cells are used, the antibody gene can be expressed using expression vectors carrying DNA in which a useful conventional promoter/enhancer, the antibody gene to be expressed, and a downstream poly A signal on the 3' side of the gene are operably linked together. Examples of suitable promoters/enhancers include human cytomegalovirus immediate early promoter/enhancer. In addition, it is possible to use other promoters/enhancers such as viral promoters/enhancers from retrovirus, polyoma virus, adenovirus, simian virus 40 (SV40), and such; and mammalian promoters/enhancers such as human elongation factor 1$\alpha$. For example, when the SV40 promoter/enhancer is used, the antibody genes can be readily expressed by the method by Mulling *et al.* (Mulling, R. C. et al., Nature 277, 108-114 (1979)). Alternatively, when the human elongation factor 1$\alpha$ is used, the antibody genes can be readily expressed by the method of Mizushima *et al.* (Mizushima, Nucleic Acids Res. 18, 5322 (1990)). When E. *coli* is used, the antibody genes can be expressed using expression vectors carrying a DNA in which an antibody gene to be expressed is operably linked to a useful conventional promoter and a signal sequence for antibody secretion. Examples of such promoters include the lacZ promoter and araB promoter.

Antibodies obtained by culturing and proliferating hybridomas or by genetic recombination can be purified to homogeneity. The antibodies can be separated and purified by conventional protein separation/purification methods. For example, the antibodies can be separated and purified by the combined use of one or more methods appropriately selected from chromatographies such as affinity chromatography, filter, ultrafiltration, salting out such as using ammonium sulfate or sodium sulfate, dialysis, SDS polyacrylamide gel electrophoresis, isoelectrofocusing, and such (Antibodies: A Laboratory Manual. Ed. Harlow and David Lane, Cold Spring Harbor Laboratory, 1988). However, the methods are not limited to the above examples. Columns used for affinity chromatography include, protein A column, protein G column, and protein L column. Whether an antibody thus obtained has the effector function can be assessed by methods known to those skilled in the art, for example, by the methods described in Examples.

Methods for producing humanized antibodies are described below as more specific examples of methods for obtaining the mutant polypeptides of the present invention. Other mutant polypeptides can also be obtained by such methods.

(1) Construction of expression vectors for humanized antibodies

The expression vector for humanized antibodies refers to an animal cell expression vector having as inserts genes encoding human antibody light chain (L chain) C region and human antibody heavy chain (H chain) C region comprising a substitution of a cysteine for an amino acid residue at EU index 295 according to the Kabat numbering system. The expression vector can be constructed by cloning into an animal cell expression vector each of the genes encoding human antibody light chain (L chain) C region and human antibody heavy chain (H chain) C region comprising a substitution of a cysteine for an amino acid residue at EU index 295 according to the Kabat numbering system.

The human antibody C regions may be any human antibody H chain and L chain C regions. Illustrative examples of suitable C regions include the H chain C region of human IgG1 subclass antibody (hereinafter abbreviated as "hC$\gamma$1") and the L chain C region of human $\kappa$ class antibody (hereinafter abbreviated as "hC$\kappa$").

The genes encoding human antibody H chain and L chain C regions may be cDNAs or chromosomal DNAs containing exons and introns.

Any animal cell expression vector can be used so long as it enables the expression of inserted genes encoding human antibody C regions. Illustrative examples of suitable vectors include pAGE107 (Cytotechnology, 3, 133 (1990)), pAGE103 (J. Biochem., 101, 1307 (1987)), pHSG274 (Gene, 27, 223 (1984)), pKCR (Proc. Natl. Acad. Sci. U.S.A., 78, 1527 (1981)), and pSG1$\beta$d2-4 (Cytotechnology, 4, 173 (1990)). Examples of promoters/enhancers suitable for use in connection with animal cell expression vectors include the SV40 early promoter/enhancer (J. Biochem., 101, 1307 (1987)), the Moloney murine leukemia virus LTR (Biochem. Biophys. Res. Commun., 149, 960 (1987)), and the immunoglobulin H chain promoter (Cell, 41, 479 (1985)) and enhancer (Cell, 33, 717 (1983)).

Humanized antibody-expression vectors may be composed of vectors that each separately carry an antibody H chain or L chain, or vectors that each carry both (hereinafter referred to as "tandem type"). However, the tandem-type humanized antibody-expression vectors are preferred because (i) humanized antibody-expression vectors can be readily

constructed; (ii) the vectors can be readily introduced into animal cells; (iii) the expression levels of antibody H chain and L chain are well balanced with each other in animal cells; and so on (J. Immunol. Methods, 167, 271 (1994)). The tandem-type expression vectors for humanized antibodies include pKANTEX93 (Mol. Immunol., 37, 1035 (2000)) and pEE18 (Hybridoma, 17, 559 (1998)).

The constructed humanized antibody-expression vectors can be used in expressing chimeric human antibodies and CDR-grafted human antibodies in animal cells.

(2) Cloning of cDNAs encoding nonhuman animal antibody V regions

cDNAs encoding antibodies of nonhuman animals, for example, mouse antibody H chain and L chain V regions, can be obtained by the procedure described below.

mRNA is extracted from hybridomas producing a mouse antibody of interest. cDNA is synthesized from the mRNA and cloned into a vector such as phage or plasmid to prepare a cDNA library. Recombinant phages or plasmids carrying cDNAs encoding the H chain V region and recombinant phages or plasmids carrying cDNAs encoding the L chain V region are isolated from the library using as probes portions of the C region or V region of a known mouse antibody. The complete nucleotide sequences of the mouse antibody H chain and L chain V regions of interest in the recombinant phages or plasmids are determined, and the complete amino acid sequences of the H chain and L chain V regions are deduced from the nucleotide sequences.

Any nonhuman animals, including mouse, rat, hamster, and rabbit, may be used, so long as they allow for the preparation of hybridomas.

Methods for preparing total RNA from hybridomas include the guanidine thiocyanate-trifluoro-cesium method (Methods in Enzymol., 154, 3 (1987)). Methods for preparing mRNA from total RNA include the oligo(dT)-immobilized cellulose column method (Molecular Cloning: A Laboratory Manual, Cold Spring Harbor Lab. Press New York, 1989). Kits for preparing mRNA from hybridomas include Fast Track mRNA Isolation Kit (Invitrogen) and Quick Prep mRNA Purification Kit (Pharmacia). Methods for synthesizing cDNA and constructing cDNA libraries include conventional methods (Molecular Cloning: A Laboratory Manual, Cold Spring Harbor Lab. Press New York, 1989; Current Protocols in Molecular Biology, Supplement 1-34) and methods using commercially available kits, for example, Super Script™ Plasmid System for cDNA Synthesis and Plasmid Cloning (GIBCO BRL) and ZAP-cDNA Synthesis Kit (Stratagene).

When a cDNA library is constructed, cDNA synthesized using as a template mRNA extracted from hybridomas can be inserted into any vector, as long as the vector allows for insertion of the cDNA. Such vectors include, for example, ZAP Express (Strategies, 5, 58 (1992)), pBluescript II SK(+) (Nucleic Acids Research, 17, 9494 (1989)), λzap II (Stratagene), λgt10, λgt11 (DNA Cloning: A Practical Approach, I, 49 (1985)), Lambda BlueMid (Clontech), λExCell, pT7T3 18U (Pharmacia), pcD2 (Mol. Cell. Biol., 3, 280 (1983)), and pUC18 (Gene, 33, 103 (1985)).

cDNA libraries constructed using phage or plasmid vectors can be introduced into any E. *coli,* as long as the E. *coli* allows for the introduction of the cDNA libraries and can express and maintain them. Such E. *coli* strains include, for example, XL1-Blue MRF' (Strategies, 5, 81 (1992)), C600 (Genetics, 39, 440 (1954)), Y1088, Y1090 (Science, 222, 778 (1983)), NM522 (J. Mol. Biol., 166, 1 (1983)), K802 (J. Mol. Biol., 16, 118 (1966)), and JM105 (Gene, 38, 275 (1985)).

Illustrative methods for selecting cDNA clones encoding antibody H chain and L chain V regions of nonhuman animals from cDNA libraries include colony hybridization methods and plaque hybridization methods using isotope-labeled or fluorescence-labeled probes (Molecular Cloning: A Laboratory Manual, Cold Spring Harbor Lab. Press New York, 1989). Alternatively, cDNAs encoding the H chain and L chain V regions can be isolated by preparing primers and conducting Polymerase Chain Reaction (hereinafter abbreviated as "PCR"; Molecular Cloning: A Laboratory Manual, Cold Spring Harbor Lab. Press New York, 1989; Current Protocols in Molecular Biology, Supplement 1-34) using as a template cDNA or cDNA library synthesized from mRNA.

cDNAs selected by the methods described above may be digested with appropriate restriction enzymes, and then cloned into plasmids such as pBluescript SK(-) (Stratagene). The cDNAs are subjected to conventional nucleotide sequencing methods, for example, the dideoxy method of Sanger et al. (Proc. Natl. Acad. Sci., U. S. A., 74, 5463 (1977)). The nucleotide sequences of the cDNAs can be determined by analysis using automatic nucleotide sequence analyzers, for example, A. L. F. DNA sequencer (Pharmacia).

The complete amino acid sequences of H chain and L chain V regions are deduced from the determined nucleotide sequences. Whether the isolated cDNAs encode the complete amino acid sequences of antibody H chain and L chain V regions containing a secretory signal can be assessed by comparing them with the complete amino acid sequences of H chain and L chain V regions of known antibodies (Sequences of Proteins of Immunological Interest, US Dept. Health and Human Services, 1991).

Furthermore, when the amino acid sequences of antibody variable regions or the nucleotide sequences of DNAs encoding the variable regions are already known, the cDNAs can be prepared by the method described below.

When an amino acid sequence is known, the amino acid sequence may be converted into a DNA sequence in consideration of the codon usage (Sequences of Proteins of Immunological Interest, US Dept. Health and Human

Services, 1991). Several synthetic DNAs of about 100 bases may be synthesized based on the designed DNA sequence. The DNAs can then be assembled to the DNA of interest by PCR. Alternatively, when a nucleotide sequence is known, several synthetic DNAs of about 100 bases may be synthesized based on the information. The DNAs can then be assembled to the DNA of interest by PCR.

(3) Analysis of amino acid sequences of antibody V regions of nonhuman animals

When the complete amino acid sequences of antibody H chain and L chain V regions containing a secretory signal is unavailable, the length of secretory signal sequence and the N terminal amino acid sequence, as well as its subgroup, can be deduced by comparing the sequences with the complete amino acid sequences of H chain and L chain V regions of known antibodies (Sequences of Proteins of Immunological Interest, US Dept. Health and Human Services, 1991). Furthermore, the amino acid sequence of each CDR in the H chain and L chain V regions can be determined by comparing the sequences with the amino acid sequences of H chain and L chain V regions of known antibodies (Sequences of Proteins of Immunological Interest, US Dept. Health and Human Services, 1991).

(4) Construction of expression vectors for chimeric human antibodies

Expression vectors for chimeric human antibodies can be constructed by cloning cDNAs encoding H chain and L chain V regions of a nonhuman animal antibody upstream of the genes encoding human antibody H chain and L chain C regions in the expression vectors for humanized antibodies described above in (1). Such expression vectors for chimeric human antibodies can be constructed, for example, by the following procedure. cDNAs encoding antibody H chain and L chain V regions of a nonhuman animal are ligated with synthetic DNAs which comprise the nucleotide sequences of the 3'-termianl portions of antibody H chain and L chain V regions of a nonhuman animal and the nucleotide sequences of the 5'-termianl portions of human antibody H chain and L chain C regions, and which also contain appropriate restriction sites at each end. Each construct is cloned upstream of the gene encoding the human antibody H chain and L chain C regions in the expression vector for humanized antibodies described above in (1) so that they can be expressed in an appropriate form.

(5) Construction of cDNAs encoding the V regions of human CDR-grafted antibodies

cDNAs encoding H chain and L chain V regions of a human CDR-grafted antibody can be constructed according to the following procedure. First, the amino acid sequences of frameworks (hereinafter abbreviated as "FRs") of H chain and L chain V regions of a human antibody are selected to graft the CDRs of H chain and L chain V regions of a nonhuman animal antibody of interest onto them. Any amino acid sequences of FRs of the human antibody H chain and L chain V regions can be used, as long as they are derived from human antibodies. Such FR amino acid sequences include, for example, the amino acid sequences of FRs of human antibody H chain and L chain V regions deposited in databases such as Protein Data Bank, and consensus amino acid sequence of FRs of human antibody H chain and L chain V regions shared by respective subgroups (Sequences of Proteins of Immunological Interest, US Dept. Health and Human Services, 1991). Of them, amino acid sequences that exhibit as high homology (at least 60% or more) as possible to the amino acid sequences of FRs of H chain and L chain V regions of nonhuman animal antibody of interest are preferably selected to produce human CDR-grafted antibodies having sufficient activity.

Next, the amino acid sequences of CDRs of H chain and L chain V regions of the nonhuman animal antibody of interest are grafted onto the amino acid sequences of FRs of H chain and L chain V regions of the selected human antibody to design the amino acid sequences of H chain and L chain V regions of a human CDR-grafted antibody. The designed amino acid sequences are converted into DNA sequences in consideration of the codon usage seen in the nucleotide sequences of antibody genes (Sequences of Proteins of Immunological Interest, US Dept. Health and Human Services, 1991) to design the DNA sequences encoding the amino acid sequences of H chain and L chain V regions of the human CDR-grafted antibody. Several synthetic DNAs of about 100 bases are synthesized based on the designed DNA sequences. Then, PCR was carried out using the DNAs. In this case, four to six synthetic DNAs are preferably synthesized for each of the H and L chains in consideration of the reaction efficiency of PCR and maximal DNA length for synthesis.

Furthermore, by introducing an appropriate sequence of restriction site into the 5' end of the synthetic DNA at each end, the DNAs can be cloned readily into the expression vectors for humanized antibodies constructed as described in (1). After PCR, the amplification products may be cloned into plasmids such as pBluescript SK(-) (Stratagene), and their nucleotide sequences are determined by the methods described in (2). Accordingly, plasmids containing DNA sequences encoding the amino acid sequences of H chain and L chain V regions of the desired human CDR-grafted antibody can be obtained by this procedure.

(6) Construction of expression vectors for human CDR-grafted antibodies

Expression vectors for human CDR-grafted antibodies can be constructed by cloning the cDNAs encoding the H chain and L chain V regions of the human CDR-grafted antibody constructed as described in (5), upstream of the genes encoding human antibody H chain and L chain C regions in the expression vectors for humanized antibodies described in (1). For example, when an appropriate sequence of restriction site is introduced into the 5' end of synthetic DNA at each end among the synthetic DNAs used to construct the H chain and L chain V regions of human CDR-grafted antibody described in (5), the expression vectors for human CDR-grafted antibodies can be constructed by linking them upstream of the genes encoding the human antibody H chain and L chain C regions in the expression vectors for humanized antibodies described in (1) so that each can be expressed in an appropriate form.

(7) Stable production of humanized antibodies

Transformed cell lines each of which express a chimeric human antibody or human CDR-grafted antibody (hereinafter both are referred to as "humanized antibody") can be prepared by introducing the vectors for humanized antibodies described in (4) and (6) into appropriate animal cells. Exemplary methods for introducing expression vectors for humanized antibodies into animal cells include electroporation (JP-A (Kokai) H02-257891; Cytotechnology, 3, 133 (1990)). The expression vectors for humanized antibodies may be introduced into any animal cells, as long as the cells can produce the humanized antibodies.

Illustrative examples of such cells include NSO and SP2/0 (both are mouse myeloma lines); CHO/dhfr- and CHO/DG44 (both are Chinese hamster ovary cell lines); YB2/0 and IR983F (both are rat myeloma lines); BHK (a cell line derived from Syrian hamster kidney); and Namalwa (a human myeloma line). Preferably, CHO/DG44, a Chinese hamster ovary cell line, and YB2/0, a rat myeloma line, are used.

After the expression vectors for humanized antibodies are introduced, transformants stably producing the humanized antibodies can be selected using animal cell culture media containing drugs such as G418 sulfate (hereinafter abbreviated as "G418"; SIGMA) by the method disclosed in JP-A (Kokai) H02-257891. Such animal cell culture media include RPMI1640 (Nissui Pharmaceutical Co), GIT (Nihon Pharmaceutical Co), EX-CELL302 (JRH), IMDM (GIBCO BRL), and Hybridoma-SFM (GIBCO BRL) with or without various additives such as fetal calf serum (hereinafter abbreviated as "FCS"). The obtained transformants can be cultured to produce and accumulate the humanized antibodies in culture supernatant. The amounts of produced humanized antibodies in culture supernatant and their antigen-binding activities can be determined by enzyme immunoassay (hereinafter abbreviated as "ELISA"; Antibodies: A Laboratory Manual, Cold Spring Harbor Laboratory, Chapter 14, 1998; Monoclonal Antibodies: Principles and Practice, Academic Press Limited, 1996) or such. Furthermore, the amounts of humanized antibodies produced by the transformants can also be increased by the method described in JP-A (Kokai) H02-257891 using the DHFR gene-mediated gene amplification system or such.

The humanized antibodies can be purified from culture supernatants of the transformants using Protein A column (Antibodies: A Laboratory Manual, Cold Spring Harbor Laboratory, Chapter 8, 1988; Monoclonal Antibodies: Principles and Practice, Academic Press Limited, 1996). In addition, it is possible to employ other conventional protein purification methods. For example, the antibodies can be purified by the combined use of gel filtration, ion-exchange chromatography, ultrafiltration, and such. The molecular weight of a purified humanized antibody, or its H chain or L chain can be estimated by polyacrylamide gel electrophoresis (hereinafter abbreviated as "SDS-PAGE"; Nature, 227, 680 (1970)), Western blotting (Antibodies: A Laboratory Manual, Cold Spring Harbor Laboratory, Chapter 12, 1988; Monoclonal Antibodies: Principles and Practice, Academic Press Limited, 1996), or such.

Methods for producing the mutant polypeptides using animal cells as the host are described above. As described above, the mutant polypeptides can also be produced by the same methods as those using animal cells when using yeast, insect cells, plant cells, animals, or plants.

If the host cells already have the ability to express the mutant polypeptides, the mutant polypeptides of the present invention can be produced by preparing the cells expressing the mutant polypeptides of the present invention by known methods, culturing the cells, and then purifying the mutant polypeptides of interest from the culture.

The present invention further provides methods for producing mutant chimeric molecules which contain a cell surface molecule recognition site and an Fc region with improved effector function. Such methods include the step of substituting a cysteine residue for an amino acid residue at EU index 295 according to the Kabat numbering system in the Fc region of a chimeric molecule with the effector function. More specifically, the present invention provides methods for producing mutant chimeric molecules having improved effector function, which include the steps of:

(1) substituting a cysteine residue for an amino acid residue at EU index 295 according to the Kabat numbering system in the Fc region of a chimeric molecule having the effector function, the molecule containing a cell surface molecule recognition site in addition to the Fc region;

(2) introducing host cells or host organisms with a DNA encoding the mutant chimeric molecule containing a cell surface molecule recognition site and an Fc region in which a cysteine residue has been substituted for an amino acid residue at EU index 295 according to the Kabat numbering system, and expressing the DNA; and
(3) collecting the expression product.

Chimeric molecules used in the methods of the present invention for producing mutant chimeric molecules may contain a peptide region between the Fc region and the cell surface molecule recognition site so long as they retain the requisite effector function. Examples of such peptide regions include, but are not limited to, (1) linkers, (2) antibody hinge regions, and (3) linker and antibody hinge regions. The term "antibody hinge region" encompasses not only full-length antibody hinge regions but also portions of antibody hinge regions, so long as they contribute to (or do not inhibit) the effector function.
In the methods for producing mutant chimeric molecules of the present invention, first, a cysteine residue is substituted for an amino acid residue at EU index 295 according to the Kabat numbering system in the Fc region of a chimeric molecule with the effector function, which includes a cell surface molecule recognition site in addition to the Fc region. The methods for substituting a cysteine residue for an amino acid residue at EU index 295 according to the Kabat numbering system are not particularly limited. The substitution can be achieved, for example, by the site-directed mutagenesis described above.
In the methods for producing mutant chimeric molecules of the present invention, next, a DNA encoding the mutant chimeric molecule containing the cell surface molecule recognition site and the Fc region having a substitution of a cysteine residue for an amino acid residue at EU index 295 according to the Kabat numbering system is introduced and expressed in host cells or host organisms by methods known to those skilled in the art. The expression products (mutant chimeric molecules) can be collected by methods those skilled in the art.
Furthermore, the present invention also provides methods for producing mutant chimeric molecules having improved effector function, which include the steps of:

(1) producing chimeric molecules that contain a cell surface molecule recognition site and an Fc region;
(2) judging whether the produced chimeric molecules have the requisite effector function;
(3) substituting a cysteine residue for an amino acid residue at EU index 295 according to the Kabat numbering system in the Fc region of a chimeric molecule that has been judged to have the effector function;
(4) introducing and expressing in host cells or host organisms a DNA encoding the mutant chimeric molecule that contains a cell surface molecule recognition site and an Fc region having a substitution of a cysteine residue for an amino acid residue at EU index 295 according to the Kabat numbering system; and
(5) collecting the expression product.

In this method, first, a chimeric molecule containing an Fc region and a cell surface molecule recognition site is produced. The produced chimeric molecule includes at least an Fc region and a cell surface molecule recognition site, and includes but is not limited to, for example, chimeric molecules in which a peptide region is situated between the Fc region and the cell surface molecule recognition site. Such chimeric molecules can be produced by the procedure described below.
First, a DNA encoding a cell surface molecule recognition site and a DNA encoding an Fc region are cloned by methods known to those skilled in the art. If necessary, DNAs encoding peptides other than these portions are obtained by cloning. The orgin of the Fc region is not particularly limited. The Fc region may be derived from an antibody having the effector function or an antibody having no effector function.
Then, the DNA encoding the Fc region may be linked to the DNA encoding the cell surface molecule recognition site by methods known to those skilled in the art (if necessary, the DNA encoding the cell surface molecule recognition site and the DNA encoding the Fc region are linked with a DNA encoding a peptide other than them) to prepare a DNA encoding the chimeric molecule that comprises the Fc region and the cell surface molecule recognition site. Then, the DNA encoding the chimeric molecule that comprise the Fc region and the cell surface molecule recognition site is introduced and expressed in host cells or host organisms using methods known to those skilled in the art. The expression products (chimeric molecules) can be collected by methods known to those skilled in the art.
In this method, next, the chimeric molecules thus produced may be assessed for the effector function. Whether the chimeric molecules have the effector function can be assessed by methods known to those skilled in the art, for example, the methods described in Examples herein.
Then, a cysteine residue is substituted for an amino acid residue at EU index 295 according to the Kabat numbering system in the Fc region of a chimeric molecule that has been judged to have the effector function. Methods for substituting a cysteine residue for an amino acid residue at EU index 295 according to the Kabat numbering system are not particularly limited. The substitution can be achieved, for example, by the site-directed mutagenesis procedure described above.

In the methods for producing mutant chimeric molecules of the present invention, the DNA encoding a mutant chimeric molecule that contains a cell surface molecule recognition site and an Fc region having a substitution of a cysteine residue for an amino acid residue at EU index 295 according to the Kabat numbering system is introduced and expressed in host cells or host organisms using methods known to those skilled in the art. The expression products (mutant chimeric molecules) can be collected by using methods known to those skilled in the art.

The amounts of purified mutant polypeptides, and their antigen-binding activity and effector function can be determined by known methods such as described in "Monoclonal Antibodies: principles and practice, 3rd Ed., Acad. Press, 1993" and "Antibodies, A Laboratory Manual, Cold Spring Harbor Laboratory, 1988". Specifically, for example, when the mutant polypeptide is a humanized antibody, the antigen-binding activity and activity of binding to cells of antigen-positive culture cell lines can be determined by ELISA or fluorescent antibody method (Cancer Immunol. Immunother., 36, 373 (1993)). The cytotoxic activity for antigen-positive culture cell lines can be assessed by CDC or ADCC assay (Cancer Immunol. Immunother., 36, 373 (1993)). Furthermore, the safety and therapeutic effect of the mutant polypeptides in human can be assessed utilizing an appropriate model using an animal species relatively close to human, such as cynomolgus monkey.

The mutant polypeptides of the present invention have improved ADCC as compared to the polypeptides before cysteine substitution. Therefore, the dose of the agents can be reduced when the polypeptides are used in antibody therapy. Thus, the polypeptides of the present invention are cost effective and can reduce adverse effects.

Since the mutant polypeptides of the present invention have the significantly improved effector function as described above, they can be widely used in treatment, diagnosis, assessment, and so on.

Therapeutic compositions of the present invention comprise the mutant polypeptides of the present invention. Because of the improved ADCC, the compositions can produce sufficient pharmacological effects even at low doses, and thus are very advantageous to provide superior antibody therapy. The therapeutic compositions may contain, for example, nucleic acids, amino acids, peptides, proteins, other pharmaceutical additives such as organic compounds, inorganic compounds, and excipients, or combination thereof, in addition to the mutant polypeptides. The therapeutic compositions of the present invention may be formulated, if needed, using a molding method, and can be administered orally or parenterally. The therapeutic compositions of the present invention can be preferably used, in particular, by parenteral administration methods such as using injection, drop, and percutaneous absorbents.

More specifically, the present invention provides therapeutic compositions containing mutant polypeptides of the present invention and pharmaceutically acceptable carriers. Furthermore, the present invention also provides methods for treating mammals, which include the step of administering a mutant polypeptide of the present invention. Examples of such mammals include human and nonhuman mammals (for example, mice, rats, and monkeys).

The therapeutic compositions of the present invention can be formulated by mixing an antibody with pharmaceutically acceptable carries by known methods. For example, the compositions can be combined with water or other pharmaceutically acceptable liquid, and the resulting sterile solutions or suspensions can be used parenterally as injection. More particularly, the compositions can be formulated by appropriately combining with pharmaceutically acceptable carriers or media, specifically sterile water, physiological saline, vegetable oils, emulsifiers, suspensions, surfactants, stabilizers, flavoring agents, excipients, vehicles, preservatives, binding agents, and such, and mixing at a unit dosage form required by accepted pharmaceutical implementations. The amount of the active ingredient in such formulations may be routinely adjusted such that the appropriate dose within the required range is obtained.

Sterile compositions for injection can be formulated using a vehicle such as distilled water used for injection, according to standard protocols.

Aqueous solutions used for injections include physiological saline and isotonic solutions comprising glucose or other adjunctive agents such as D-sorbitol, D-mannose, D-mannitol, and sodium chloride. They may also be combined with an appropriate solubilizing agent such as alcohol, specifically, ethanol, polyalcohol such as propylene glycol or polyethylene glycol, or non-ionic surfactant such as polysorbate 80™ or HCO-50.

Oils include sesame oils and soybean oils, and can be combined with solubilizing agents such as benzyl benzoate or benzyl alcohol. The injections may also be formulated with buffers such as phosphate buffers or sodium acetate buffers, soothing agents such as procaine hydrochloride, stabilizers such as benzyl alcohol or phenol, or antioxidants. The prepared injections are typically aliquoted into appropriate ampules.

The administration is preferably carried out parenterally. Specific examples of suitable modes of administration include injection, intranasal administration, intrapulmonary administration, and percutaneous administration. Injections include intravenous injections, intramuscular injections, intraperitoneal injections, and subcutaneous injections. It is also possible to administer the injection solutions systemically or locally.

Furthermore, appropriate administration methods can be selected according to the patient's age and symptoms. The single dose of a pharmaceutical composition comprising a mutant polypeptide of the present invention can be selected, for example, from the range of about 1 μg/kg to 100 mg/kg body weight (for example, about 1 μg/kg to 15 mg/kg, about 10 μg/kg to 20 mg/kg, or about 0.1 mg/kg to 20 mg/kg). The pharmaceutical compositions of the present invention may be administered at a single site or multiple sites. Furthermore, it is possible to continuously administer

the pharmaceutical compositions of the present invention. The compositions may be repeatedly administered for several days or more depending on conditions until a pathological condition is desirably suppressed. The doses and administration methods vary depending on the patient's body weight, age, symptoms, and so on, but those skilled in the art can select appropriate doses and administration methods.

The therapeutic compositions of the present invention can be used in the treatment of the various diseases to which antibody therapy is applicable. Such diseases and symptoms include, for example, metastatic breast cancer (anti-HER2 antibody), CD20-positive B cell non-Hodgkin lymphoma (anti-CD20 antibody), rheumatoid arthritis and Crohn's disease (anti-TNFα antibody), and acute rejection after kidney transplantation (anti-CD3 antibody and anti-CD25 antibody). Specific antibodies known for use in the context of therapy are shown in parentheses. The therapeutic effects can be significantly improved by using the therapeutic compositions of the present invention instead of conventional antibodies in treating these diseases. As a result, the physical and economic burdens of patients are reduced. Furthermore, the compositions of the present invention can be preferably used as antibody pharmaceuticals in antibody therapy in the future.

The present invention also provides methods for improving the effector function of antibodies, which include the step of substituting a cysteine residue for an amino acid residue at EU index 295 according to the Kabat numbering system in antibodies having the effector function.

Furthermore, the present invention provides methods for improving the effector function of chimeric molecules containing a cell surface molecule recognition site and Fc region, which include the step of substituting a cysteine residue for an amino acid residue at EU index 295 according to the Kabat numbering system in the Fc region of a chimeric molecule with the effector function.

In the above method, the substitution for an amino acid residue at EU index 295 according to the Kabat numbering system can be achieved by the methods described above.

The present invention further provides methods for improving the effector function of chimeric molecules, which include the steps of:

(1) producing a chimeric molecule containing an Fc region and a cell surface molecule recognition site;
(2) assessing whether the produced chimeric molecule has the requisite effector function; and
(3) substituting a cysteine residue for an amino acid residue at EU index 295 according to the Kabat numbering system in the Fc region of a chimeric molecule that has been judged to have the effector function.

In this method, the production of the chimeric molecule, assessment of the produced chimeric molecule for the effector function, and substitution for an amino acid residue at EU index 295 according to the Kabat numbering system can be achieved by the methods described above.

All prior-art documents cited herein are incorporated herein by reference.

[Examples]

Herein below, the present invention will be specifically described using examples; however, it is not to be construed as being limited thereto.

Preparation Example 1: Isolation and purification of human peripheral blood mononuclear cells

Human blood was collected into a blood collecting bag (TERUMO CORP.) containing anticoagulant. This blood was laid onto a Lymphoprep solution (AXIS-SHIELD), which was dispensed into 50 ml centrifuge tubes (Falcon) in 15 ml-aliquots, and then centrifuged in a conventional swing-type centrifuge for cell isolation at 1,600 rotations for 30 minutes at room temperature according to the manufacturer's instructions. Following centrifugation, the mononuclear cell layer was collected, washed three times with calcium and magnesium free phosphate buffered saline supplemented with bovine serum albumin (BSA, Sigma) at a final concentration of 0.5% (w/v) (BSA-PBS, Sigma), and dispersed into RPMI-1640 medium containing fetal calf serum (FCS) at a final concentration of 10% (10% FCS-RPMI-1640), according to the manufacturer's instructions. This solution was used in the following examples as the cell suspension of mononuclear cells derived from human peripheral blood.

[Example 1] Humanized anti-CD20 chimeric antibody and humanized anti-CD20 chimeric antibody Cys mutant

*1. Preparation of humanized anti-CD20 chimeric antibody expression vectors:*

*1-1. Construction of cDNA encoding the L chain V region of mouse anti-CD20 monoclonal antibody:*

A cDNA encoding the amino acid sequence of the light chain variable region (hereinafter, referred to as "VL") of the mouse anti-CD20 monoclonal antibody, 2B8, described in WO 94/11026 was constructed using the PCR method described below.

First, to facilitate cloning into an expression vector, restriction enzyme recognition sequences were added to 5' and 3' ends of the cDNA sequence of the VL described in WO94/11026, the sequence at 5'-end being restriction enzyme Sac I sequence and the sequence at 3'-end being restriction enzyme Hind III sequence. The designed nucleotide sequence was then divided into six nucleotide segments of about 100 bases, starting from the 5' end and with adjacent nucleotide sequences have overlapping sequences of about 20 bases at the ends. Each of the sequences was then synthesized in the alternative order of sense strand and antisense strand to give rise to six DNA oligonucleotides.

Each synthesized oligonucleotide was added to a PCR reaction solution at a final concentration of 0.1 μM. 0.25 μl of ExTaq polymerase (TaKaRa) and 5 μl of the buffer attached to ExTaq polymerase were added to the reaction system having a final volume of 50 μl to perform 30 cycles of PCR reaction. After the PCR reaction, electrophoresis was performed in a 1% agarose gel according to conventional procedures, and the cDNA fragment amplified by PCR was recovered and purified using Wizard SV Gel and PCR Clean-up System (Promega) according to the manufacturer's instructions. cDNA concentration was calculated from the absorbance at 260 nm (1 O.D.260 = 50 μg/ml). 1 μg of this fragment was digested with ten units each of restriction enzymes Sac I and Hind III at 37°C for three hours according to the manufacturer's instructions, and the cDNA fragment of VL region with the termini digested with restriction enzymes Sac I and Hind III was recovered in the same manner as described above.

*1-2. Construction of cDNA encoding H chain V region of mouse anti-CD20 monoclonal antibody:*

cDNA encoding the amino acid sequence of the heavy chain variable region (hereinafter, referred to as "VH") of the mouse anti-CD20 monoclonal antibody, 2B8, described in WO 94/11026 was constructed using PCR method as described below.

First, to facilitate cloning into an expression vector, restriction enzyme recognition sequences were added to 5' and 3' ends of the cDNA sequence of the VH described in WO94/11026, the sequence at 5'-end being restriction enzyme Xho I sequence and the sequence at 3'-end being restriction enzyme Nhe I sequence. The designed nucleotide sequence was then divided into six nucleotide segments of about 100 bases, starting from the 5' end and with adjacent nucleotide sequences have overlapping sequences of about 20 bases at the ends. Each of the sequences was then synthesized in the alternative order of sense strand and antisense strand to give rise to six DNA oligonucleotides.

Each synthesized oligonucleotide was added to a PCR reaction solution at a final concentration of 0.1 μM. 0.25 μl of ExTaq polymerase (TaKaRa) and 5 μl of the buffer attached to ExTaq polymerase were added to the reaction system having a final volume of 50 μl to perform 30 cycles of PCR reaction. After the PCR reaction, electrophoresis was performed in a 1% agarose gel according to conventional procedures, and the cDNA fragment amplified by PCR was recovered and purified using Wizard SV Gel and PCR Clean-up System (Promega) according to the manufacturer's instructions. cDNA concentration was calculated from the absorbance at 260 nm (1 O.D.260 = 50 μg/ml). 1 μg of this fragment was digested with ten units each of restriction enzymes Xho I and Nhe I at 37°C for three hours according to the manufacturer's instructions, and the cDNA fragment of VH region with the termini digested with restriction enzymes Xho I and Nhe I was recovered in the same manner as described above.

*1-3. Construction of humanized anti-CD20 chimeric antibody-expression vectors:*

Vectors for expressing a humanized antibody were prepared by separating cDNAs encoding a human antibody missing the variable region from Baculovirus Expression Vector pAc-k-CH3 (PROGEN Biotechnik GmbH). First, cDNAs each encoding the IgG light chain (CH1) missing the variable region and the IgG heavy chain (CH1, CH2, and CH3) missing the variable region were separated from pAc-k-CH3 vector using restriction enzymes. Each of these cDNAs was inserted into a pIRES puro2 vector and a pIRES hyg2 vector by conventional procedures, respectively, to construct a human IgG light chain expression vector and a human IgG heavy chain expression vector. The human IgG light chain expression vector was digested with restriction enzymes Sac I and Hind III, and the human IgG heavy chain expression vector was digested with restriction enzymes Xho I and Nhe I by conventional procedures, and each expression vector was prepared using an agarose gel by conventional procedures. To each of the vectors, the cDNA of interest, which was obtained in 1-1 through digestion of the VL cDNA with restriction enzymes Sac I and Hind III by conventional

procedures and separation in an agarose gel or was obtained in 1-2 through digestion of the VH cDNA with restriction enzymes Xho I and Nhe I by conventional procedures and separation in an agarose gel, was ligated by using Ligation High (Toyobo Co., Ltd.) according to the manufacturer's instructions. Subsequently, *Escherichia coli* for transformation, DH5α (Toyobo Co., Ltd.), was transformed, and the colonies obtained were liquid-cultured in NZY broth for 16 hours. Plasmids were purified from the transformed *E. coli* using Wizard plus SV Minipreps DNA Purification Kit (Promega). Subsequently, the DNA sequences were confirmed by conducting a reaction using ABI 3100-Avant (Applied Biosystems) according to the manufacturer's instructions to obtain pIRESpuro2-IgG-L and pIRESpuro2-IgG-H vectors expressing the light chain and the heavy chain of humanized anti-CD20 chimeric antibody, respectively.

*2. Preparation of humanized anti-CD20 chimeric antibody producing cells using CHO cells:*

The humanized anti-CD20 chimeric antibody light and heavy chain expression vectors obtained in 1-3, which were pIRESpuro2-IgG-L and pIRESpuro2-IgG-H respectively, were used for expressing anti-CD20 chimeric antibody in animal cells in the following manner.

Gene introduction into 1 x $10^6$ CHO-K1 cells was performed using 36 μl of FuGENE and 12 μg of pIRESpuro2-IgG-L vector according to the manufacturer's instructions. Two days after the gene introduction, puromycin was added to RPMI-1640 medium supplemented with 10% fetal calf serum (FCS) at a final concentration of 0.5-5 μg/ml, and the cells were cultured for nine days. After selecting drug-resistant CHO-K1 cells with puromycin, cells were cloned by the limiting dilution method, and cells expressing the light chain of anti-CD20 chimeric antibody at a high level were screened and selected by ELISA.

Next, gene introduction into 1 x $10^6$ CHO-K1 cells that express the light chain of humanized anti-CD20 chimeric antibody at a high level was performed using 36 μl of FuGENE and 12 μg of pIRESpuro2-IgG-H vector according to the manufacturer's instructions. Two days after the gene introduction, hygromycin was added at various concentrations to RPMI-1640 medium supplemented with 10% fetal calf serum (FCS), and the cells were cultured for nine days. After selecting drug-resistant CHO-K1 cells with hygromycin, cells were cloned by the limiting dilution method, and a clone, anti-CD20 mAb-CHO cells, expressing humanized anti-CD20 chimeric antibody at a high level was obtained by ELISA.

*3. Purification of humanized anti-CD20 chimeric antibody from culture supernatant:*

The anti-CD20 mAb-CHO cells expressing the humanized anti-CD20 chimeric antibody, obtained in step 2, were cultured in RPMI-1640 medium supplemented with 10% FCS until they grew to 60% of the maximum cell density of the culture dish. The growth medium was removed by twice wash with $Ca^{2+}$- and $Mg^{2+}$-free phosphate buffered saline (PBS(-)) and was replaced with RPMI-1640 supplemented with 0.1% BSA. The cells were cultured for three days to collect supernatant. The supernatant was filtered through a 0.2 μm filter and let adsorbed to a Protein G column (Amersham Bioscience) by conventional procedures, followed by elution with 0.1 M Glycin buffer (pH 2.8) and pH neutralization using 0.75 M Tris-HCl (pH 9.0). Subsequently, dialysis was performed with PBS(-) by conventional procedures. This sample was measured for its concentration of humanized anti-CD20 chimeric antibody by the ELISA method using anti-human IgG antibody, and was used in the subsequent experiments.

[Example 2] Preparation of anti-CD20 chimeric antibodies

*1) Preparation of anti-CD20 chimeric antibodies:*

Chimeric antibodies were obtained as purified chimeric antibodies through the following steps A to F:

A) cloning of genes required for the preparation of chimeric antibodies;
B) mutagenesis of a cloned gene;
C) construction of chimeric antibody-expression vectors into which a cloned gene or its mutated gene is combined;
D) gene introduction into CHO cells with the chimeric antibody-expression vectors;
E) culture of chimeric antibody-expressing CHO cells; and
F) column purification from culture supernatant of the chimeric antibody-expressing cells.

Steps A to F are described in further detail below.

A) Cloning of genes required for the preparation of chimeric antibodies:

Four kinds of genes are required for the preparation of a single kind of anti-CD20 chimeric antibody. The four kinds of genes are a mouse anti-CD20 L chain variable region gene, a mouse anti-CD20 H chain variable region gene,

a human IgG 1 L chain constant region gene, and a human IgG1 H chain constant region gene. Examples of cloning of these genes are described in further detail below.

(Mouse anti-CD20 L chain variable region gene)

mRNA was obtained from hybridoma cells producing mouse anti-CD20 monoclonal antibody, which hybridoma the Applicant possesses, by using QuickPrep micro mRNA purification kit (Amersham Biosciences, code 27-9255-01). cDNA was synthesized from the mRNA by using First-Strand cDNA Synthesis kit (Amersham Biosciences, code 27-9261-01). To amplify a gene by PCR method using this cDNA as a template, PCR reaction was carried out with the following eleven patterns of primer combinations.
The PCR reaction conditions were as shown below:

| | |
|---|---|
| cDNA from mouse hybridoma | 4 μL |
| 2.5 mM dNTPs | 4 μL |
| One of the eleven kinds of primers, MKV 1 to MKV11 (20 μM) | 2.5 μL |
| MKC primer (20 μM) | 2.5 μL |
| DMSO | 2.5 μL |
| x10 pfu polymerase Buffer | 5 μL |
| pfu polymerase | 1 μL |
| Sterile water | 28.5 μL |
| Total | 50 μL |

94°C for 2 min;
94°C for 1 min, 55°C for 2 min, 72°C for 2 min (30 cycles);
72°C for 4 min; and
4°C for an unlimited time.

DNA sequences of primers utilized are shown below.

MKV 1 primer: ATGAAGTTGCCTGTTAGGCTGTTGGTGCTG (SEQ ID NO: 1)
MKV2 primer: ATGGAGWCAGACACACTCCTGYTATGGGTG (SEQ ID NO: 2)
MKV3 primer: ATGAGTGTGCTCACTCAGGTCCTGGSGTTG (SEQ ID NO: 3)
MKV4 primer: ATGAGGRCCCCTGCTCAGWTTYTTGGMWTCTTG (SEQ ID NO: 4)
MKV5 primer: ATGGATTTWCAGGTGCAGATTWTCAGCTTC (SEQ ID NO: 5)
MKV6 primer: ATGAGGTKCYYTGYTSAGYTYCTGRGG (SEQ ID NO: 6)
MKV7 primer: ATGGGCWTCAAGATGGAGTCACAKWYYCWGG (SEQ ID NO: 7)
MKV8 primer: ATGTGGGGAYCTKTTTYCMMTTTTTCAATTG (SEQ ID NO: 8)
MKV9 primer: ATGGTRTCCWCASCTCAGTTCCTTG (SEQ ID NO: 9)
MKV10 primer: ATGTATATATGTTTGTTGTCTATTTCT (SEQ ID NO: 10)
MKV 11 primer: ATGGAAGCCCCAGCTCAGCTTCTCTTCC (SEQ ID NO: 11)
MKC primer: ACTGGATGGTGGGAAGATGG (SEQ ID NO: 12)
(M=A or C, R=A or G, W=A or T, S=C or G, Y=C or T, K=G or T)

In the PCR reaction, mouse anti-CD20 L chain variable region gene was amplified with the combination of MKV5 and MKC primers, and this gene was temporarily inserted into the pCR2.1 vector (Invitrogen) and stored. Herein, the vector with this gene inserted is referred to as pCR2.1-MLV

(Mouse anti-CD20 H chain variable region gene)

In the same manner as in the cloning of the mouse anti-CD20 L chain variable region gene, the following twelve patterns of PCR amplification were performed using the cDNA, prepared from hybridoma cells producing mouse anti-CD20 monoclonal antibody, as a template.

| | |
|---|---|
| cDNA from mouse hybridoma | 4 μL |
| 2.5 mM dNTPs | 4 μL |
| One of the twelve kinds of primers, MHV 1 to MHV 12 (20 μM) | 2.5 μL |

(continued)

| | |
|---|---|
| MHCG2b primer (20 μM) | 2.5 μL |
| DMSO | 2.5 μL |
| x10 pfu polymerase Buffer | 5 μL |
| pfu polymerase | 1 μL |
| Sterile water | 28.5 μL |
| Total | 50 μL |

94°C for 2 min;
94°C for 1 min, 55°C for 2 min, 72°C for 2 min (30 cycles);
72°C for 4 min; and
4°C for an unlimited time.

DNA sequences of primers are shown below.

MHV 1 primer: ATGAAATGCAGCTGGGGCATSTTCTTC (SEQ ID NO: 13)
MHV2 primer: ATGGGATGGAGCTRTATCATSYTCTT (SEQ ID NO: 14)
MHV3 primer: ATGAAGWTGTGGTTAAACTGGGTTTTT (SEQ ID NO: 15)
MHV4 primer: ATGRACTTTGGGYTCAGCTTGRTTT (SEQ ID NO: 16)
MHV5 primer: ATGGACTCCAGGCTCAATTTAGTTTTCCTT (SEQ ID NO: 17)
MHV6 primer: ATGGCTGTCYTRGSGCTRCTCTTCTGC (SEQ ID NO: 18)
MHV7 primer: ATGGRATGGAGCKGGRTCTTTMTCTT (SEQ ID NO: 19)
MHV8 primer: ATGAGAGTGCTGATTCTTTTGTG (SEQ ID NO: 20)
MHV9 primer: ATGGMTTGGGTGTGGAMCTTGCTATTCCTG (SEQ ID NO: 21)
MHV10 primer: ATGGGCAGACTTACATTCTCATTCCTG (SEQ ID NO: 22)
MHV11 primer: ATGGATTTTGGGCTGATTTTTTTTATTG (SEQ ID NO: 23)
MHV12 primer: ATGATGGTGTTAAGTCTTCTGTACCTG (SEQ ID NO: 24)
MHCG2b primer: CAGTGGATAGACTGATGGGGG (SEQ ID NO: 25)
(M=A or C, R=A or G, W=A or T, S=C or G, Y=C or T, K=G or T)

In the PCR reaction, mouse anti-CD20 H chain variable region gene was amplified with the combination of MHV7 and MHCG2b primers, and this gene was temporarily inserted into the pCR2.1 vector (Invitrogen) and stored. Herein, the vector with this gene inserted is referred to as pCR2.1-MHV.

(Human IgG1 L chain constant region gene)

Lymphocytes were collected from human blood using a Lymphoprep solution (Axis Shield). mRNA was obtained from these lymphocytes using QuickPrep micro mRNA purification kit (Amersham Biosciences, code 27-9255-01). cDNA was synthesized from the mRNA using First-Strand cDNA Synthesis kit (Amersham Biosciences, code 27-9261-01). The following PCR reaction was performed using this cDNA from human lymphocytes as a template to obtain human IgG1 L chain constant region gene. This gene was also temporarily inserted into the pCR2.1 vector (Invitrogen) and stored. Herein, the vector with this gene inserted is referred to as pCR2.1-LC.

The PCR reaction conditions were as shown below:

| | |
|---|---|
| Human lymphocyte cDNA | 4 μL |
| 2.5 mM dNTPs | 4 μL |
| hIgG1 LCF primer (20 μM) | 2.5 μL |
| hIgG1 LCR primer (20 μM) | 2.5 μL |
| DMSO | 2.5 μL |
| x10 pfu polymerase Buffer | 5 μL |
| pfu polymerase | 1 μL |
| Sterile water | 28.5 μL |
| Total | 50 μL |

94°C for 2 min;
94°C for 1 min, 55°C for 2 min, 72°C for 2 min (30 cycles);
72°C for 4 min; and
4°C for an unlimited time.

DNA sequences of primers are shown below.

hIgG1 LCF primer: ACTGTGGCTGCACCATCTGTCTTC (SEQ ID NO: 26)
hIgG1 LCR primer: TTAACACTCTCCCCTGTTGAAGCTCTT (SEQ ID NO: 27)

(Human IgG1 H-chain constant region gene)

In the same manner as in the cloning of the human IgG 1 L chain constant region gene, the following PCR reaction was performed using the cDNA from human lymphocytes as a template to obtain human IgG1 H chain constant region gene. This gene was also temporarily inserted into the pCR2.1 vector (Invitrogen) and stored. Herein, this vector is referred to as pCR2.1-HC (wild type).

The PCR reaction conditions were as shown below:

| | |
|---|---|
| Human lymphocyte cDNA | 4 μL |
| 2.5 mM dNTPs | 4 μL |
| hIgG1 HCF primer (20 μM) | 2.5 μL |
| hIgG1 HCR primer (20 μM) | 2.5 μL |
| DMSO | 2.5 μL |
| x10 pfu polymerase Buffer | 5 μL |
| pfu polymerase | 1 μL |
| Sterile water | 28.5 μL |
| Total | 50 μL |

94°C for 2 min;
94°C for 1 min, 55°C for 2 min, 72°C for 2 min (30 cycles);
72°C for 4 min; and
4°C for an unlimited time.

DNA sequences of primers are shown below.

hIgG1 HCF primer: GCCTCCACCAAGGGCCCATCGGTC (SEQ ID NO: 28)
hIgG1 HCR primer: TTATTTACCCGGAGACAGGGAGAGGCT (SEQ ID NO: 29)

B) Mutagenesis of a cloned gene:

To obtain a 295Cys chimeric antibody, a mutation must be introduced into a specific position in the cloned human IgG1 H chain constant region. Unless otherwise indicated, amino acids are herein numbered according to the EU Index numbers of human IgG1 H chain constant region, indicated in "Sequence of Proteins of Immunological Interest" (NIH Publication No.91-3242, 1991) by Elvin A. Kabat *et al.* To replace the 295th Gln by Cys, mutagenesis was performed by a PCR method using pCR2.1-HC (wild type), a pCR2.1 vector with the cloned human IgG1 H-chain constant region inserted, as a template. Details of this mutagenesis method are described below.

| Mutagenesis to 295 (Gln→Cys) | |
|---|---|
| pCR2.1-HC (wild type) (25 ng/μL) | 2 μL |
| 2.5 mM dNTPs | 4 μL |
| 295Cys1 primer (20 μM) | 1.25 μL |
| 295Cys2 primer (20 μM) | 1.25 μL |
| x10 pfu polymerase Buffer | 5 μL |
| pfu polymerase | 1 μL |
| Sterile water | 35.5 μL |

(continued)

| Mutagenesis to 295 (Gln→Cys) | |
|---|---|
| Total | 50 μL |

95°C for 30 sec
95°C for 30 sec, 55°C for 1 min, 68°C for 13 min (12 cycles)
68°C for 4 min
4°C for an unlimited time

The nucleotide sequences of primers are as shown below.

295Cys1 primer: ACAAAGCCGCGTGAGGAGTGCTACAACAGCACGTACCGT (SEQ ID NO: 30)
295Cys2 primer: ACGGTACGTGCTGTTGTAGCACTCCTCACGCGGCTTTGT (SEQ ID NO: 31)

After the above PCR reaction was completed, 1 μL of Dpn I (New England BioLabs) was added to the reaction solution, and it was incubated at 37°C for two hours. Using the solution after the incubation, *Escherichia coli* JM109 was transformed, and plasmids were purified from the transformed *E. coli.* The DNA sequence of the purified plasmids was confirmed, thus obtaining the pCR2.1 vector inserted with human IgG 1 H chain constant region carrying the 295 mutation (Gln→Cys). This vector with the mutation was named pCR2.1-HC (295Cys).

C) Construction of chimeric antibody-expression vectors into which a cloned gene or its mutated gene is combined:

The expression of a single kind of anti-CD20 chimeric antibody is achieved via transfection of two kinds of expression vectors, those for the L and H chains of anti-CD20 chimeric antibody, into CHO cells. Herein, the expression vector of the L chain of anti-CD20 chimeric antibody refers to an expression vector into which mouse anti-CD20 L chain variable region gene + human IgG 1 L chain constant region gene is combined and incorporated, and the expression vector of the H chain of anti-CD20 chimeric antibody refers to an expression vector into which mouse anti-CD20 H chain variable region gene + human IgG 1 H chain constant region gene is combined and incorporated. When preparing wild-type and 295Cys-type anti-CD20 chimeric antibodies, one expression vector of the L chain of anti-CD20 chimeric antibody is used commonly for the expression of both chimeric antibodies; however, regarding the expression vector of the H chain of anti-CD20 chimeric antibodies, dedicated H-chain expression vectors for each of wild-type and 295Cys-type chimeric antibodies are required. Herein, the construction of an anti-CD20 chimeric antibody L chain expression vector, which can be used commonly for the expression of each chimeric antibody, is described in detail. Next, the construction of dedicated anti-CD20 chimeric antibody H chain expression vectors, required for the expression of wild-type and 295Cys-type chimeric antibodies, is described.

Construction of an anti-CD20 chimeric antibody L chain expression vector:

BCMGneo vector is used as an expression vector; and mouse anti-CD20 L chain variable region gene + human IgG 1 L chain constant region gene are inserted into the Xho I and Not I sites of this vector. A fragment of mouse anti-CD20 L chain variable region gene was obtained by conducting the following PCR reaction using pCR2.1-MLV prepared above as a template. Further, a fragment of human IgG1 L chain constant region gene was obtained by conducting the following PCR reaction using pCR2.1-LC prepared above as a template.

| (Amplification reaction of mouse anti-CD20 L chain variable region gene) | |
|---|---|
| pCR2.1-MLV (20 ng/μL) | 2 μL |
| 2.5 mM dNTPs | 4 μL |
| L 1 primer (20 μM) | 2.5 μL |
| L2 primer (20 μM) | 2.5 μL |
| DMSO | 2.5 μL |
| x10 pfu polymerase Buffer | 5 μL |
| pfu polymerase | 1 μL |
| Sterile water | 30.5 μL |
| Total | 50 μL |

94°C for 2 min
94°C for 1 min, 55°C for 2 min, 72°C for 2 min (20 cycles)
72°C for 4 min
4°C for an unlimited time

L1 primer: ACCGCTCGAGATGGATTTTCAGGTGCAGATTATCAGC (SEQ ID NO: 32)
L2 primer: TTTCAGCTCCAGCTTGGTCCCAGCACC (5'-phosphorylated) (SEQ ID NO: 33)

(Amplification reaction of human IgG1 L chain constant region gene)

| | |
|---|---|
| pCR2.1-LC (20 ng/μL) | 2 μL |
| 2.5 mM dNTPs | 4 μL |
| L3 primer (20 μM) | 2.5 μL |
| L4 primer (20 μM) | 2.5 μL |
| DMSO | 2.5 μL |
| x10 pfu polymerase Buffer | 5 μL |
| pfu polymerase | 1 μL |
| Sterile water | 30.5 μL |
| Total | 50 μL |

94°C for 2 min

94°C for 1 min, 55°C for 2 min, 72°C for 2 min (20 cycles)

72°C for 4 min

4°C for an unlimited time

L3 primer: ACTGTGGCTGCACCATCTGTCTTCATC (5'-phosphorylated) (SEQ ID NO: 34)
L4 primer: ATAGTTTAGCGGCCGCTTAACACTCTCCCCTGTTGAAGCTCTTTGT (SEQ ID NO: 35)

The fragment of the mouse anti-CD20 L chain variable region gene, obtained in the above PCR reaction, was digested with restriction enzyme Xho I (Takara) and purified. Further, the fragment of the human IgG1 L chain constant region gene, obtained in the PCR reaction, was digested with restriction enzyme Not I (Takara) and purified. The fragment that was obtained from BCMGneo vector via digestion with Xho I and Not I and purification was mixed with the above-described purified mouse anti-CD20 L chain variable region gene fragment and the purified human IgG 1 L chain constant region gene fragment, for ligation. Sequencing demonstrated that the fragment of interest was inserted into the vector obtained by this ligation. This expression vector of the anti-CD20 chimeric antibody L chain was named p-chimeric-LC.

Construction of an anti-CD20 chimeric antibody H chain expression vector:

BCMGneo vector is used as an expression vector; and mouse anti-CD20 H chain variable region gene + human IgG 1 H chain constant region gene are inserted into the Xho I and Not I sites of this vector. A fragment of mouse anti-CD20 H chain variable region gene was obtained by the following PCR reaction using pCR2.1-MHV prepared above as a template. For dedicated H chain expression vectors for each of wild-type and 295Cys-type chimeric antibodies, different plasmids were used as templates in the PCR amplification of human IgG1 H chain constant region gene; however, each fragment of human IgG 1 H chain gene was obtained by exactly the same procedures. Details, including the PCR reactions, will be described below

(Amplification reaction of mouse anti-CD20 H chain variable region gene)

| | |
|---|---|
| pCR2.1-MHV (20 ng/μL) | 2 μL |
| 2.5 mM dNTPs | 4 μL |
| H1 primer (20 μM) | 2.5 μL |
| H2 primer (20 μM) | 2.5 μL |

(continued)

| (Amplification reaction of mouse anti-CD20 H chain variable region gene) | |
|---|---|
| DMSO | 2.5 μL |
| x10 pfu polymerase Buffer | 5 μL |
| pfu polymerase | 1 μL |
| Sterile water | 30.5 μL |
| Total | 50 μL |

94°C for 2 min
94°C for 1 min, 55°C for 2 min, 72°C for 2 min (20 cycles)
72°C for 4 min
4°C for an unlimited time

H1 primer: ACCGCTCGAGATGGGATGGAGCTGGGTCTTTCTCTTC (SEQ ID NO: 36)
H2 primer: TGAGGAGACGGTGACCGTGGTCCC (5'-phosphorylated) (SEQ ID NO: 37)

| (Amplification reaction of human IgG 1 H chain constant region gene) | |
|---|---|
| # Each template plasmid (20 ng/μL) | 2 μL |
| 2.5 mM dNTPs | 4 μL |
| H3 primer (20 μM) | 2.5 μL |
| H4 primer (20 μM) | 2.5 μL |
| DMSO | 2.5 μL |
| x10 pfu polymerase Buffer | 5 μL |
| pfu polymerase | 1 μL |
| Sterile water | 30.5 μL |
| Total | 50 μL |

94°C for 2 min
94°C for 1 min, 55°C for 2 min, 72°C for 2 min (20 cycles)
72°C for 4 min
4°C for an unlimited time

H3 primer: GCCTCCACCAAGGGCCCATCGGTC (5'-phosphorylated) (SEQ ID NO: 38)
H4 primer: ATAGTTTAGCGGCCGCTTATTTACCCGGAGACAGGGAGAGGCTCTT (SEQ ID NO: 39)

#: To prepare a gene fragment for wild-type anti-CD20 chimeric antibody, pCR2.1-HC (wild type) was used as a template. To prepare a gene fragment for 295Cys-type anti-CD20 chimeric antibody, pCR2.1-HC (295Cys) was used as a template.

The mouse anti-CD20 H chain variable region gene fragment, obtained in the above PCR reaction, was digested with restriction enzyme Xho I (Takara) and purified. Further, the human IgG1 H chain constant region gene fragments, obtained in each of PCR reactions using different templates, were digested with restriction enzyme Not I (Takara) and purified. The fragment that was obtained from BCMGneo vector via digestion with Xho I and Not I and purification was mixed with the above described purified mouse anti-CD20 H chain variable region gene fragment and a purified human IgG 1 H chain constant region gene fragment for ligation. Sequencing demonstrated that a fragment of interest was inserted into each of the vectors obtained by this ligation. These expression vectors of anti-CD20 chimeric antibody H chain were named p-chimeric-HC (wild type) and p-chimeric-HC (295Cys).

D) Gene introduction into CHO cells with chimeric antibody-expression vectors:

The constructed various expression vectors were used in the following combinations for gene introduction into CHO cells with TransIT-CHO Transfection kit (Mirus, MIR2170). Wild-type chimeric antibody: p-chimeric-LC + p-chimeric-HC (wild type) 295Cys chimeric antibody: p-chimeric-LC + p-chimeric-HC (295Cys)

(ELISA measurement)

Cells expressing anti-CD20 chimeric antibody at a high level were screened and selected by ELISA.

E) Culture of chimeric antibody-expressing CHO cells

The chimeric antibody-expressing CHO cells, selected by ELISA measurement, were grown by culturing under the conditions of 37°C, 5% $CO_2$ in Dulbecco's Modified Eagle's Medium supplemented with 10% fetal bovine serum and 0.1 mg/mL geneticin. The fully-grown chimeric antibody-expressing CHO cells were subjected to culturing in a serum-free medium, CHO-S-SFMII (GIBCO, 12052-098), supplemented with 0.1 mg/mL geneticin, and the culture was continued in approximately 1000 mL.

F) Column purification from culture supernatant of the chimeric antibody-expressing cells:

Culture supernatant of the chimeric antibody-expressing CHO cells cultured in a serum-free medium, CHO-S-SFMII, was collected. As the Fc region structure of wild-type chimeric antibody was not changed, the wild-type chimeric antibody was purified using a Protein A column that is conventional in the art of antibody purification. However, since the 295Cys-type chimeric antibody was mutated within its Fc region structure, it carries structural change in its Fc region and is hardly adsorbed to the usual Protein A column. For this reason, the Cys-type chimeric antibody was purified using a Protein L column. The variable region of the anti-CD20 chimeric antibody, prepared herein, is κ chain. This Protein L is a protein that can recognize and bind to this variable region κ chain. Hereinafter, Protein A column purification of the wild-type chimeric antibody and Protein L column purification of the Cys-type chimeric antibody will be described in detail.

Protein A column purification of the wild-type chimeric antibody

1) The culture supernatant, obtained by culturing wild-type chimeric antibody-expressing CHO cells in 1000 mL of CHO-S-SFMII medium, was concentrated from 1000 mL to 100 mL by using Amicon Stirred Ultrafiltration Cell (Millipore, Model 8400) equipped with Amicon Ultrafiltration Membranes (Millipore, Code YM10).
2) To the concentrated culture supernatant, 8 mL of rProtein A Sepharose Fast Flow (Amersham Bioscience, 17-1279-03), which is a Protein A-Sepharose, was added. This was stirred at 4°C for two days to allow the wild-type chimeric antibody to be adsorbed to Protein A.
3) The Protein A-Sepharose to which the wild-type chimeric antibody was adsorbed was filled into a column of 1.5 cm diameter and 8 cm length.
4) The column into which Protein A-Sepharose was filled was washed with 100 mL of PBS, followed by elution of the wild-type chimeric antibody from the column with elution buffer (0.17 M Glycine-HCl, pH 2.3). An appropriate amount of 1 M Tris-HCl (pH8.5) was added immediately to the eluted solution of the wild-type chimeric antibody to neutralize pH.
5) The eluate of the wild-type chimeric antibody was put into a dialysis tube, Cell Sep T2 (Membrane filtration products Inc, 8030-23), and dialyzed against 5 L of PBS at 4°C. After dialysis, the collected wild-type chimeric antibody was used as a purified preparation.

Protein L column purification of the Cys-type chimeric antibody:

1) The culture supernatant, obtained by culturing Cys-type chimeric antibody-expressing CHO cells in 1000 mL of CHO-S-SFMII medium, was concentrated from 1000 mL to 100 mL by using Amicon Stirred Ultrafiltration Cell (Millipore, Model 8400) equipped with Amicon Ultrafiltration Membranes (Millipore, Code YM10).
2) 100 mL of Protein L Binding Buffer (0.1 M Phosphate, 0.15 M NaCl, pH 7.2) was added to 100 mL of the concentrated culture supernatant and mixed.
3) 2 mL of ImmunoPure Immobilized Protein L Plus (PIERCE, 20250), which is a Protein L-Sepharose, was filled into a column of 1.5 cm diameter and 8 cm length.
4) The solution prepared in 2) was loaded from the top of the column to flow out slowly from the bottom of the column. This procedure allowed the Cys-type chimeric antibody to be adsorbed to Protein L-Sepharose. Subsequently, the column was washed with 50 mL of Protein L Binding Buffer to remove excessive proteins adsorbed to the column.
5) The Cys-type chimeric antibody was eluted from the column with elution buffer (0.17 M Glycine-HCl, pH 2.3). An

appropriate amount of 1 M Tris-HCl pH8.5 was added immediately to the eluted solution of the Cys-type chimeric antibody to neutralize pH.
6) The eluate of the Cys-type chimeric antibody was put into a dialysis tube, Cell Sep T2 (Membrane filtration products Inc, 8030-23), and dialyzed against 5 L of PBS at 4°C. After dialysis, the collected Cys-type chimeric antibody was used as a purified preparation.

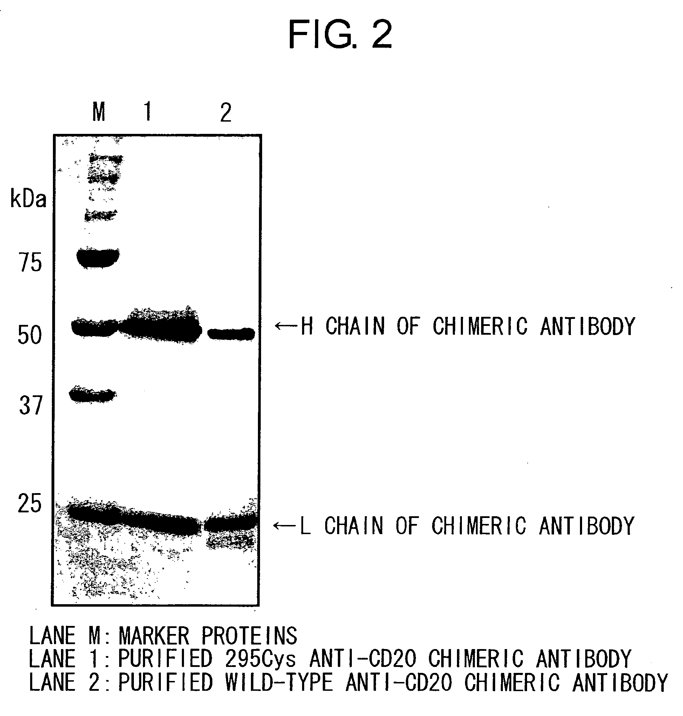

The wild-type chimeric antibody and Cys-type chimeric antibody purified as described above were electrophoresed on 12.5% SDS-PAGE and silver-stained. The electrophoretic profile is shown in Fig. 2.

<u>Flow cytometric analysis of purified chimeric antibodies:</u>

To confirm that the reactivity of each anti-CD20 chimeric antibodies, obtained by column purification, to CD20 molecule is the same, flow cytometric analysis was conducted. Raji cells, which are neoplastic B cells, are known to have many CD20 molecules on their cell membrane surfaces. In this flow cytometric analysis, whether or not each of the anti-CD20 chimeric antibodies could recognize CD20 molecules on Raji cells was examined. Hereinafter, the measurement method will be described in detail.

1) 10 mL of Raji cells (cell count: $2 \times 10^7$), cultured in RPMI-1640 (Sigma, R8758) medium supplemented with 10% fetal bovine serum, was centrifuged (1200 rpm, 5 min). The supernatant was removed and a pellet of Raji cells was obtained.
2) 10 mL of wash buffer (PBS + 10% FCS) was added to the pellet of Raj cells to suspend cells. This was centrifuged (1200 rpm, 5 min) again and the supernatant was removed.
3) The pellet of Raji cells was suspended in 2 mL of wash buffer, and 0.3 mL each of the cell suspension was put into tubes. After further adding 0.5 mL of wash buffer into each tube for suspension, centrifugation (2000 rpm, 5 min) was performed and the supernatant was discarded.
4) 5 μl each of purified anti-CD20 chimeric antibodies was added to the pellet of Raji cells, and allowed to react at room temperature for 30 minutes.

(i) 1 mL of wash buffer was added for suspension; centrifugation (2000 rpm, 5 min) was conducted; and the supernatant was discarded.
(ii) 30 μL of anti-human IgG (γ-chain) FITC conjugated (MBL, code 214), which was 50-fold diluted with wash buffer, was added to the Raji cells and allowed to react at room temperature for 15 minutes.
(iii) 1 mL of wash buffer was added for suspension; centrifugation (2000 rpm, 5 min) was conducted; and the supernatant was discarded.
(iv) 0.5 mL of wash buffer was added to the pellet of Raji cells for suspension, and flow cytometric analysis was conducted by FACSCalibur (Becton Dickinson).

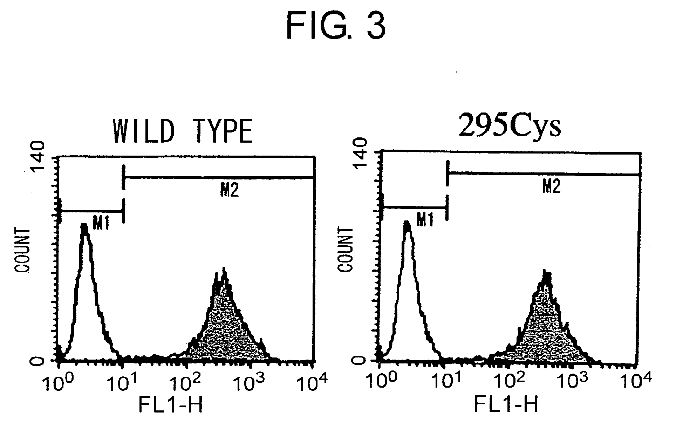

The results of the above flow cytometric analysis are shown in Fig. 3. The results demonstrate that the reactivity to CD20 was the same for the wild-type and the 295Cys-type chimeric antibodies.

[Example 3] Preparation of anti-EGFR antibodies:

Since the ADCC activity of the 295Cys-type anti-CD20 chimeric antibody was very high as compared with that of the wild-type anti-CD20 chimeric antibody, wild-type and 295Cys-type anti-EGFR antibodies were newly prepared to examine whether or not they exhibit the same trend as the anti-CD20 chimeric antibodies.

<u>(Construction of expression vectors of human anti-EGFR antibodies)</u>

For the expression of wild-type and 295Cys-type human anti-EGFR antibodies in CHO cells, three kinds of expression vectors (human anti-EGFR antibody L chain-expression vector, wild-type human anti-EGFR antibody H chain-expression vector, and 295Cys-type human anti-EGFR antibody H chain-expression vector) were constructed as expression vectors. The construction of each expression vector will be described in detail below.

*1) Construction of a human anti-EGFR antibody L chain-expression vector:*

A DNA fragment was obtained by PCR reaction using the primers given below with the gene including the variable region and the constant region of human anti-EGFR antibody L chain (The DNA sequence is shown in SEQ ID NO: 90), as a template.

EGFR-L1 primer: ACCGCTCGAGATGGACATGAGGGTCCCCGCTCAGCTC (SEQ ID NO: 40)
EGFR-L2 primer: ATAGTTTAGCGGCCGCTTACGAACATTCTGTAGGGGCCACTGTCTT (SEQ ID NO: 41)

The DNA fragment obtained in the PCR reaction was purified by ethanol precipitation and then treated with restriction enzymes Xho I (TAKARA BIO INC., 1094A) and Not I (TAKARA BIO INC., Code 1166A). Similarly, BCMG-neo vector was treated with restriction enzymes Xho I and Not I. Next, the PCR-amplified DNA fragment and the BCMG-neo vector, treated with the restriction enzymes, were electrophoresed on a 1% agarose gel containing ethidium bromide. Among the DNA fragments visualized by UV irradiation after the electrophoresis, the DNA fragments of the desired sizes were excised from the gel, extracted by using SUPREC-01 (TAKARA BIO INC., code 9040), and purified. The PCR-amplified fragment and the BCMG-neo vector, purified from the excised gel, were subjected to ligation reaction using DNA Ligation kit Ver.2.1 (TAKARA BIO INC., code 6022). The DNA solution after the ligation reaction was used for transformation into *Escherichia coli* JM109. Plasmid DNA was purified by alkali SDS method from the obtained transformants. Sequence analysis was conducted on the purified plasmid to confirm that it contained the L chain (variable region and constant region) gene of human anti-EGFR antibody, and the plasmid was used as a human anti-EGFR antibody L chain-expression vector.

*2) Construction of a wild-type human anti-EGFR antibody H chain-expression vector and a 295Cys-type human anti-EGFR antibody H chain-expression vector:*

A DNA fragment was obtained by PCR reaction using the primers given below with the gene including the variable region of human anti-EGFR antibody H chain (the DNA sequence is shown in SEQ ID NO: 42), as a template.

EGFR-H1 primer: ACCGCTCGAGATGAAACACCTGTGGTTCTTCCTC (SEQ ID NO: 43)
EGFR-H2 primer: CGAGACGGTGACCATTGTCCCTTG (5'-phosphorylated) (SEQ ID NO: 44)

The DNA fragment obtained in the PCR reaction was purified by ethanol precipitation and treated with restriction enzyme Xho I (TAKARA BIO INC., 1094A). Next, the PCR-amplified DNA fragment, treated with the restriction enzyme, was electrophoresed on a 1% agarose gel containing ethidium bromide. Among the DNA fragments visualized by UV irradiation after the electrophoresis, the DNA fragment of the desired size was excised from the gel, extracted by using the SUPREC-01 (TAKARA BIO INC., code 9040), and purified. This purified DNA fragment is the human anti-EGFR antibody H chain variable region gene.

As the human IgG 1 H chain constant region gene, necessary for the preparation of human anti-EGFR antibody, the same DNA fragment that was used for the preparation of anti-CD20 chimeric antibody was used. A DNA fragment was obtained by PCR amplification using H3 and H4 primers with plasmid pCR2.1-HC (wild type) as a template. The DNA fragment was treated with restriction enzyme Not I and purified. This DNA fragment is the wild-type human anti-EGFR antibody H chain constant region gene. The DNA fragment that was obtained by PCR amplification using H3 and H4 primers with plasmid pCR2.1-HC (295Cys) as a template, followed by treatment with restriction enzyme Not I and purification, is the 295Cys-type human anti-EGFR antibody H chain variable region gene.

The DNA fragments of the human anti-EGFR antibody H chain variable region gene and the wild-type human anti-EGFR antibody H chain constant region gene, prepared as described above, were mixed with the DNA fragment that was prepared from BCMG-neo vector via digestion with Xho I and Not I and purification, and subjected to ligation reaction using DNA Ligation kit Ver.2.1 (TAKARA BIO INC., code 6022). The DNA solution after the ligation reaction was used for transformation into *Escherichia coli* JM109. Plasmid DNA was purified by alkali SDS method from the obtained transformants. Sequence analysis was conducted on the purified plasmid to confirm that it contained the wild-type H chain (variable region and constant region) gene of human anti-EGFR antibody, and the plasmid was used as a wild-type human anti-EGFR antibody H chain-expression vector. Similarly, the DNA fragments of the human anti-EGFR antibody H chain variable region gene and the 295Cys-type human anti-EGFR antibody H chain constant region gene were mixed with the DNA fragment that was prepared from the BCMG-neo vector via digestion with Xho I and Not I and purification, and subjected to ligation reaction using DNA Ligation kit Ver.2.1 (TAKARA BIO INC., code 6022). The DNA solution after the ligation reaction was used for transformation into *Escherichia coli* JM109. Plasmid DNA was purified by alkali SDS method from the obtained transformants. Sequence analysis was conducted on the purified plasmid to confirm that it contained the 295Cys-type H chain (variable region and constant region) gene of human anti-EGFR antibody, and the plasmid was used a 295Cys-type human anti-EGFR antibody H chain-expression vector.

(Introduction of human anti-EGFR antibody expression vectors into CHO cells)

The constructed expression vectors were used in the following combinations for gene introduction into CHO cells with TransIT-CHO Transfection kit (Mirus, MIR2170).

For the production of wild-type human anti-EGFR antibody in CHO cells:

human anti-EGFR antibody L chain-expression vector + wild-type human anti-EGFR antibody H chain-expression vector

For the production of 295Cys-type human anti-EGFR antibody in CHO cells:

human anti-EGFR antibody L chain-expression vector + 295Cys-type human anti-EGFR antibody H chain-expression vector

(Culture of CHO cells with high expression of human anti-EGFR antibodies)

Clones with high expression of human antibodies were selected in exactly the same manner as in the preparation of the anti-CD20 chimeric antibodies described above, and the selected CHO cells with high expression of human antibodies were cultured.

(Column purification from the culture supernatants of the human anti-EGFR antibody-expressing_ CHO cells)

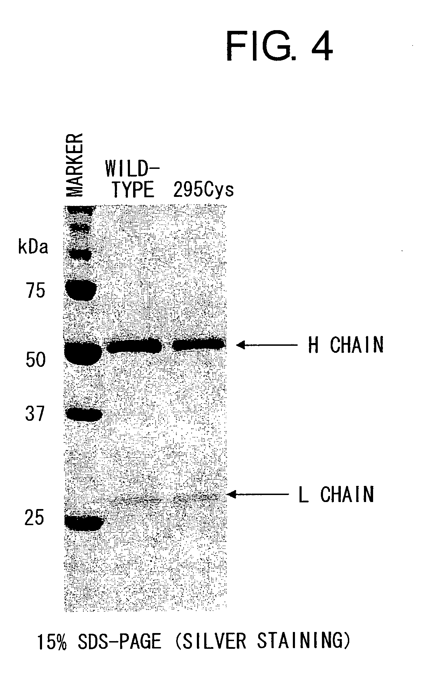

Wild-type and 295Cys-type human anti-EGFR antibodies were obtained at high purity by Protein A column purification in the same manner as in the purification of the wild-type anti-CD20 chimeric antibody. The obtained human anti-EGFR antibodies (wild type and 295Cys type) were electrophoresed on 15% SDS-PAGE and silver-stained. The electrophoretic profile is shown in Fig. 4.

(Flow cytometric analysis of purified anti-EGFR antibodies)

EGFR (epidermal growth factor receptor) is a 170 kDa membrane protein expressed in epithelial tissue, and is also known as HER-1. Since this EGFR is known to be highly expressed in squamous cell carcinoma (lung cancer), human anti-EGFR antibody is expected as a candidate of therapeutic agent for lung cancer. Based on these backgrounds, whether or not NCI-H226, squamous cell carcinoma cells derived from lung cancer, could be stained with the human anti-EGFR antibody prepared herein was examined by flow cytometric analysis. Details of the experimental procedures will be described below.

1) Using a 75 $cm^2$ dish as a culture container, NCI-H226 cells were cultured in RPMI-1640 (Sigma, R8758) medium supplemented with 10% fetal bovine serum in a $CO_2$ incubator at 37°C.
2) Since the cultured NCI-H226 cancer cells are adherent cells, they had to be detached from the dish. Thus, after discarding the medium, the cells were detached by adding 5 mL of Cell Dissociation Buffer Enzyme-Free PBS-based (GIBCO, 13151-014) and by incubating in a $CO_2$ incubator at 37°C for five minutes.
3) The solution including NCI-H226 cells detached from the dish was centrifuged (1200 rpm, 5 min), and the supernatant was removed to recover a NCI-H226 cell pellet.
4) 10 mL of wash buffer (PBS + 2% FCS) was added to the NCI-H226 cell pellet to suspend cells. This was centrifuged (1200 rpm, 5 min) again and the supernatant was removed.
5) The pellet of NCI-H226 cells was suspended in 2 mL of wash buffer, and 0.3 mL each of the cell suspension was put into tubes. After further adding 0.5 mL of wash buffer into each tube for suspension, centrifugation (2000 rpm, 5 min) was performed and the supernatant was discarded.
6) 5 $\mu$L each of purified, wild-type or 295Cys-type human anti-EGFR antibody was added to the NCI-H226 cell pellet, and allowed to react at room temperature for 30 minutes.
7) 1 mL of wash buffer was added for suspension; centrifugation (2000 rpm, 5 min) was conducted; and the supernatant was discarded.
8) 30 $\mu$L of anti-human IgG ($\gamma$-chain) FITC conjugated (MBL, code 214), which was 50-fold diluted with wash buffer, was added to the NCI-H226 cells and allowed to react at room temperature for 15 minutes.
9) 1 mL of wash buffer was added for suspension; centrifugation (2000 rpm, 5 min) was conducted; and the supernatant was discarded. Finally, 0.5 mL of wash buffer was added to the pellet of NCI-H226 cells for suspension, and flow cytometric analysis was conducted by FACSCalibur (Becton Dickinson).

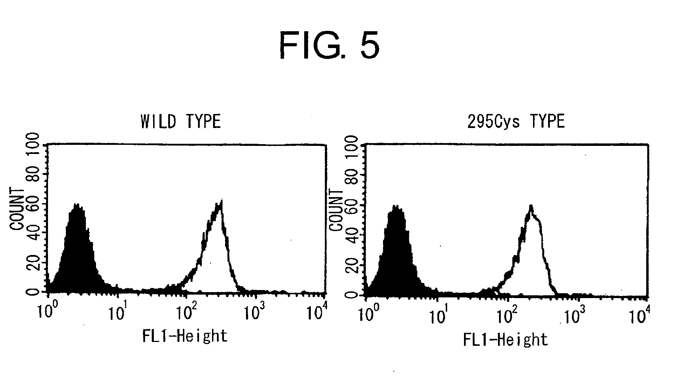

The results of the above flow cytometric analysis are shown in Fig. 5. From these results, it can be said that EGFR is highly expressed on the cell surface of NCI-H226, squamous cell carcinoma cells derived from lung cancer, since NCI-H226 was strongly stained with the wild-type or 295Cys-type human anti-EGFR antibody. Further, the ability to recognize EGFR on the cell surface was considered to be the same for the wild-type and 295Cys-type human anti-EGFR antibodies since the degree of the staining was the same between them.

[Example 4] AILIM/ICOS-IgFc and AILIM/ICOS-IgFc Cys mutant

*1. Preparation of an AILIM/ICOS-IgFc chimeric molecule-expression vector:*

*1-1. Construction of cDNA encoding the extracellular domain region of AILIM/ICOS:*

cDNA encoding the extracellular domain region of AILIM/ICOS was isolated from mRNA of activated T cells by a PCR method according to conventional procedures using primers designed based on the known sequence information of AILIM/ICOS. Specific examples are described in additional detail below.

From the human peripheral blood mononuclear cells obtained in Preparation Example 1, T cells were purified using PanT-Isolation Kit (Miltenyi) according to the manufacturer's instructions. The purified T cells were plated at $10^5$ cells/well on an ELISA plate that was coated at 37°C for one hour with a solution which contained anti-CD3 antibody (clone OKT3, Ortho Biotech) and anti-CD28 antibody (Clone 28.2, BD), diluted with $Ca^{2+}$- and $Mg^{2+}$-free phosphate buffered saline (PBS (-)) at final concentrations of 1 μg/ml and 5 μg/ml, respectively. The cells were cultured in a $CO_2$ incubator containing 5% $CO_2$ at 37°C for 24 hours, to obtain activated T cells. RNA was extracted from the activated T cells using TRIzol Reagent (Invitrogen), and 1 μg of this RNA was used as a template to synthesize cDNA using ReverTra Ace-a (Toyobo Co., Ltd.) according to the manufacturer's instructions. Using 0.2 μl of this solution of synthesized cDNA and 50 pmol each of the following sense primer (SEQ ID NO: 45) and antisense primer (SEQ ID NO: 46) as synthetic oligonucleotides, 35 cycles of PCR reaction were performed in a reaction system having a final volume of 50 μl, to which system 0.25 μl of ExTaq polymerase (TaKaRa) and 5 μl of the buffer attached to the ExTaq polymerase had been added.

SEQ ID NO: 45: 5'-tgttgctagcaaacatgaagtcaggcctc-3' (Nhe I sequence was added to the sequence of AILIM/ICOS)
SEQ ID NO: 46: 5'-aacggatccttcagctggcaacaaag-3' (Bam HI sequence was added to the sequence of AILIM/ICOS)

After the PCR reaction, electrophoresis was performed in a 1% agarose gel according to conventional procedures, and an approximately 0.45 kbp cDNA fragment was recovered and purified using Wizard SV Gel and PCR Clean-up System (Promega) according to the manufacturer's instructions. cDNA concentration was calculated from the absorbance at 260 nm (1 O.D. 260 = 50 μg/ml). 1 μg of this fragment was digested with ten units each of restriction enzymes Nhe I and Bam HI at 37°C for three hours according to the manufacturer's instructions, and the approximately 0.45 kbp cDNA fragment of AILIM/ICOS extracellular region, having the termini digested with restriction enzymes Nhe I and Bam HI, was recovered in the same manner as described above.

*1-2. Construction of cDNA encoding human IgFc:*

As cDNA encoding the Fc region of human immunoglobulin 1 (IgFc), cDNA encoding the amino acid sequence of IgFc shown in GenBank Accession No. J00228 was constructed as follows using PCR method. First, primer binding nucleotide sequences for PCR amplification (including restriction enzyme Bam HI and Not I sequences at the 5' and 3' ends of the IgFc cDNA, respectively) were added to the 5' and 3' ends of the cDNA encoding the amino acid sequence of IgFc of GenBank Accession No. J00228. The designed nucleotide sequence was divided into eight nucleotide segments of about 100 bases, starting from the 5' end with adjacent nucleotide sequences have overlapping sequences of about 20 bases at the ends. Each of the sequences were synthesized in the alternative order of sense strand and antisense strand to give rise to oligonucleotides including the following sense primer (SEQ ID NO: 47) and antisense primer (SEQ ID NO: 48). Each of synthesized oligonucleotides was added to the PCR reaction solution at a final concentration of 0.1 μM, and 0.25 μl of ExTaq polymerase (TaKaRa) and 5 μl of the buffer attached to ExTaq polymerase were added to the reaction system having a final volume of 50 μl to perform 30 cycles of PCR reaction.

SEQ ID NO: 47: 5'-tgaaggatcccgaggagcccaaatcttgtgacaa-3' (Bam HI sequence was added to the sequence of IgFc)
SEQ ID NO: 48: 5'-gaagcggccgctcatttacccggagacagggagaggctc-3' (Not I sequence was added to the sequence of IgFc)

After the PCR reaction, electrophoresis was performed in a 1% agarose gel according to conventional procedures, and an approximately 0.45 kbp cDNA fragment was recovered and purified using Wizard SV Gel and PCR Clean-up System (Promega) according to the manufacturer's instructions. cDNA concentration was calculated from the absorbance at 260 nm (1 O.D. 260 = 50 μg/ml). 1 μg of this fragment was digested with ten units each of restriction enzymes Bam HI and Not I at 37°C for three hours according to the manufacturer's instructions, and the approximately 0.76 kbp cDNA fragment of IgFc region, having the termini digested with restriction enzymes Bam HI and Not I was recovered in

the same manner as described above.

*1-3. Construction of an AILIM/ICOS-IgFc chimeric molecule-expression vector:*

To construct an expression vector with these cDNA fragments incorporated, 1 μg of expression vector pIRES-puro2 (Clontech) was digested with ten units each of restriction enzymes Nhe I and Not I at 37°C for three hours according to the manufacturer's instructions, and then pIRES-puro2 cDNA fragment with termini digested with restriction enzymes Nhe I and Not I was obtained in the same manner as described above. This fragment, the approximately 0.45 kbp cDNA fragment of AILIM/ICOS extracellular region with the termini digested with restriction enzymes Nhe I and Bam HI, and the approximately 0.76 kbp cDNA fragment of the IgFc region with the termini digested with restriction enzymes Bam HI and Not I were ligated by using Ligation High (Toyobo Co., Ltd.) according to the manufacturer's instructions, and then were transformed into *Escherichia coli* DH5α for transformation (Toyobo Co., Ltd.). The obtained colonies were liquid-cultured in NZY broth for 16 hours. Plasmids were purified from the transformed E. *coli* using Wizard plus SV Minipreps DNA Purification Kit (Promega). Subsequently, the DNA sequence was confirmed by conducting a reaction using ABI 3100-Avant (Applied Biosystems) according to the manufacturer's instructions to obtain pIRESpuro2-AILIM/ICOS-IgFc, the vector expressing the cDNA of the chimeric molecule in which the extracellular region of AILIM/ICOS and IgFc are linked.

*2. Preparation of an AILIM/ICOS-IgFc Cys mutant-expression vector:*

To introduce a mutation altering the 223rd (the 295th in EUIndex of Kabat) glutamine residue (codon of DNA sequence, CAG) of Quick Change Site-Directed AILIM/ICOS-IgFc molecule to cysteine residue (codon of DNA sequence, TGC), the following sense primer (SEQ ID NO: 49) and antisense primer (SEQ ID NO: 50) were synthesized, and pIRESpuro2-AILIM/ICOS-IgFc was used as a template to perform reaction using Mutagenesis Kit (STRATAGENE) according to the manufacturer's instructions.

SEQ ID NO: 49: 5'-caaagccgcgggaggagtgctacaacagcacgtaccgg-3'
SEQ ID NO: 50: 5'-ccggtacgtgctgttgtagcactcctcccgcggctttg-3'

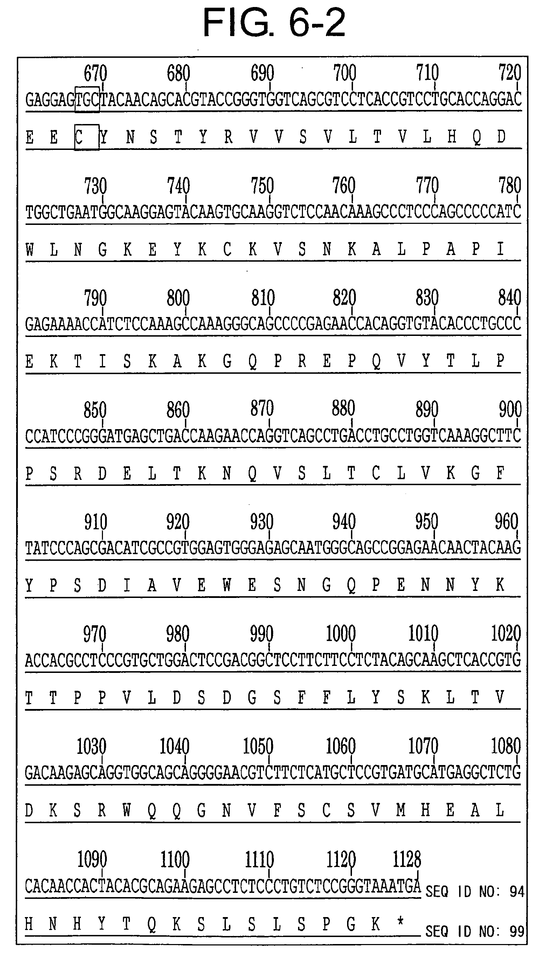

After the mutagenesis, the colonies obtained by transformation of competent *Escherichia coli* were liquid-cultured in NZY broth for 16 hours. Plasmids were purified from the transformed *E. coli* using Wizard plus SV Minipreps DNA Purification Kit (Promega). Subsequently, the DNA sequence was confirmed by reaction using ABI 3100-Avant (Applied Biosystems) according to the manufacturer's instructions, and the expression vector, pIRES-puro2 AILIM-IgFc Cys mutant, having a mutation at the relevant position only was obtained. A nucleotide sequence encoding AILIM-IgFc Cys is shown in SEQ ID NO: 94, and the amino acid sequence generated from this nucleotide sequence is shown in SEQ ID NO: 99 (See Figs. 6-1 and 6-2). The 144th to the 375th amino acids in the amino acid sequence indicated in SEQ ID NO: 99 correspond to the 216th to the 447th amino acids by the EUIndex number by Kabat. In Figs. 6-1 and 6-2, the sequence of the extracellular region of AILIM/ICOS is underlined with a wavy line, and that of the human IgGI Fc region is underlined. The position where the 295th glutamine by the EUIndex number by Kabat (the 223th in the amino acid sequence of SEQ ID NO: 99) was replaced by cysteine is boxed. Here, regarding the mutant of the chimeric molecule, the amino acid sequence before the introduction of the mutation is shown in SEQ ID NO: 102, and the nucleotide sequence encoding it is shown in SEQ ID NO: 101.

*3. Preparation of CHO-K1 cells expressing AILIM/ICOS-IgFc and AILIM/ICOS-IgFc Cys mutant:*

Gene introduction into 1 x $10^6$ CHO-K1 cells was performed using 36 μl of FuGENE and 12 μg of pIREpuro2-AILIM/ICOS-IgFc or pIRESpuro2-AILIM/ICOS-IgFc Cys mutant, according to the manufacturer's instructions. Two days after the gene introduction, puromycin was added at a final concentration of 0.5-5 μg/ml to RPMI-1640 medium supplemented with 10% fetal calf serum (FCS), followed by culturing for nine days. After selecting drug resistant CHO-K1 cells with puromycin, cells were cloned by the limiting dilution method, and cells with high expression of AILIM/ICOS-IgFc or AILIM/ICOS-IgFc Cys mutant were screened and selected by ELISA using anti-AILIM/ICOS antibody.

*4. Production and purification of AILIM/ICOS-IgFc and AILIM/ICOS-IgFc Cys mutant:*

Cells with high expression of AILIM/ICOS-IgFc and AILIM/ICOS-IgFc Cys mutant were cultured in RPMI-1640 medium supplemented with 10% FCS, and were grown to 60% of the maximum cell density of the culture dish. The

growth medium was removed by twice washing with $Ca^{2+}$- and $Mg^{2+}$ -free phosphate buffered saline (PBS (-)), and was replaced by RPMI-1640 supplemented with 0.1% BSA. The cells were cultured for three days, and the supernatant was collected. The supernatant was filtered through a 0.2 μm filter and let adsorbed to a Protein G column (Amersham Bioscience) by conventional procedures, followed by elution with 0.1 M glycin buffer (pH 2.8) and pH neutralization using 0.75 M Tris-HCl (pH 9.0). Subsequently, dialysis was performed with PBS (-) by conventional procedures. The concentrations of AILIM/ICOS-IgFc or AILIM/ICOS-IgFc Cys mutant in the samples were measured by an ELISA method using anti-AILIM/ICOS antibody.

(Evaluation method)

Measurement of antibody-dependent cellular cytotoxicity (ADCC) activity:

(1) Measurement of the ADCC activities for the anti-CD20 antibody and the Cys mutant thereof, obtained in Example 1:

In the measurement of ADCC activities by anti-CD20 antibodies, Daudi cells were used as target cells, and human peripheral blood mononuclear cells, effector cells. Daudi cells were suspended in 10% FCS RPMI-1640 medium at the concentration of $2 \times 10^5$ cells/ml by conventional procedures, and plated in 25 μl aliquots onto a U-bottom 96 well plate (Falcon). Anti-CD20 chimeric antibodies were diluted with 10% FCS RPMI-1640 medium at various concentrations, and plated in 25 μl aliquots onto the U-bottom 96 well plate (Falcon), followed by reaction at 37°C for one hour. Subsequently, peripheral blood mononuclear cells were added at $2.5 \times 10^5$ cells/well and cultured at 37 degrees for 16 hours. After the culturing, the culture plate was centrifuged, and 50 μl of the supernatant was collected from each well and transferred into a new flat bottom 96 well plate. 50 μl of assay buffer (CytoTox96 Non-Radioactive Cytotoxicity Assay, Promega) was added to each well, followed by reaction at room temperature for 30 minutes with light shielding. Subsequently, 50 μl of stop buffer (CytoTox96 Non-Radioactive Cytotoxicity Assay) was added, and each OD value was measured at 490 nm.

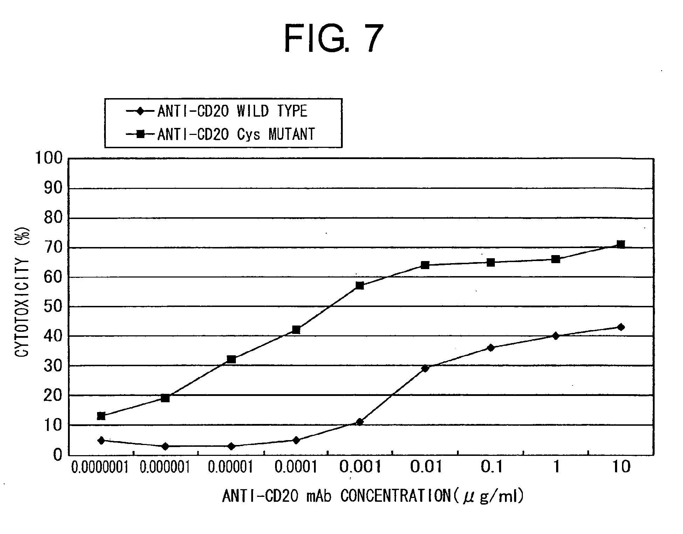

As the standards for calculating cytotoxicity rates, wells containing Daudi cells and wells containing human peripheral blood mononuclear cells were prepared in a final volume of 100 μl. 10 μl of lysis buffer (CytoTox96 Non-Radioactive Cytotoxicity Assay) was added to the half of the wells containing Daudi cells, followed by reaction for 45 minutes to lyse cells. Subsequently, in the same manner as in the experimental condition groups, 50 μl each of the supernatants was transferred to a new flat-bottom 96 well plate, and 50 μl of assay buffer (CytoTox96 Non-Radioactive Cytotoxicity Assay) was added to each well, followed by reaction at room temperature for 30 minutes with light shielding. Subsequently, 50 μl of stop buffer (CytoTox96 Non-Radioactive Cytotoxicity Assay) was added, and each OD value was measured at 490 nm. Here, a standard line for calculating cytotoxicity was prepared by setting the sum of the OD value of the wells containing Daudi cells and that of the wells containing human peripheral blood mononuclear cells as 0% cytotoxicity, and the OD value obtained by subtracting the OD value of the wells containing Daudi cells without lysis buffer from that of the wells containing Daudi cells supplemented with lysis buffer as 100% cytotoxicity. Cytotoxic activities under various conditions were calculated from this standard line. Experiments were conducted on three or more wells under each experimental condition to calculate the mean value and standard error of experimental data. The results are shown in Fig. 7. As shown in Fig. 7, the Cys mutant of anti-CD20 antibody had a high ADCC activity at low antibody concentrations, as compared with the anti-CD20 antibody, and the improvement in its maximum ADCC activity at antibody concentrations higher than 0.01 μg/ml was significant.

(2) Measurement of the ADCC activities of the anti-CD20 antibody and the Cys mutant antibody, obtained in Example 2:

Antibody-dependent cellular cytotoxicity (ADCC) of chimeric antibody was evaluated by lactate dehydrogenase release assay. In this assay, Raji cells, which are human B lymphocytes having CD20 molecule on the cell membrane surfaces, were used as target cells, and human peripheral blood mononuclear cells (PBMCs) were used as effector cells. The human PBMCs were prepared from human blood using Lymphoprep (Axis Shield).

Raji cells ($1 \times 10^4$ cells/50 μL) were put into each well of a 96-well U-bottomed plate, and $2 \times 10^5$ human PBMCs were added as effector cells to achieve the E/T ratio of 20/1. An anti-CD20 chimeric antibody was further added to this cell solution to prepare a dilution series, followed by incubation at 37°C for four to six hours. After the incubation, the 96-well U-bottomed plate was centrifuged, and the lactate dehydrogenase activities in the supernatants were measured using the CytoTox 96 Non-Radioactive Cytotoxicity Assay Kit (Promega, G1780). Antibody-dependent and antibody-specific cell damage (cytotoxicity) % was calculated using the following formula:

$$\% \text{ Cytotoxicity} = 100 \times (E - S_E - S_T) / (M - S_T)$$

Herein, E indicates "experimental release", which is the activity of lactate dehydrogenase released from the target cells when antibody and effector cells were incubated with the target cells. $S_E$ is the activity of lactate dehydrogenase released spontaneously from the effector cells. $S_T$ is the activity of lactate dehydrogenase released spontaneously from the target cells. M indicates the maximum releasable lactate dehydrogenase activity of the target cells, which is the activity of lactate dehydrogenase released from the target cells by the addition of lysis solution (9% Triton X-100).

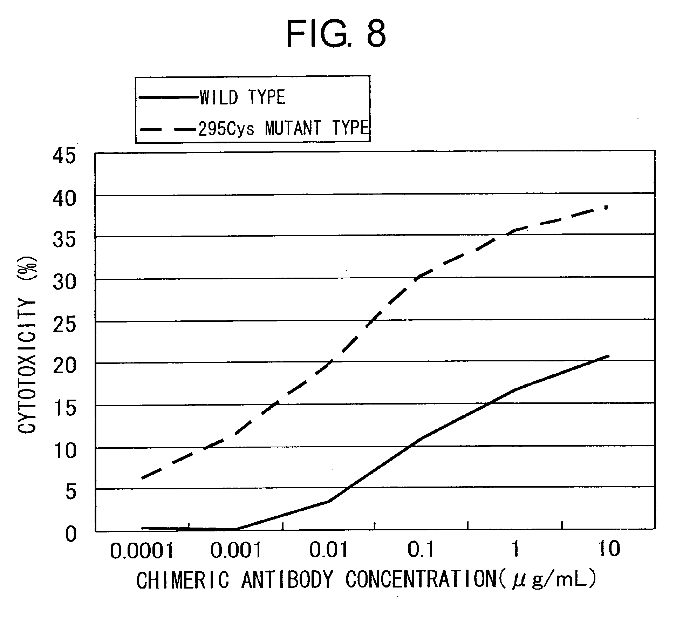

The ADCC activities of the wild-type and 295Cys-type anti-CD20 chimeric antibodies evaluated by the above evaluation method are shown in Fig. 8. Fig. 8 demonstrates that the 295Cys-type chimeric antibody has ADCC activity that is very high as compared with the ADCC activity of the wild-type chimeric antibody.

Further, it was confirmed that the 295Cys-type chimeric antibody could also exhibit ADCC activity in concentrations lower than a usual concentration (the usual concentration being 0.001 μg/mL or more) (Fig. 8).

The percentages of cytotoxicity (cell damage) with the anti-CD20 wild-type antibody and the 295Cys-type chimeric antibody were compared by lactate dehydrogenase release assay. As a result, the percentages of cytotoxicity at the concentrations of added antibody of 0.0001, 0.001, 0.01, 0.1, 1, and 10 μg/mL were 0.31%, 1.25%, 3.52%, 10.93%, 16.63%, and 20.52% for the wild-type antibody, respectively, and were 6.38%, 11.52%, 19.66%, 30.25%, 35.69%, and 38.24% for the 295Cys-type chimeric antibody, respectively. Thus, it was demonstrated that the ratios of the ADCC activities (a ratio of ADCC activity refers to the ratio of the percentage of cytotoxicity of the mutant antibody to that of the wild-type antibody at two points where the concentrations of wild-type and mutant-type antibodies are the same) were 20.6 fold, 9.2 fold, 5.6 fold, 2.8 fold, 2.1 fold, and 1.9 fold, at each points (Table 8).

[Table 8]

| | Concentration (μg/ml) | | | | | |
|---|---|---|---|---|---|---|
| Cytotoxicity (%) | 0.0001 | 0.001 | 0.01 | 0.1 | 1 | 10 |
| Wild-type anti-CD20 antibody | 0.31 | 1.25 | 3.52 | 10.93 | 16.63 | 20.52 |
| 295Cys-type anti-CD20 antibody | 6.38 | 11.52 | 19.66 | 30.25 | 35.69 | 38.24 |
| 295Cys type / Wild type (fold) | 20.6 | 9.2 | 5.6 | 2.8 | 2.1 | 1.9 |

Further, from Table 8, two points with comparable percentages of cytotoxicity between the wild-type antibody and the 295Cys-type chimeric antibody were selected to compare the ADCC activities. For example, the 295Cys-type chimeric antibody showed approximately 20% of cytotoxicity at 0.01 μg/mL, and the wild-type antibody showed approximately 20% of cytotoxicity at 1 μg/mL. When compared using these two points, the antibody concentration required to achieve the same ADCC activity decreased to 1/1000; therefore, it can be judged that the ADCC activity increased approximately 1000 fold (the ratio of the concentration of the wild-type antibody to that of the mutant-type antibody at two points where the percentages of cytotoxicity of wild-type and mutant-type antibodies were the same was 1000 fold).

Such increase in ADCC activity (approximately 100 to 1000 fold) was also confirmed in other concentration ranges. Those skilled in the art can calculate the increase of ADCC activity from Fig. 8 and Table 8 according to the above method.

(3) Measurement of the ADCC activities of the anti-EGFR antibody and the Cys mutant thereof, obtained in Example 3:

Antibody-dependent cellular cytotoxicity (ADCC) activity of human anti-EGFR antibodies was evaluated by lactate dehydrogenase release assay. In this assay, NCI-H226 cells having EGFR molecules on the cell membrane surfaces were used as target cells, and human peripheral blood mononuclear cells (PBMCs), effector cells. The human PBMCs were prepared from human blood using the Lymphoprep (Axis Shield).

NCI-H226 cells (1 x $10^4$ cells/50 μL) were put into each well of a 96-well U-bottomed plate, and 2 x $10^5$ human PBMCs, the effector cells, were added to achieve the E/T ratio of 20/1. Wild-type or 295Cys-type human anti-EGFR chimeric antibody was further added to this cell solution to prepare a dilution series, followed by incubation at 37°C for 16 hours. After the incubation, the 96-well U-bottomed plate was centrifuged, and the lactate dehydrogenase activities in the supernatants were measured using CytoTox 96 Non-Radioactive Cytotoxicity Assay Kit (Promega, G 1780). Antibody-dependent and antibody-specific cell damage (cytotoxicity) % was calculated using the following formula:

$$\% \text{ Cytotoxicity} = 100 \text{ x } (E - S_E - S_T>) / (M - S_T)$$

Herein, E indicates "experimental release", which is the activity of lactate dehydrogenase released from the target cells when antibody and effector cells were incubated with the target cells. $S_E$ is the activity of lactate dehydrogenase

released spontaneously from the effector cells. $S_T$ is the activity of lactate dehydrogenase released spontaneously from the target cells. M indicates the maximum releasable lactate dehydrogenase activity of the target cells, which is the activity of lactate dehydrogenase released from the target cells by the addition of lysis solution (9% Triton X-100).

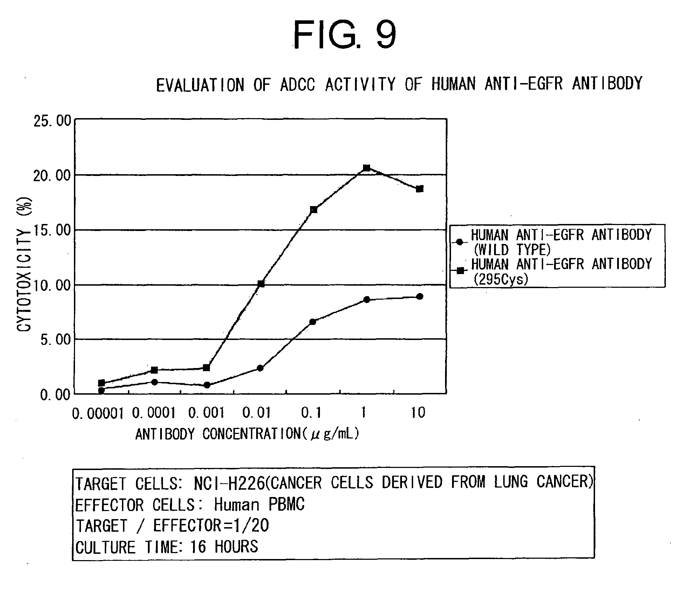

The ADCC activities of the wild-type and 295Cys-type human anti-EGFR antibodies evaluated by the above evaluation method are shown in Fig. 9. The results shown in Fig. 9 demonstrate that the 295Cys-type human anti-EGFR antibody has 1000-fold or more higher ADCC activity as compared with the wild-type one, as in the case of anti-CD20 chimeric antibodies.

Further, it was confirmed that the 295Cys-type anti-EGFR antibody could also exhibit ADCC activity in concentrations lower than a usual concentration (the concentration of 0.00001-0.0001 μg/mL or more) (Fig. 9).

The percentages of cytotoxicity (cell damage) with the wild-type anti-EGFR antibody and the 295Cys-type chimeric antibody were compared by lactate dehydrogenase release assay. As a result, the percentages of cytotoxicity at the concentrations of added antibody of 0.00001, 0.0001, 0.001, 0.01, 0.1, 1, and 10 μg/mL were 0.5%, 1.1%, 0.8%, 2.4%, 6.7%, 8.7%, and 8.9% for the wild-type antibody, respectively, and were 1.0%, 2.2%, 2.4%, 10.1%, 16.8%, 20.6%, and 18.6% for the 295Cys-type chimeric antibody, respectively. Thus, it was demonstrated that the ratios of the ADCC activities (a ratio of ADCC activity refers to the ratio of the percentage of cytotoxicity of the mutant antibody to that of the wild-type antibody at two points where the concentrations of wild-type and mutant-type antibodies are the same) were 1.9 fold, 2.0 fold, 2.9 fold, 4.3 fold, 2.5 fold, 2.4 fold, and 2.1 fold, at each points (Table 9).

[Table 9]

| | Concentration (μg/ml) | | | | | | |
|---|---|---|---|---|---|---|---|
| Cytotoxicity (%) | 0.00001 | 0.0001 | 0.001 | 0.01 | 0.1 | 1 | 10 |
| Wild-type anti-EGFR antibody | 0.5 | 1.1 | 0.8 | 2.4 | 6.7 | 8.7 | 8.9 |
| 295Cys-type anti-EGFR antibody | 1.0 | 2.2 | 2.4 | 10.1 | 16.8 | 20.6 | 18.6 |
| 295Cys type / Wild type (fold) | 1.9 | 2.0 | 2.9 | 4.3 | 2.5 | 2.4 | 2.1 |

Further, from Table 9, two points with comparable percentages of cytotoxicity between the wild-type antibody and the 295Cys-type chimeric antibody were selected to compare the ADCC activities. For example, the 295Cys-type chimeric antibody showed approximately 10% of cytotoxicity at 0.01 μg/mL, and the wild-type antibody showed approximately 9% of cytotoxicity at 10 μg/mL.

When compared using these two points, the antibody concentration required to achieve the same ADCC activity decreased to 1/1000; therefore, it can be judged that the ADCC activity increased approximately 1000 fold or more (the ratio of the concentrations of the wild-type antibody to that of the mutant-type antibody at two points where the percentages of cytotoxicity of wild-type and mutant-type antibodies were the same was 1000 fold or more).

Such increase in ADCC activity (approximately 10 to 100 fold) was also confirmed in other concentration ranges. Those skilled in the art can calculate the increase of ADCC activity from Fig. 9 and Table 9 by the above method.

(4) Measurement of the ADCC activities of the AILIM/ICOS-IgFc chimeric antibody and the Cys mutant thereof, obtained in Example 4:

In the measurement of ADCC activities by AILIM/ICOS-IgFc chimeric molecules, B7h-expressing Ba/F3 cells were used as target cells, and human peripheral blood mononuclear cells, effector cells. B7h-expressing Ba/F3 cells were suspended in 10% FCS RPMI-1640 medium (10% WEHI-3 culture supernatant (IL-3)) at a concentration of 4 x $10^5$ cells/ml by conventional procedures, and 25 μl each was plated onto a U-bottom 96 well plate (Falcon). AILIM/ICOS-IgFc chimeric antibodies were diluted with 10% FCS RPMI-1640 medium (supplemented with 10% WEHI-3 culture supernatant (IL-3)) at various concentrations, and 25 μl each was plated onto a U-bottom 96 well plate (Falcon), followed by reaction at 37°C for one hour. Subsequently, peripheral blood mononuclear cells were added at 5 x $10^5$ cells/well and cultured at 37 degrees for 16 hours. After the culture, the culture plate was centrifuged, and 50 μl of the supernatant was collected from each well and transferred into a new flat bottom 96 well plate. 50 μl of assay buffer (CytoTox96 Non-Radioactive Cytotoxicity Assay, Promega) was added to each well, followed by reaction at room temperature for 30 minutes with light shielding. Subsequently, 50 μl of stop buffer (CytoTox96 Non-Radioactive Cytotoxicity Assay) was added, and each OD value was measured at 490 nm.

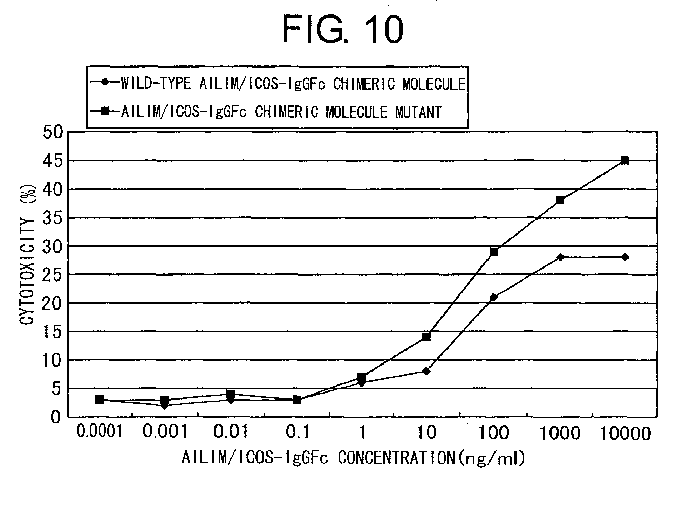

As the standards for calculating cytotoxicity rates, wells containing B7h-expressing Ba/F3 cells and wells containing human peripheral blood mononuclear cells were prepared in a final volume of 100 μl. 10 μl of lysis buffer (CytoTox96 Non-Radioactive Cytotoxicity Assay) was added to the half of the wells containing B7h-expressing Ba/F3 cells, followed by reaction for 45 minutes to lyse cells. Subsequently, in the same manner as in the experimental condition

groups, 50 μl each of the supernatants was transferred to a new flat-bottom 96 well plate, and 50 μl of assay buffer (CytoTox96 Non-Radioactive Cytotoxicity Assay) was added to each well, followed by reaction at room temperature for 30 minutes with light shielding. Subsequently, 50 μl of stop buffer (CytoTox96 Non-Radioactive Cytotoxicity Assay) was added, and each OD value was measured at 490 nm. Here, a standard line for calculating cytotoxicity was prepared by setting the sum of the OD value of the wells containing B7h-expressing Ba/F3 cells and that of the wells containing human peripheral blood mononuclear cells as 0% cytotoxicity, and the OD value obtained by subtracting the OD value of the wells containing B7h-expressing Ba/F3 cells without lysis buffer from that of the wells containing B7h-expressing Ba/F3 cells supplemented with lysis buffer as 100% cytotoxicity. Cytotoxic activities under various conditions were calculated from this standard line. Experiments were conducted on three or more wells under each experimental condition to calculate the mean value and standard error of experimental data. The results are shown in Fig. 10. As shown in Fig. 10, the Cys mutant of AILIM/ICOS-IgFc chimeric molecule had a high ADCC activity at low antibody concentrations, as compared with the wild-type AILIM/ICOS-IgFc chimeric molecule. In addition, the improvement in its maximum ADCC activity at antibody concentrations higher than 1 μg/ml was significant.

[Example 5] Reaction analysis with CD16 using the anti-CD20 antibody Cys295 mutant:

CHO-K1 highly expressing CD16 was suspended at 1 x $10^5$ cells/ml (cell suspending solution: 3 mM EDTA; 0.5% BSA; PBS (-)), and this was dispensed into polystyrene round tubes in 50 μl aliquots (50000 cells). Wild-type and Cys295 mutant anti-CD20 antibodies were serially diluted from 20 μg/ml in two-fold-steps at eight dilutions (antibody dilution solution: 3 mM EDTA; 0.5% BSA; PBS (-)), and 50 μl of this was added per tube for reaction at 37°C for one hour.

After washing with the solution of 3 mM EDTA and 0.5% BSA in PBS (-), 500-fold diluted biotin-labeled anti-Ig κ chain (Pharmingen, Cat.#34172D) was added in a 100 μl aliquot per tube for reaction at 37°C for 1.5 hour. After washing with the solution of 3 mM EDTA and 0.5% BSA in PBS (-), 400-fold diluted Streptavidin-PE (Pharmingen, Cat. #554061 (Was:13025D)) was added in a 100 μl aliquot per tube for reaction at 37°C for 30 minutes. After washing with the solution of 3mM EDTA and 0.5%BSA in PBS (-) following the reaction, cells were scanned by the FACSCALibur and analyzed using CellQuest (BD).

Results:

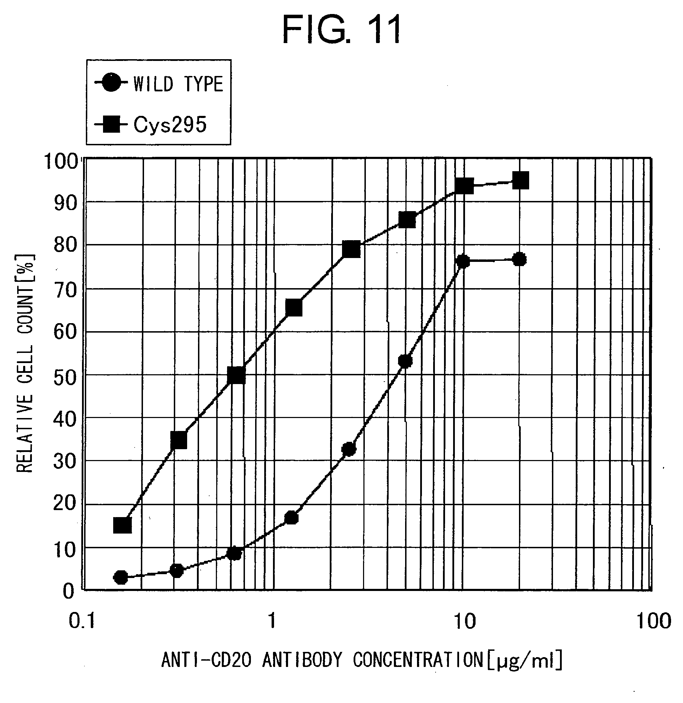

Results of the reaction analysis of the anti-CD20 antibody Cys295 mutant with CD16 are shown in Fig. 11. It was elucidated that the anti-CD20 antibody Cys295 mutant had about 8-fold higher affinity for CD 16 as compared with wild-type anti-CD20 antibody. Furthermore, the wild-type anti-CD20 antibody showed approximately 80% binding at a maximum, while the anti-CD20 antibody Cys295 mutant showed approximately 95% binding. Therefore, the anti-CD20 antibody Cys295 mutant is suggested to have increased antibody function in ADCC.

The results of BiaCore measurement demonstrated that the binding strength between the receptor and the chimeric antibody was about 10-fold increased as compared with that between the receptor and the wild-type antibody.

[Example 6] Analysis on binding reactivity of AILIM-IgFc chimeric molecule Gln223Cys with CD 16:

*1. Objective:*

It is known that N-binding type sugar chain is added to the 225th amino acid in the IgFc domain of AILIM-IgFc chimeric molecule. Analysis was conducted on the ADCC activity when glutamine (Gln, Q) at the neighboring 223rd position (the 295th by the EUIndex by Kabat) was replaced by cysteine (Cys, C) (AILIM-IgFc Gln223Cys).

*2. Materials:*

1) Antibodies:

AILIM-IgFc Wt 1.42 mg/ml
AILIM-IgFc Cys223 1.11 mg/ml
HRP-labeled mouse anti-human IgG antibody: ZYMED, Lot#; 40789491

2) Cells:

CD16a-expressing CHO-K1

3) Other reagents:

Phosphate Buffered Saline (PBS (-))
Bovine Serum Albumin (BSA): Thermo, Cat#; 303050-500GM, Lot.#; 513
2NA (EDTA-2Na): DOJINDO, Cat.#; 345-01865

4) Instruments and equipments:

FACSCalibur (Becton Dickinson)
FACS tubes (FALCON. Cat#; 352008)
Low-speed centrifuge (HITACHI)

*3. Methods:*

*1. Primary antibody reaction:*

CD16a-expressing CHO-K1 cells were detached using 3 mM EDTA-BSA-PBS(-) (EBP), and the cells were counted. The cell concentration was 7.2 x $10^5$ cells/ml. Required amount of the cell suspension was put into a 15 ml tube and centrifuged at 1,800 rpm for three minutes. The cells were placed in EBP, and dispensed at 5 x $10^4$ cells/25 μl/tube. Wild-type and Cys223 AILIM-IgFc were adjusted at 20 μl/ml, and a total eight serial dilutions in two-fold steps were prepared. Each dilution was poured into the tubes, and allowed to react at 4°C for one hour.

*2. Secondary antibody reaction:*

After one hour, EBP was dispensed at 4 ml/tube; centrifugation was carried out at 1,800 rpm for three minutes; and the supernatant was removed (washing). After the removal of the supernatant, the residual amount of the solution was confirmed to be approximately 60 μl, and biotin-labeled anti-AILIM antibody, which was 500-fold diluted with EBP, was added at 1 μl/tube and then allowed to react at 4°C for one hour.

*3. Tertiary antibody reaction:*

After the reaction, EBP was dispensed at 4 ml/tube; centrifugation was carried out at 1,800 rpm for three minutes; and the supernatant was removed (washing). After the removal of the supernatant, the residual amount of the solution was confirmed to be approximately 60 μl, and PE-labeled streptavidin, which was 400-fold diluted with EBP, was added at 100 μl/tube and then allowed to react at 4°C for 30 minutes.

*4. FACS Analysis:*

After the reaction, EBP was dispensed at 4 ml/tube; centrifugation was carried out at 1,800 rpm for three minutes; and the supernatant was removed (washing). Then analysis was conducted by FACSCalibur.

*4. Results:*

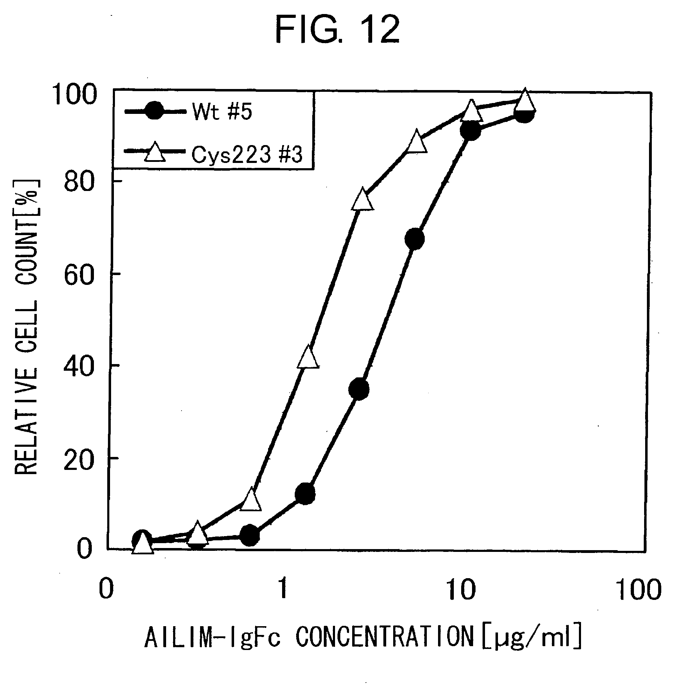

Results of the analysis on the binding reaction of AILIM-IgFc chimeric molecule Gln223Cys with CD16 are shown in Fig. 12. As a result of comparing the proportions of cells bound with PE-labeled antibody between Wt and Cys223 AILIM-IgFc, the proportion begun to increase at the concentration of 1.25 μg/ml for the Wt, while it begun to increase at 0.625 μg/ml for Cys223. The graph demonstrated that Cys223 had about 2-fold increased reactivity with CD16a, as compared with Wt.

The results of BiaCore measurement demonstrated that the binding strength between the receptor and the chimeric antibody was about 10-fold increased, as compared with that between the receptor and the wild-type chimeric molecule.

*5. Discussion:*

These results suggest that the replacement of the 223rd Gln of AILIM-IgFc by Cys increases the binding affinity for CD16a. This increase in binding affinity is considered to have contributed to the increased ADCC activity by AILIM-IgFc Gln223Cys described in Example 4.

Industrial applicability

With the polypeptide mutants of the present invention, effector function can markedly be increased by mutating a specific amino acid residue in the Fc region of an antibody. Therapeutic compositions comprising such polypeptide mutants are very useful as antibody drugs, since they can provide excellent pharmacologic effects even in a small amount. Further, a decrease in their usage can reduce the cost; in addition, the compositions can greatly reduce patients' economical and physical burden due to their high specificity and few side effects. Other treatment methods that have been used concomitantly with treatment using an antibody, such as chemotherapies and radioactive isotope-labeled antibodies, can be avoided, to thereby permit the avoidance of adverse effects caused by these treatment methods.

## Claims

1. A mutant polypeptide that comprises an Fc region in which a cysteine residue is substituted for an amino acid that is second from the glycosylation site on the N terminal side in the Fc region of an IgG.

2. A mutant polypeptide that comprises an Fc region in which a cysteine residue is substituted for an amino acid at EU index 295 according to the Kabat numbering system.

3. The mutant polypeptide of claim 1 or 2, whose effector function is improved as compared to that before substitution.

4. The mutant polypeptide of any one of claims 1 to 3, wherein the Fc region is a human IgG Fc region.

5. The mutant polypeptide of any one of claims 1 to 4, which has an N-linked sugar chain at the glycosylation site of the Fc region.

6. The mutant polypeptide of any one of claims 1 to 5, which comprises a site that recognizes a human cell surface molecule.

7. The mutant polypeptide of any one of claims 1 to 6, which is a mutant antibody comprising a substitution of a cysteine residue for an amino acid at EU index 295 according to the Kabat numbering system.

8. The mutant polypeptide of claim 7, which recognizes at least any one selected from the group consisting of cytokine receptors, cell adhesion molecules, cancer cell surface molecules, cancer stem cell surface molecules, blood cell surface molecules, and surface molecules of virus-infected cells.

9. The mutant polypeptide of claim 8, which recognizes at least any one selected from the group consisting of antigens CD3, CD11a, CD20, CD22, CD25, CD28, CD33, and CD52, Her2/neu, EGF receptor, EpCAM, MUC1, GD3, CEA, CA125, HLA-DR, TNFalpha receptor, VEGF receptor, CTLA-4, AILIM/ICOS, and integrin molecules.

10. The mutant polypeptide of any one of claims 1 to 6, which is a mutant chimeric molecule comprising a cell surface molecule recognition site and an Fc region that comprises a substitution of a cysteine residue for an amino acid at EU index 295 according to the Kabat numbering system.

11. The mutant polypeptide of claim 10, which binds to at least one human cell surface molecule selected from the group consisting of cytokine receptors, cell adhesion molecules, cancer cell surface molecules, cancer stem cell surface molecules, blood cell surface molecules, and surface molecules of virus-infected cells.

12. The mutant polypeptide of claim 11, which binds to at least one human cell surface molecule selected from the group consisting of CD3, CD11a, CD20, CD22, CD25, CD28, CD33, CD52, Her2/neu, EGF receptor, EpCAM, MUC1, GD3, CEA, CA125, HLA-DR, TNFalpha receptor, VEGF receptor, AILIM/ICOS, CTLA-4, B7h, CD80, CD86, and integrin molecules.

**13.** An isolated nucleic acid encoding the mutant polypeptide of any one of claims 1 to 12.

**14.** A vector carrying the nucleic acid of claim 13.

**15.** A host cell or host organism harboring the vector of claim 14.

**16.** A method for producing a mutant polypeptide, which comprises the step of culturing the host cell or host organism of claim 15 so that the host expresses the mutant polypeptide encoded by a nucleic acid.

**17.** A therapeutic composition comprising the mutant polypeptide of any one of claims 1 to 12.

**18.** A method for producing a mutant antibody having improved effector function, which comprises the steps of:

(1) substituting a cysteine residue for an amino acid residue at EU index 295 according to the Kabat numbering system in an antibody having effector function;
(2) introducing a host cell or host organism with a DNA encoding an L chain and a DNA encoding an H chain having an Fc region comprising the substitution of a cysteine residue for an amino acid residue at EU index 295 according to the Kabat numbering system, and expressing the DNA; and
(3) collecting the expression product.

**19.** A method for producing a mutant chimeric molecule having improved effector function, which comprises the steps of:

(1) substituting a cysteine residue for an amino acid residue at EU index 295 according to the Kabat numbering system in the Fc region of a chimeric molecule with the effector function, which comprises a cell surface molecule recognition site in addition to the Fc region;
(2) introducing a host cell or host organism with a DNA encoding the mutant chimeric molecule comprising a cell surface molecule recognition site and the Fc region comprising a substitution of a cysteine residue for an amino acid residue at EU index 295 according to the Kabat numbering system, and expressing the DNA; and
(3) collecting the expression product.

**20.** A method for producing a mutant chimeric molecule having improved effector function, which comprises the steps of:

(1) producing a mutant chimeric molecule comprising a cell surface molecule recognition site and an Fc region;
(2) judging whether the produced chimeric molecule has the effector function;
(3) substituting a cysteine residue for an amino acid residue at EU index 295 according to the Kabat numbering system in the Fc region of the chimeric molecule that has been judged to have the effector function;
(4) introducing and expressing in a host cell or host organism a DNA encoding the mutant chimeric molecule that comprises the cell surface molecule recognition site and the Fc region comprising a substitution of a cysteine residue for an amino acid residue at EU index 295 according to the Kabat numbering system; and
(5) collecting the expression product.

**21.** A method for improving antibody effector function, which comprises the step of substituting a cysteine residue for an amino acid residue at EU index 295 according to the Kabat numbering system in an antibody having effector function.

**22.** A method for improving the effector function of a chimeric molecule, which comprises the step of substituting a cysteine residue for an amino acid residue at EU index 295 according to the Kabat numbering system in the Fc region of the chimeric molecule with the effector function, which comprises a cell surface molecule recognition site in addition to the Fc region.

**23.** A method for improving the effector function of a chimeric molecule, which comprises the steps of:

(1) producing a chimeric molecule that comprises a cell surface molecule recognition site and an Fc region;
(2) judging whether the produced chimeric molecule has the effector function; and
(3) substituting a cysteine residue for an amino acid residue at EU index 295 according to the Kabat numbering system in the Fc region of the chimeric molecule that has been judged to have the effector function.

FIG. 1

ANTIGEN-BINDING SITE
VL
VH
VARIABLE REGION
CH1
CL
Fab
CONSTANT REGION
SUGAR
CH2
Fc
CH3

FIG. 2

M
1
2
kDa
75
50
37
25
←H CHAIN OF CHIMERIC ANTIBODY
←L CHAIN OF CHIMERIC ANTIBODY

LANE M: MARKER PROTEINS
LANE 1: PURIFIED 295Cys ANTI-CD20 CHIMERIC ANTIBODY
LANE 2: PURIFIED WILD-TYPE ANTI-CD20 CHIMERIC ANTIBODY

# FIG. 3

WILD TYPE
295Cys
140
0
COUNT
M1
M2
$10^0$
$10^1$
$10^2$
$10^3$
$10^4$
FL1-H

# FIG. 4

MARKER
WILD-TYPE
295Cys
kDa
75
50
37
25
H CHAIN
L CHAIN

15% SDS-PAGE (SILVER STAINING)

# FIG. 5

WILD TYPE
COUNT
0
20
40
60
80
100
$10^0$
$10^1$
$10^2$
$10^3$
$10^4$
FL1-Height
295Cys TYPE
COUNT
0
20
40
60
80
100
$10^0$
$10^1$
$10^2$
$10^3$
$10^4$
FL1-Height

# FIG. 6-1

```
                  10         20         30         40         50         60
SEQ ID NO: 94 ATGAAGTCAGGCCTCTGGTATTTCTTTCTCTTCTGCTTGCGCATTAAAGTTTTAACAGGA
SEQ ID NO: 99 M  K  S  G  L  W  Y  F  F  L  F  C  L  R  I  K  V  L  T  G
                  70         80         90        100        110        120
              GAAATCAATGGTTCTGCCAATTATGAGATGTTTATATTTCACAACGGAGGTGTACAAATT
              E  I  N  G  S  A  N  Y  E  M  F  I  F  H  N  G  G  V  Q  I
                 130        140        150        160        170        180
              TTATGCAAATATCCTGACATTGTCCAGCAATTTAAAATGCAGTTGCTGAAAGGGGGGCAA
              L  C  K  Y  P  D  I  V  Q  Q  F  K  M  Q  L  L  K  G  G  Q
                 190        200        210        220        230        240
              ATACTCTGCGATCTCACTAAGACAAAAGGAAGTGGAAACACAGTGTCCATTAAGAGTCTG
              I  L  C  D  L  T  K  T  K  G  S  G  N  T  V  S  I  K  S  L
                 250        260        270        280        290        300
              AAATTCTGCCATTCTCAGTTATCCAACAACAGTGTCTCTTTTTTTCTATACAACTTGGAC
              K  F  C  H  S  Q  L  S  N  N  S  V  S  F  F  L  Y  N  L  D
                 310        320        330        340        350        360
              CATTCTCATGCCAACTATTACTTCTGCAACCTATCAATTTTTGATCCTCCTCCTTTTAAA
              H  S  H  A  N  Y  Y  F  C  N  L  S  I  F  D  P  P  P  F  K
                 370        380        390        400        410        420
              GTAACTCTTACAGGAGGATATTTGCATATTTATGAATCACAACTTTGTTGCCAGCTGAAG
              V  T  L  T  G  G  Y  L  H  I  Y  E  S  Q  L  C  C  Q  L  K
                 430        440        450        460        470        480
              GATCCCGAGGAGCCCAAATCTTGTGACAAAACTCACACATGCCCACCGTGCCCAGCACCT
              D  P  E  E  P  K  S  C  D  K  T  H  T  C  P  P  C  P  A  P
                 490        500        510        520        530        540
              GAACTCCTGGGGGGACCGTCAGTCTTCCTCTTCCCCCCAAAACCCAAGGACACCCTCATG
              E  L  L  G  G  P  S  V  F  L  F  P  P  K  P  K  D  T  L  M
                 550        560        570        580        590        600
              ATCTCCCGGACCCCTGAGGTCACATGCGTGGTGGTGGACGTGAGCCACGAAGACCCTGAG
              I  S  R  T  P  E  V  T  C  V  V  V  D  V  S  H  E  D  P  E
                 610        620        630        640        650        660
              GTCAAGTTCAACTGGTACGTGGACGGCGTGGAGGTGCATAATGCCAAGACAAAGCCGCGG
              V  K  F  N  W  Y  V  D  G  V  E  V  H  N  A  K  T  K  P  R
```

# FIG. 6-2

```
      670       680       690       700       710       720
GAGGAGTGCTACAACAGCACGTACCGGGTGGTCAGCGTCCTCACCGTCCTGCACCAGGAC
E  E  C  Y  N  S  T  Y  R  V  V  S  V  L  T  V  L  H  Q  D

      730       740       750       760       770       780
TGGCTGAATGGCAAGGAGTACAAGTGCAAGGTCTCCAACAAAGCCCTCCCAGCCCCCATC
W  L  N  G  K  E  Y  K  C  K  V  S  N  K  A  L  P  A  P  I

      790       800       810       820       830       840
GAGAAAACCATCTCCAAAGCCAAAGGGCAGCCCCGAGAACCACAGGTGTACACCCTGCCC
E  K  T  I  S  K  A  K  G  Q  P  R  E  P  Q  V  Y  T  L  P

      850       860       870       880       890       900
CCATCCCGGGATGAGCTGACCAAGAACCAGGTCAGCCTGACCTGCCTGGTCAAAGGCTTC
P  S  R  D  E  L  T  K  N  Q  V  S  L  T  C  L  V  K  G  F

      910       920       930       940       950       960
TATCCCAGCGACATCGCCGTGGAGTGGGAGAGCAATGGGCAGCCGGAGAACAACTACAAG
Y  P  S  D  I  A  V  E  W  E  S  N  G  Q  P  E  N  N  Y  K

      970       980       990      1000      1010      1020
ACCACGCCTCCCGTGCTGGACTCCGACGGCTCCTTCTTCCTCTACAGCAAGCTCACCGTG
T  T  P  P  V  L  D  S  D  G  S  F  F  L  Y  S  K  L  T  V

     1030      1040      1050      1060      1070      1080
GACAAGAGCAGGTGGCAGCAGGGGAACGTCTTCTCATGCTCCGTGATGCATGAGGCTCTG
D  K  S  R  W  Q  Q  G  N  V  F  S  C  S  V  M  H  E  A  L

     1090      1100      1110      1120     1128
CACAACCACTACACGCAGAAGAGCCTCTCCCTGTCTCCGGGTAAATGA SEQ ID NO: 94
H  N  H  Y  T  Q  K  S  L  S  L  S  P  G  K  *    SEQ ID NO: 99
```

# FIG. 7

ANTI-CD20 WILD TYPE
ANTI-CD20 Cys MUTANT
CYTOTOXICITY (%)
100
90
80
70
60
50
40
30
20
10
0
0.0000001
0.000001
0.00001
0.0001
0.001
0.01
0.1
1
10
ANTI-CD20 mAb CONCENTRATION(μg/ml)

WILD TYPE
295Cys MUTANT TYPE
45
40
35
30
25
20
15
10
5
0
CYTOTOXICITY (%)
0.0001
0.001
0.01
0.1
1
10
CHIMERIC ANTIBODY CONCENTRATION(μg/mL)

# FIG. 9

EVALUATION OF ADCC ACTIVITY OF HUMAN ANTI-EGFR ANTIBODY

25.00
20.00
15.00
10.00
5.00
0.00
CYTOTOXICITY (%)
0.00001
0.0001
0.001
0.01
0.1
1
10
ANTIBODY CONCENTRATION(μg/mL)
HUMAN ANTI-EGFR ANTIBODY (WILD TYPE)
HUMAN ANTI-EGFR ANTIBODY (295Cys)

TARGET CELLS: NCI-H226(CANCER CELLS DERIVED FROM LUNG CANCER)
EFFECTOR CELLS: Human PBMC
TARGET / EFFECTOR=1/20
CULTURE TIME: 16 HOURS

# FIG. 10

WILD-TYPE AILIM/ICOS-IgGFc CHIMERIC MOLECULE
AILIM/ICOS-IgGFc CHIMERIC MOLECULE MUTANT
CYTOTOXICITY (%)
50
45
40
35
30
25
20
15
10
5
0
0.0001
0.001
0.01
0.1
1
10
100
1000
10000
AILIM/ICOS-IgGFc CONCENTRATION(ng/ml)

FIG. 11

WILD TYPE
Cys295
100
90
80
70
60
50
40
30
20
10
0
RELATIVE CELL COUNT[%]
0.1
1
10
100
ANTI-CD20 ANTIBODY CONCENTRATION[μg/ml]

FIG. 12

Wt #5
Cys223 #3
100
80
60
40
20
0
RELATIVE CELL COUNT[%]
0
1
10
100
AILIM-IgFc CONCENTRATION[μg/ml]

**INTERNATIONAL SEARCH REPORT**

International application No.
PCT/JP2007/058103

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C12N15/09, A01K67/027, A61K39/395, A61P1/00, A61P19/02, A61P29/00, A61P35/00, A61P35/02, A61P35/04, A61P37/04, A61P37/06, C07K16/28, C07K16/46, C12N1/15, C12N1/19, C12N1/21, C12N5/10, C12P21/02, C12P21/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS/MEDLINE/WPIDS(STN), CAplus(STN), JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| P,X | WO 2006/105062 A2 (DIVERSA CORP.), 05 October, 2006 (05.10.06), (Family: none) | 1-23 |
| P,X | WO 2006/104989 A2 (DIVERSA CORP.), 05 October, 2006 (05.10.06), (Family: none) | 1-23 |
| P,X | US 2006/134709 A1 (Stavenhagen, Jeffery), 22 June, 2006 (22.06.06), & WO 2007/024249 A2 | 1-23 |
| X | WO 2006/019447 A1 (XENCOR, INC.), 23 February, 2006 (23.02.06), & AU 2005272993 A1 & CA 2565961 A1 & IN 2005 MN01288 A | 1-23 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>01 June, 2007 (01.06.07) | Date of mailing of the international search report<br>12 June, 2007 (12.06.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2007/058103

C (Continuation). DOCUMENTS CONSIDERED TO BE RELEVANT

| Category* | Citation of document, with indication, where appropriate, of the relevant passages | Relevant to claim No. |
|---|---|---|
| X | WO 2004/063351 A2 (Macrogenics, Inc.), 29 July, 2004 (29.07.04), & AU 2004204494 A1 & CA 2512729 A1 & EP 1587540 A2 & JP 2006-524039 T & IN 2005 CN01849 A | 1-23 |
| X | WO 2004/074455 A2 (APPLIED MOLECULAR EVOLUTION), 02 September, 2004 (02.09.04), & US 2004/002587 A1 & EP 1606314 A2 | 1-23 |
| X | WO 2005/070963 A1 (APPLIED MOLECULAR EVOLUTION, INC), 04 August, 2005 (04.08.05), & AU 2005206473 A1 & CA 2552788 A1 & EP 1706424 A1 & CN 1918178 A & NO 2006-003624 A | 1-23 |
| A | Nettleton MY, et al., Role of glycosylation sites in the IgE Fc molecule., Int Arch Allergy Immunol., 1995 May-Jun, 107(1-3): 328-9. | 1-23 |
| A | Chamow SM, et al., Immunoadhesins: principles and applications., Trends Biotechnol. 1996 Feb; 14(2):52-60. Review. | 1-23 |

Form PCT/ISA/210 (continuation of second sheet) (April 2005)

**INTERNATIONAL SEARCH REPORT**

International application No.
PCT/JP2007/058103

Continuation of A. CLASSIFICATION OF SUBJECT MATTER
(International Patent Classification (IPC))

*C12N15/09*(2006.01)i, *A01K67/027*(2006.01)i, *A61K39/395*(2006.01)i, *A61P1/00*(2006.01)i, *A61P19/02*(2006.01)i, *A61P29/00*(2006.01)i, *A61P35/00*(2006.01)i, *A61P35/02*(2006.01)i, *A61P35/04*(2006.01)i, *A61P37/04*(2006.01)i, *A61P37/06*(2006.01)i, *C07K16/28*(2006.01)i, *C07K16/46*(2006.01)i, *C12N1/15*(2006.01)i, *C12N1/19*(2006.01)i, *C12N1/21*(2006.01)i, *C12N5/10*(2006.01)i, *C12P21/02*(2006.01)i, *C12P21/08*(2006.01)i

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005224240 A
- WO 0042072 A
- US 20050054832 A1
- US 20050054832 A
- US 5091178 A
- JP H0322979 A
- JP H02227075 A
- JP S63299 A
- JP 2606856 B
- JP 2517813 B
- JP S59140885 A
- JP S6070080 A
- WO 9400977 A
- JP S60251887 A
- JP H05336963 A
- JP H06823021 A
- JP S63309192 A
- JP H20504970 A
- WO 9515393 A
- WO 9201047 A
- WO 9220791 A
- WO 9306213 A
- WO 9311236 A
- WO 9319172 A
- WO 9501438 A
- WO 9515388 A
- WO 9846777 A
- WO 9634096 A
- JP H0159878 B
- WO 9312227 A
- WO 9203918 A
- WO 9402602 A
- WO 9633735 A
- JP H02257891 A
- WO 9411026 A


### Non-patent literature cited in the description


- *Eur. J. Immunol.,* 1993, vol. 23, 1098
- *Immunology,* 1995, vol. 86, 319
- *Chemical Immunology,* 1997, vol. 65, 88
- *The Third Symposium of Japan Consortium for Glycobiology and Glycotechnology,* 2005, 30
- Sequence of Proteins of Immunological Interest. National Institutes of Health, 1991
- **HOPP, T. P. et al.** *BioTechnology,* 1988, vol. 6, 1204-1210
- Molecular Cloning 2nd Ed.; Current Protocols in Molecular Biology; Antibodies, A Laboratory manual. Cold Spring Harbor Laboratory, 1988
- Monoclonal Antibodies: principles and practice. Acad. Press, 1993
- Antibody Engineering, A Practical Approach. IRL Press at Oxford University Press, 1996
- *Methods. Enzymol.,* 1990, vol. 194, 182
- *Proc. Natl. Acad. Sci. U.S.A,* 1978, vol. 84, 1929
- *J. Bacteriology,* 1983, vol. 153, 163
- *Proc. Natl. Acad. Sci. U.S.A,* 1978, vol. 75, 1929
- *Cytotechnology,* 1990, vol. 3, 133
- *Nature,* 1987, vol. 329, 840
- *J. Biochemistry,* 1987, vol. 101, 1307
- *Proc. Natl. Acad. Sci. U.S.A.,* 1987, vol. 84, 7413
- *The Journal of the American Medical Association,* 1967, vol. 199, 519
- *Science,* 1952, vol. 122, 501
- *Virology,* 1959, vol. 8, 396
- *Proceeding of the Society for the Biological Medicine,* 1950, vol. 73, 1
- *Nature,* 1962, vol. 195, 788
- **PAULSON et al.** *J. Biol. Chem.,* 1989, vol. 264, 17619
- **LOWE et al.** *Proc. Natl. Acad. Sci. U.S.A.,* 1989, vol. 86, 8227
- *Genes Develop.,* 1990, vol. 4, 1288
- *American Journal of Clinical Nutrition,* 1996, vol. 63, 639S
- *American Journal of Clinical Nutrition,* 1996, vol. 63, 627S
- *Bio/Technology,* 1991, vol. 9, 830
- **SOSHIKI BAIYO.** *Tissue Culture,* 1994, vol. 20
- **SOSHIKI BAIYO.** *Tissue Culture,* 1995, vol. 21
- *Trends in Biotechnology,* 1997, vol. 15, 45
- **MARK J. ZOLLER ; MICHAEL SMITH.** *Nucleic Acids Research,* 1982, vol. 10 (20), 6487-6500

- **ASAKO SAWANO ; ATSUSHI MIYAWAKI.** *Nucleic Acids Research,* 2000, vol. 28 (16), e78
- **CLACKSON et al.** *Nature,* 1991, vol. 352, 624-8
- **MARKS et al.** *J. Mol. Biol.,* 1991, vol. 222, 581-97
- **WATERHOUSES et al.** *Nucleic Acids Res.,* 1993, vol. 21, 2265-6
- **GRIFFITHS et al.** *EMBO J.,* 1994, vol. 13, 3245-60
- **VAUGHAN et al.** *Nature Biotechnology,* 1996, vol. 14, 309-14
- **G. KOHLER ; C. MILSTEIN.** *Methods Enzymol.,* 1981, vol. 73, 3-46
- **MENDEZ et al.** *Nat. Genet.,* 1997, vol. 15, 146-56
- Goding, Monoclonal Antibodies: Principles and Practice. Academic Press, 1986, 59-103
- **MULLING, R. C. et al.** *Nature,* 1979, vol. 277, 108-114
- **MIZUSHIMA.** *Nucleic Acids Res.,* 1990, vol. 18, 5322
- Antibodies: A Laboratory Manual. Cold Spring Harbor Laboratory, 1988
- *J. Biochem.,* 1987, vol. 101, 1307
- *Gene,* 1984, vol. 27, 223
- *Proc. Natl. Acad. Sci. U.S.A.,* 1981, vol. 78, 1527
- *Cytotechnology,* 1990, vol. 4, 173
- *Biochem. Biophys. Res. Commun.,* 1987, vol. 149, 960
- *Cell,* 1985, vol. 41, 479
- *Cell,* 1983, vol. 33, 717
- *J. Immunol. Methods,* 1994, vol. 167, 271
- *Mol. Immunol.,* 2000, vol. 37, 1035
- *Hybridoma,* 1998, vol. 17, 559
- *Methods in Enzymol.,* 1987, vol. 154, 3
- Molecular Cloning: A Laboratory Manual. Cold Spring Harbor Lab. Press, 1989
- Current Protocols in Molecular Biology
- *Strategies,* 1992, vol. 5, 58
- *Nucleic Acids Research,* 1989, vol. 17, 9494
- *DNA Cloning: A Practical Approach,* 1985, vol. I, 49
- *Mol. Cell. Biol.,* 1983, vol. 3, 280
- *Gene,* 1985, vol. 33, 103
- *Strategies,* 1992, vol. 5, 81
- *Genetics,* 1954, vol. 39, 440
- *Science,* 1983, vol. 222, 778
- *J. Mol. Biol.,* 1983, vol. 166, 1
- *J. Mol. Biol.,* 1996, vol. 16, 118
- *Gene,* 1985, vol. 38, 275
- **SANGER et al.** *Proc. Natl. Acad. Sci., U. S. A.,* 1977, vol. 74, 5463
- Antibodies: A Laboratory Manual. Cold Spring Harbor Laboratory, 1998
- Monoclonal Antibodies: Principles and Practice. Academic Press Limited, 1996
- *Nature,* 1970, vol. 227, 680
- Antibodies, A Laboratory Manual. Cold Spring Harbor Laboratory, 1988
- *Cancer Immunol. Immunother.,* 1993, vol. 36, 373
- *Cancer Immunol. Immunother.,* vol. 36, 373